# EUROPEAN PATENT SPECIFICATION

(11) **EP 1 379 507 B1**
(45) Date of publication and mention of the grant of the patent: **17.08.2011**
(21) Application number: 02725709.6
(22) Date of filing: 17.04.2002
(51) Int. Cl.: C07D 213/75, C07D 217/22, C07D 401/12, C07D 215/38, C07D 403/12, A61K 31/44, A61K 31/47, A61P 29/00

(54) **HETEROARYL UREAS CONTAINING NITROGEN HETERO-ATOMS AS p38 KINASE INHIBITORS**
STICKSTOFF ATOM ENTHALTENDEN HETEROARYL-HARNSTOFFE ALS INHIBITOREN DER P38-KINASE
UREES HETEROARYLES CONTENANT DES HETERO-ATOMES D'AZOTE COMME INHIBITEURS DE KINASE P38

(30) Priority: 20.04.2001 US 838286
(43) Date of publication of application: 14.01.2004
(60) Divisional application: 11170364.1
(73) Proprietor: Bayer HealthCare, LLC, Tarrytown, NY 10591 (US)
(72) Inventor: DUMAS, Jacques, Bethany, CT 06524 (US); RIEDL, Bernd, 42329 Wuppertal (DE); KHIRE, Uday, Hamden, CT 06518 (US); SIBLEY, Robert, N., North Haven, CT 06473 (US); HATOUM-MOKDAD, Holia, Hamden, CT 06514 (US); MONAHAN, Mary-Katherine, Hamden, CT 06517 (US); GUNN, David, E., Hamden, CT 06517 (US); LOWINGER, Timothy, B., D-42117 Wuppertal (DE); SCOTT, William, J., Guilford, CT 06437 (US); SMITH, Roger, A., Madison, CT 06443 (US); WOOD, Jill, E., North Haven, CT 06473 (US)
(74) Representative: Hart-Davis, Jason
(86) International application number: PCT/US2002/012064
(87) International publication number: WO 2002/085859

(56) References cited:
- WO-A-00/41698
- WO-A-01/36403
- WO-A-99/32111
- WO-A-99/32463
- US-A- 4 279 639
- MUIJLWIJK-KOEZEN, J.E. ET. AL.: "Isoquinoline and Quinazoline Urea Analogues as Antagonists for the Human Adenosine A3 Receptor." JOURNAL OF MEDICINAL CHEMISTRY, vol. 43, no. 11, 2000, pages 2227-38, XP002147879
- CARLING, R.W. ET. AL.: "1-(3-Cyanobenzylpiperidin-4-yl)-5-methyl- 4-phenyl 1,3-dihydroimidazol-2-one: A Selective High Affinity Antagonist for the Human Dopamine D4 Receptor with Excellent Selectivity over Ion Channels" JOURNAL OF MEDICINAL CHEMISTRY, vol. 42, no. 14, 1999, pages 2706-15, XP001093697
- DUMAS, J. ET. AL.: "1-Phenyl-5-pyrazolyl Ureas: Potent ans Selective p38 Kinase Inhibitors." BIOORGANIC AND MEDICINAL CHEMISTRY LETTERS, vol. 10, 2000, pages 2051-4, XP004208309

## Description

### Field of the Invention

This invention relates to the use of a group of heteroaryl ureas containing nitrogen hetero atoms in treating cytokine mediated diseases and proteolytic enzyme mediated diseases, and pharmaceutical compositions for use in such therapy.

### Background of the Invention

Two classes of effector molecules which are critical for the progression of rheumatoid arthritis are pro-inflammatory cytokines and tissue degrading proteases. Recently, a family of kinases was described which is instrumental in controlling the transcription and translation of the structural genes coding for these effector molecules.

The mitogen-activated protein (MAP) kinase family is made up of a series of structurally related proline-directed serine/threonine kinases which are activated either by growth factors (such as EGF) and phorbol esters (ERK), or by IL-1, TNFα or stress (p38, JNK). The MAP kinases are responsible for the activation of a wide variety of transcription factors and proteins involved in transcriptional control of cytokine production. A pair of novel protein kinases involved in the regulation of cytokine synthesis was recently described by a group from SmithKline Beecham (Lee et al. Nature 1994, 372, 739). These enzymes were isolated based on their affinity to bond to a class of compounds, named CSAIDSs (cytokine suppressive anti-inflammatory drugs) by SKB. The CSAIDs, bicyclic pyridinyl imidazoles, have been shown to have cytokine inhibitory activity both *in vitro* and *in vivo.* The isolated enzymes, CSBP-1 and -2 (CSAID binding protein 1 and 2) have been cloned and expressed. A murine homologue for CSBP-2, p38, has also been reported (Han et al. Science 1994, 265, 808).

Early studies suggested that CSAIDs function by interfering with m-RNA translational events during cytokine biosynthesis. Inhibition of p38 has been shown to inhibit both cytokine production (eg., TNFα, IL-1, IL-6, IL-8) and proteolytic enzyme production (eg., MMP-1, MMP-3) *in vitro* and/or *in vivo.*

Clinical studies have linked TNFα production and/or signaling to a number of diseases including rheumatoid arthritis (Maini. J. Royal Coll. Physicians London 1996, 30, 344). In addition, excessive levels of TNFα have been implicated in a wide variety of inflammatory and/or immunomodulatory diseases, including acute rheumatic fever (Yegin et al. Lancet 1997, 349, 170), bone resorption (Pacifici et al. J. Clin. Endocrinol. Metabol. 1997, 82, 29), postmenopausal osteoperosis (Pacifici et al. J. Bone Mineral Res. 1996, 11, 1043), sepsis (Blackwell et al. Br. J. Anaesth. 1996, 77, 110), gram negative sepsis (Debets et al. Prog. Clin. Biol. Res. 1989, 308, 463), septic shock (Tracey et al. Nature 1987, 330, 662; Girardin et al. New England J. Med. 1988, 319, 397), endotoxic shock (Beutler et al. Science 1985, 229, 869; Ashkenasi et al. Proc. Nat'l. Acad. Sci. USA 1991, 88, 10535), toxic shock syndrome, (Saha et al. J. Immunol. 1996, 157, 3869; Lina et al. FEMS Immunol. Med. Microbiol. 1996, 13, 81), systemic inflammatory response syndrome (Anon. Crit. Care Med. 1992, 20, 864), inflammatory bowel diseases (Stokkers et al. J. Inflamm. 1995-6, 47, 97) including Crohn's disease (van Deventer et al. Aliment. Pharmacol. Therapeu. 1996, 10 (Suppl. 2), 107; van Dullemen et al. Gastroenterology 1995, 109, 129) and ulcerative colitis (Masuda et al. J. Clin. Lab. Immunol. 1995, 46, 111), Jarisch-Herxheimer reactions (Fekade et al. New England J. Med. 1996, 335, 311), asthma (Amrani et al. Rev. Malad. Respir. 1996, 13, 539), adult respiratory distress syndrome (Roten et al. Am. Rev. Respir. Dis. 1991, 143, 590; Suter et al. Am. Rev. Respir. Dis. 1992, 145, 1016), acute pulmonary fibrotic diseases (Pan et al. Pathol. Int. 1996, 46, 91), pulmonary sarcoidosis (Ishioka et al. Sarcoidosis Vasculitis Diffuse Lung Dis. 1996, 13, 139), allergic respiratory diseases (Casale et al. Am. J. Respir. Cell Mol. Biol. 1996, 15, 35), silicosis (Gossart et al. J. Immunol. 1996, 156, 1540; Vanhee et al. Eur. Respir. J. 1995, 8, 834), coal worker's pneumoconiosis (Borm et al. Am. Rev. Respir. Dis. 1988, 138, 1589), alveolar injury (Horinouchi et al. Am. J. Respir. Cell Mol. Biol. 1996, 14, 1044), hepatic failure (Gantner et al. J. Pharmacol. Exp. Therap. 1997, 280, 53), liver disease during acute inflammation (Kim et al. J. Biol. Chem. 1997, 272, 1402), severe alcoholic hepatitis (Bird et al. Ann. Intern. Med. 1990, 112, 917), malaria (Grau et al. Immunol. Rev. 1989, 112, 49; Taverne et al. Parasitol. Today 1996, 12, 290) including Plasmodium falciparum malaria (Perlmann et al. Infect. Immunit. 1997, 65, 116) and cerebral malaria (Rudin et al. Am. J. Pathol. 1997, 150, 257), non-insulin-dependent diabetes mellitus (NIDDM; Stephens et al. J. Biol. Chem. 1997, 272, 971; Ofei et al. Diabetes 1996, 45, 881), congestive heart failure (Doyama et al. Int. J. Cardiol. 1996, 54, 217; McMurray et al. Br. Heart J. 1991, 66, 356), damage following heart disease (Malkiel et al. Mol. Med. Today 1996, 2, 336), atherosclerosis (Parums et al. J. Pathol. 1996, 179, A46), Alzheimer's disease (Fagarasan et al. Brain Res. 1996, 723, 231; Aisen et al. Gerontology 1997, 43, 143), acute encephalitis (Ichiyama et al. J. Neurol. 1996, 243, 457), brain injury (Cannon et al. Crit. Care Med. 1992, 20, 1414; Hansbrough et al. Surg. Clin. N. Am. 1987, 67, 69; Marano et al. Surg. Gynecol. Obstetr. 1990, 170, 32), multiple sclerosis (M.S.; Coyle. Adv. Neuroimmunol. 1996, 6, 143; Matusevicius et al. J. Neuroimmunol. 1996, 66, 115) including demyelation and oligiodendrocyte loss in multiple sclerosis (Brosnan et al. Brain Pathol. 1996, 6, 243), advanced cancer (MucWierzgon et al. J. Biol. Regulators Homeostatic Agents 1996, 10, 25), lymphoid malignancies (Levy et al. Crit. Rev. Immunol. 1996, 16, 31), pancreatitis (Exley et al. Gut 1992, 33, 1126) including systemic complications in acute pancreatitis (McKay et al. Br. J. Surg. 1996, 83, 919), impaired wound healing in infection inflammation and cancer (Buck et al. Am. J. Pathol. 1996, 149, 195), myelodysplastic syndromes (Raza et al. Int. J. Hematol. 1996, 63, 265), systemic lupus erythematosus (Maury et al. Arthritis Rheum. 1989, 32, 146), biliary cirrhosis (Miller et al. Am. J. Gasteroenterolog. 1992, 87, 465), bowel necrosis (Sun et al. J. Clin. Invest. 1988, 81, 1328), psoriasis (Christophers. Austr. J. Dermatol. 1996, 37, S4), radiation injury (Redlich et al. J. Immunol. 1996, 157, 1705), and toxicity following administration of monoclonal antibodies such as OKT3 (Brod et al. Neurology 1996, 46, 1633). TNFα levels have also been related to host-versus-graft reactions (Piguet et al. Immunol. Ser. 1992, 56, 409) including ischemia reperfusion injury (Colletti et al. J. Clin. Invest. 1989, 85, 1333) and allograft rejections including those of the kidney (Maury et al. J. Exp. Med. 1987, 166, 1132), liver (Imagawa et al. Transplantation 1990, 50, 219), heart (Bolling et al. Transplantation 1992, 53, 283), and skin (Stevens et al. Transplant. Proc. 1990, 22, 1924), lung allograft rejection (Grossman et al. Immunol. Allergy Clin. N. Am. 1989, 9, 153) including chronic lung allograft rejection (obliterative bronchitis; LoCicero et al. J. Thorac. Cardiovasc. Surg. 1990, 99, 1059), as well as complications due to total hip replacement (Cirino et al. Life Sci. 1996, 59, 86). TNFα has also been linked to infectious diseases (review: Beutler et al. Crit. Care Med. 1993, 21, 5423; Degre. Biotherapy 1996, 8, 219) including tuberculosis (Rook et al. Med. Malad. Infect. 1996, 26, 904), Helicobacter pylori infection during peptic ulcer disease (Beales et al. Gastroenterology 1997, 112, 136), Chaga's disease resulting from Trypanosoma cruzi infection (Chandrasekar et al. Biochem. Biophys. Res. Commun. 1996, 223, 365), effects of Shiga-like toxin resulting from E. coli infection (Harel et al. J. Clin. Invest. 1992, 56, 40), the effects of enterotoxin A resulting from Staphylococcus infection (Fischer et al. J. Immunol. 1990, 144, 4663), meningococcal infection (Waage et al. Lancet 1987, 355; Ossege et al. J. Neurolog. Sci. 1996, 144, 1), and infections from Borrelia burgdorferi (Brandt et al. Infect. Immunol. 1990, 58, 983), Treponema pallidum (Chamberlin et al. Infect. Immunol. 1989, 57, 2872), cytomegalovirus (CMV; Geist et al. Am. J. Respir. Cell Mol. Biol. 1997, 16, 31), influenza virus (Beutler et al. Clin. Res. 1986, 34, 491a), Sendai virus (Goldfield et al. Proc. Nat'l. Acad. Sci. USA 1989, 87, 1490), Theiler's encephalomyelitis virus (Sierra et al. Immunology 1993, 78, 399), and the human immunodeficiency virus (HIV; Poli. Proc. Nat'l. Acad. Sci. USA 1990, 87, 782; Vyakaram et al. AIDS 1990, 4, 21; Badley et al. J. Exp. Med. 1997, 185, 55).

Because inhibition of p38 leads to inhibition of TNFα production, p38 inhibitors will be useful in treatment of the above listed diseases.

A number of diseases are thought to be mediated by excess or undesired matrix-destroying metalloprotease (MMP) activity or by an imbalance in the ratio of the MMPs to the tissue inhibitors of metalloproteinases (TIMPs). These include osteoarthritis (Woessner et al. J. Biol. Chem. 1984, 259, 3633), rheumatoid arthritis (Mullins et al. Biochim. Biophys. Acta 1983, 695, 117; Woolley et al. Arthritis Rheum. 1977, 20, 1231; Gravallese et al. Arthritis Rheum. 1991, 34, 1076), septic arthritis (Williams et al. Arthritis Rheum. 1990, 33, 533), tumor metastasis (Reich et al. Cancer Res. 1988, 48, 3307; Matrisian et al. Proc. Nat'l. Acad. Sci., USA 1986, 83, 9413), periodontal diseases (Overall et al. J. Periodontal Res. 1987, 22, 81), corneal ulceration (Bums et al. Invest. Opthalmol. Vis. Sci. 1989, 30, 1569), proteinuria (Baricos et al. Biochem. J. 1988, 254, 609), coronary thrombosis from atherosclerotic plaque rupture (Henney et al. Proc. Nat'l. Acad. Sci., USA 1991, 88, 8154), aneurysmal aortic disease (Vine et al. Clin. Sci. 1991, 81, 233), birth control (Woessner et al. Steroids 1989, 54, 491), dystrophobic epidermolysis bullosa (Kronberger et al. J. Invest. Dermatol. 1982, 79, 208), degenerative cartilage loss following traumatic joint injury, osteopenias mediated by MMP activity, tempero mandibular joint disease, and demyelating diseases of the nervous system (Chantry et al. J. Neurochem. 1988, 50, 688).

Because inhibition of p38 leads to inhibition of MMP production, p38 inhibitors will be useful in treatment of the above listed diseases.

Inhibitors of p38 are active in animal models of TNFα production, including a muirne lipopolysaccharide (LPS) model of TNFα production. Inhibitors of p38 are active in a number of standard animal models of inflammatory diseases, including carrageenan-induced edema in the rat paw, arachadonic acid-induced edema in the rat paw, arachadonic acid-induced peritonitis in the mouse, fetal rat long bone resorption, murine type II collagen-induced arthritis, and Fruend's adjuvant-induced arthritis in the rat. Thus, inhibitors of p38 will be useful in treating diseases mediated by one or more of the above-mentioned cytokines and/or proteolytic enzymes.

The need for new therapies is especially important in the case of arthritic diseases. The primary disabling effect of osteoarthritis, rheumatoid arthritis and septic arthritis is the progressive loss of articular cartilage and thereby normal joint function. No marketed pharmaceutical agent is able to prevent or slow this cartilage loss, although nonsteroidal antiinflammatory drugs (NSAIDs) have been given to control pain and swelling. The end result of these diseases is total loss of joint function which is only treatable by joint replacement surgery. P38 inhibitors will halt or reverse the progression of cartilage loss and obviate or delay surgical intervention.

Several patents have appeared claiming polyarylimidazoles and/or compounds containing polyarylimidazoles as inhibitors of p38 (for example, Lee et al. WO 95/07922; Adams et al. WO 95/02591; Adams et al. WO 95/13067; Adams et al. WO 95/31451). It has been reported that arylimidazoles complex to the ferric form of cytochrome P450_{cam} (Harris et al. *Mol. Eng.* **1995,** *5*, 143, and references therein), causing concern that these compounds may display structure-related toxicity (Howard-Martin et al. Toxicol. Pathol. 1987, 15, 369). Therefore, there remains a need for improved p38 inhibitors.
J. Med. Chem., 2000, 43 (11), pp 2227-2238 provides Isoquinoline and Quinazoline Urea analogues as antagonists for the human adenosine A₃ receptor.
J. Med. Chem., 1999, 42 (14), pp 2706-2715 deals with 1-(3-Cyanobenzylpiperidin-4-yl)-5-methyl-4-phenyl-1,3-dihydroimidazol-2-one which is a selective high-affinity antagonist for the human dopamine D4 receptor with selectivity over ion channels. US4279639 deals with 1-Pyridyl-3-aryl-urea derivatives which are useful as plant growth regulators.
WO1999032463 relates to the use of a group of aryl ureas in treating cytokine mediated diseases and proteolytic enzyme mediated diseases, and pharmaceutical compositions for use in such therapy.
WO 0041698 concerns method of treating a disease mediated by p38 kinase within a host comprising administration of aryl urea derivatives e.g. for the treatment of cancer or arthritis. This invention also relates to pharmaceutical compositions for use in such treatment.

### Summary of the Invention

The present invention as defined in any one of claims 1 to 25 relates to a compound of Formula I or a pharmaceutically acceptable salt thereof for use in a method of treating a disease mediated by p38, a use of N-quinolinyl-ureas and N-isoquinolinyl-ureas for the preparation of medicaments for the treatment of p38 mediated diseases, pharmaceutical compositions for the treatment of p38 mediated diseases comprising N-quinolinyl-ureas and N-isoquinolinyl-ureas as well as a use of said compositions for the preparation of medicaments for the treatment of p38 mediated diseases.

This invention provides compounds, generally described as heteroaryl ureas containing nitrogen hetero atoms, including quinoline and isoquinoline ureas, which inhibit p38 mediated events and thus inhibit the production of cytokines (such as TNFa, IL-1 and IL-8) and proteolytic enzymes (such as MMP-1 and MMP-3). The invention also provides compositions which contain heteroaryl ureas and a use of said heteroaryl ureas for the preparation of a medicament for treating a cytokine mediated disease state in humans or mammals, wherein the cytokine is one whose production is affected by p38. Examples of such cytokines include, but are not limited to TNFα, IL-1 and IL-8. The invention also provides a use of said heteroaryl ureas for the preparation of a medicament for treating a protease mediated disease state in humans or mammals, wherein the protease is one whose production is affected by p38, e.g. disease states mediated by one or more cytokines or proteolytic enzymes produced and/or activated by a p38 mediated process. Examples of such proteases include, but are not limited to collagenase (MMP-1) and stromelysin (MMP-3).

Accordingly, these compounds are useful therapeutic agents for such acute and chronic inflammatory and/or immunomodulatory diseases as rheumatoid arthritis, osteoarthritis, septic arthritis, rheumatic fever, bone resorption, postmenopausal osteoperosis, sepsis, gram negative sepsis, septic shock, endotoxic shock, toxic shock syndrome, systemic inflammatory response syndrome, inflammatory bowel diseases including Crohn's disease and ulcerative colitis, Jarisch-Herxheimer reactions, asthma, adult respiratory distress syndrome, acute pulmonary fibrotic diseases, pulmonary sarcoidosis, allergic respiratory diseases, silicosis, coal worker's pneumoconiosis, alveolar injury, hepatic failure, liver disease during acute inflammation, severe alcoholic hepatitis, malaria including Plasmodium falciparum malaria and cerebral malaria, non-insulin-dependent diabetes mellitus (NIDDM), congestive heart failure, damage following heart disease, atherosclerosis, Alzheimer's disease, acute encephalitis, brain injury, multiple sclerosis including demyelation and oligiodendrocyte loss in multiple sclerosis, advanced cancer, lymphoid malignancies, tumor metastasis, pancreatitis, including systemic complications in acute pancreatitis, impaired wound healing in infection, inflammation and cancer, periodontal diseases, corneal ulceration, proteinuria, myelodysplastic syndromes, systemic lupus erythematosus, biliary cirrhosis, bowel necrosis, psoriasis, radiation injury, toxicity following administration of monoclonal antibodies such as OKT3, host-versus-graft reactions including ischemia reperfusion injury and allograft rejections including kidney, liver, heart, and skin allograft rejections, lung allograft rejection including chronic lung allograft rejection (obliterative bronchitis) as well as complications due to total hip replacement, and infectious diseases including tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus, and the human immunodeficiency virus (HIV).

The present invention, therefore, provides hetaryl urea compounds containing nitrogen hetero-atoms, and compositions which comprise hetaryl urea compounds containing nitrogen heteroatoms and a use of these compounds or compositions for the preparation of a medicament for treating of p38-mediated disease states in humans or mammals, e.g., disease states mediated by one or more cytokines or proteolytic enzymes produced and/or activated by a p38 mediated process. In these uses a compound of formula I, or a pharmaceutically acceptable salt thereof, is represented by:

A-D-B (I).

In formula I,
D is -NH-C(O)-NH-,
A is a substituted or unsubstituted quinolinyl or isoquinolinyl group,
B is preferably a substituted or unsubstituted bridged cyclic structure of up to 30 carbon atoms of the formula-L-(ML¹)_{q}.
L in the formula -L(ML¹)_{q} is a 5 or 6 membered cyclic structure bond directly to D, L¹ is a cyclic moiety of at least 5 members,
M is a bridging group having at least one atom and q is an integer of 1-3.
Each cyclic structure of L and L¹ contains from 0-4 members of the group consisting ofN, O and S.
L¹ can be substituted by the substituents -C(O)R^{a}, -C(NR³)R^{b}, -C(0)NR³R^{b}, - SO₂NR^{a}R^{b}, -and -SO₂R^{a} wherein each R^{a} and R^{b} are independently hydrogen or a carbon based moiety of up to 24 carbon atoms, optionally containing heteroatoms selected from N, S and O, and optionally substituted by halogen.

The substituents for the groups of A are preferably selected from the group consisting of halogen, up to per-halo, and Wn, where n is 0-3 and each W is independently selected from the group consisting of C₁₋₁₀ alkyl, C₁₋₁₀ alkoxy, C₃₋₁₀ cycloalkyl having at least five cyclic members and 0-3 heteroatoms, C₂₋₁₀ alkenyl, C₁₋₁₀ alkenoyl, substituted C₁₋₁₀ alkyl, substituted C₁₋₁₀ alkoxy, a substituted C₃₋₁₀ cycloalkyl having at least 5 cyclic members and 0-3 heteroatoms selected from N, S and O; substituted C₂₋₁₀ alkenyl, substituted C₁₋₁₀ alkenoyl, C₆-C₁₄ aryl, C₇-C₂₄ alkaryl, C₇-C₂₄ aralkyl, C₃-C₁₂ hetaryl having at least 5 cyclic members and 1-3 heteroatoms selected from O, N and S, C₄-C₂₃ alkheteroaryl having at least 5 cyclic members and 1-3 heteroatoms selected from O, N and S, substituted C₆-C₁₄ aryl, substituted C₃-C₁₂ hetaryl having at least 5 members and 1-3 heteroatoms selected from O, N and S, substituted C₇-C₂₄ aralkyl, substituted C₇-C₂₄ alkaryl, substituted C₄-C₂₃ alkheteroaryl having at least 5 cyclic members and 1-3 heteroatoms selected from O, N and S; -CN, -CO₂R⁷,-C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl, C₃-C₁₀ cycloalkyl having at least 5 cyclic members and 0-3 heteroatoms selected from O, S and N, C₆-C₁₄ aryl, C₃-C₁₀ hetaryl having at least 5 cyclic members and 0-3 heteroatoms selected from O, S and N, up to per halosubstituted C₃-C₁₀ cycloalkyl having at least 6 cyclic members and 0-3 heteroatoms selected from O, S and N, up to per halo substituted C₆-C₁₄ aryl, and up to per halo substituted C₃-C₁₀ hetaryl having at least 6 cyclic members and 0-3 heteroatoms selected from O, S and N.

Where W is a substituted group, it is substituted by halogen, up to per halo, or by one or more substituents independently selected from the group consiting of -CN, -CO₂R⁷, -C(O)NR⁷R^{7'}, -C(O)-R⁷, -NO₂, -OR⁷, -SR⁷, -NR⁷R⁷, -NR⁷C(O)OR⁷,-NR⁷C(O)R^{7'} with each R⁷ and R^{7'} independently as defined above.

Where A is a substituted quinolinyl or isoquinolinyl group, A is preferably substituted 1 to 3 times by 1 or more substituents selected from the group consisting of - CN, halogen, C₁-C₁₀ alkyl, C₁-C₁₀ alkoxy, -OH, up to per halo substituted C₁-C₁₀ alkyl, up to per halo substituted C₁-C₁₀ alkoxy or phenyl substituted by halogen up to per halo.

R^{a} and R^{b} preferably are each, independently, C₁₋₁₀ alkyl, C₁₋₁₀ alkoxy, C₃₋₁₀ cycloalkyl having at least 5 cyclic members and 0-3 heteroatoms selected from N, S and O, C₂₋₁₀ alkenyl, C₁₋₁₀ alkenoyl, C₆₋₁₄ aryl, C₁₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having at least 5 cyclic members and 0-3 heteroatoms selected from N, S and O, substituted C₂₋₁₀ alkenyl, substituted C₁₋₁₀ alkenoyl, substituted C₆-C₁₄ aryl, substituted C₃₋₁₂ hetaryl having at least 5 cyclic members and 1-3 heteroatoms selected from N, S and O, substituted C₇₋₂₄ alkaryl or substituted C₇-C₂₄ aralkyl. Where R³ and/or R^{b} are a substituted group, they are substituted by halogen up to per halo hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g}.

R^{a} and R^{b} can also be
-OSi(R_{f})₃ where R_{f} is hydrogen or a carbon based moiety of up to 24 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen; or
b) bound together to form a 5-7 member heterocyclic structure of 1-3 heteroatoms selected from N, S and O, or a substituted 5-7 member heterocyclic structure of 1-3 heteroatoms selected from N, S and O substituted by halogen, hydroxy or carbon based substituents of up to 24 carbon atoms, which optionally contain heteroatoms selected from N, S and O and are optionally substituted by halogen; or
c) one of R^{a} or R^{b} can be -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, and carbon based substituents of up to 24 carbon atoms, which optionally contain heteroatoms selected from N, S and O and are optionally substituted by halogen.

The carbon based moieties of R_{f} and the substituents on R^{a} and R^{b} include C₁₋₁₀ alkyl, C₁₋₁₀ alkyl, C₁₋₁₀ alkoxy, C₃-C₁₀ cycloalkyl having 0-3 heteroatoms selected from O, S and N, C₆₋₁₂ aryl, C₃-C₁₂ hetaryl having 1-3 heteroatoms selected from O, S and N, C₇₋₂₄ aralkyl, substituted C₁₋₁₀ alkyl, substituted C₁-C₁₀ alkoxy, substituted C₃-C₁₂ cycloalkyl having 0-3 heteroatoms selected from O, S and N, substituted C₃-C₁₂ heteraryl having 1-3 heteroatoms selected from O, S, and N, substituted C₆₋₁₂ aryl, and substituted C₇₋₂₄ alkaryl, where R_{f} is a substituted group it is substituted halogen up to per halo, hydroxy, C₁₋₁₀ alkyl, C₃₋₁₂ cycloalkyl having 0-3 heteroatoms selected from O, S andN, C₃₋₁₂ hetaryl having 1-3 heteroatoms selected from N, S and 0, C₁₋₁₀ alkoxy, C₆₋₁₂ aryl, C_{y-}-C₂₄ alkaryl, C₇-C₂₄ aralkyl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g};
where R_{g} is C₁₋₁₀ alkyl; -CN, -CO₂R_{d}, -OR_{d}, -SR_{d}, -NO₂, -C(O) Rₑ, -NR_{d}Rₑ, -NR_{d} C(O)ORₑ and -NR_{d}C(O)Rₑ, and R_{d} and Rₑ are independently selected from the group consisting of hydrogen, C₁₋₁₀, alkyl, C₁₋₁₀ alkoxy, C₃₋₁₀ cycloalkyl having 0-3 heteroatoms selected from O, N and S, C₆₋₁₂ aryl, C₃-C₁₂ hetaryl with 1-3 heteroatoms selected from O, N and S and C₇-C₂₄ aralkyl, C₇-C₂₄ alkaryl, up to per halo substituted C₁-C₁₀ alkyl, up to per halo substituted C₃-C₁₀ cycloalkyl having 0-3 heteroatoms selected from O, N and S, up to per halo substituted C₆-C₁₄ aryl, up to per halo substituted C₃-C₁₂ hetaryl having 1-3 heteroatoms selected from O, N, and S, halo substituted C₇-C₂₄ alkaryl up to per halo alkaryl, and up to per halo substituted C₇-C₂₄ aralkyl.

The bridging group M in the formula -L-(ML¹)q, for B is preferably selected from the group consisting of -O-, -S-, -N(R⁷)-, -(CH₂)ₘ-, -C(O)-, -CH(OH)-, -(CH₂)ₘO-, -(CH₂)ₘS-, -(CH₂)ₘN(R⁷)-, -O(CH₂)ₘ- CHX^{a}-, -CX^{a}₂-, -S-(CH₂)ₘ-, -N(R⁷)(CH₂)ₘ- and -CR³R^{b}- where m=1-3, X^{a} is hydrogen, R⁷, R^{a} and R^{b} are as defined above and q is 1. More preferably, M is -O-, -CH₂-, -S-, -NH-, -C(O)-, -O-CH₂- and -CH₂-O-.

The moieties L and L¹ in the formula -L-(ML¹)_{q} for B are typically each, independently, a substituted aryl moiety having at least 6 cyclic members, a substituted heterocyclic moiety having at least 5 cyclic members, an unsubstituted aryl moiety having at least 6 cyclic members or an unsubstituted heterocyclic moiety having at least 5 cyclic members. The heterocyclic and hetaryl moietes for L and L' typically have 1 to 4 members selected from the group of hetero atoms consisting of nitrogen, oxygen and sulfur with the balance of the hetaryl or heterocyclic moiety being carbon. More typical moieties for L¹ and L are selected from the group consisting of thiophene, substituted thiophene, phenyl, substituted phenyl, pyridinyl, substituted pyridinyl, pyrimidinyl and substituted pyrimidinyl.

Where L is substituted or L¹ is additionally substituted, the substituents are selected from the group consisting of halogen, up to per-halo, and Jn where n is 0-3, and J is as defined as follows.

Each J is independently selected from the group consisting of -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently as defined above , C₁₋₁₀ alkyl, C₁₋₁₀ alkoxy, C₃₋₁₀ cycloalkyl having at least five cyclic members and 0-3 heteroatoms, C₂₋₁₀ alkenyl, C₁₋₁₀ alkenoyl, C₆₋₁₄ aryl, C₃₋₁₂ hetaryl having at least five cyclic members and 1-3 heteroatoms selected from N, S and O, C₇₋₂₄ aralkyl, C₇₋₂₄ alkaryl, C₄-C₂₃ alkheteroaryl having at least 5 cyclic members and 1-3 heteroatoms selected from O, N and S, substituted C₁₋₁₀ alkyl, substituted C₁₋₁₀ alkoxy, substituted C₃₋₁₀ cycloalkyl having at least five cyclic members and 0-3 heteroatoms selected from N, S and O, substituted C₂₋₁₀ alkenyl substituted C₁₋₁₀ alkenoyl, substituted C₆ -C₁₂ aryl, substituted C₃₋₁₂ hetaryl having at least five cyclic members and 1-3 heteroatoms selected from N, S and O, substituted C₇₋₂₄ alkaryl , substituted C₇-C₂₄ aralkyl substituted C₄-C₂₃ alkheteroaryl having at least 5 cyclic members and 1-3 heteroatoms selected from O, N and S, and -Q-Ar.

Where J is a substituted group, it is substituted by halogen, up to per halo, or by one or more substituents independently selected from the group consisting of -CN, -CO₂R⁷, -C(O)-R⁷, -C(O)NR⁷R^{7'}, -OR⁷, -SR⁷, -NR⁷R^{7'}, -NO₂, -NR⁷C(O)R^{7'}, and -NR⁷C(O)OR^{7'}; with each R⁷ and R^{7'} independently as defined above for W.

Where J is -Q-Ar, Q is preferably a single bond, -O-, -S-, -N(R⁷)-, -(CH₂)ₘ-, -C(O)-, -CH(OH)-, -(CH₂)ₘO-, -(CH₂)ₘS-, -(CH₂)ₘN(R⁷)-, -O(CH₂)ₘ- ,-CHX^{a}-, -CX^{a}₂-, -S-(CH₂)ₘ- and -N(R⁷)(CH₂)ₘ-, where m= 1-3, and X^{a} is halogen and

Ar is a 5- or 6-member aromatic structure. This aromatic structure of Ar
a) contains 0-2 members selected from the group consisting of nitrogen, oxygen and sulfur,
b) is optionally substituted by halogen, up to per-halo, and
c) is optionally substituted by Zₙ₁, wherein n1 is 0 to 3 and each Z is independently selected from the group consisting of -CN, -NO₂, -OR⁷, - SR⁷, -NR⁷R^{7'}, -NR⁷C(O)OR^{7'}, -NR⁷C(O)R^{7'}, with each R⁷ and R^{7'} independently as defined above for W, C₁₋₁₀ alkyle, C₁₋₁₀ alkoxy, C₂₋₁₀ alkenyl and C₁₋₁₀ alkenoyl halo substituted C₁₋₁₀ alkyl up to per halo, halo substituted C₁₋₁₀ alkoxy up to per halo, halosubstituted C₂₋₁₀ alkenyl up to per halo and halosubstituted C₁₋₁₀ alkenoyl up to per halo.

Preferred compounds of Formula I include those wherein the cyclic structures of B and L bound directly to D are not substituted in the ortho position by-OH.

In Formula I, suitable hetaryl groups include, but are not limited to, 4-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 5-7 member atoms.

Suitable alkyl groups and alkyl portions of groups, e.g., alkoxy, etc. throughout include methyl, ethyl, propyl, butyl, etc., including all straight-chain and branched isomers such as isopropyl, isobutyl, sec-butyl, *tert-*butyl, etc.

Suitable aryl groups which do not contain heteroatoms include, for example, phenyl and 1- and 2-naphthyl.

The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl", as used herein also includes saturated heterocyclic groups.

Suitable halogen groups include F, Cl, Br, and/or I, from one to per-substitution (i.e. all H atoms on a group replaced by a halogen atom) being possible where an alkyl group is substituted by halogen, mixed substitution of halogen atom types also being possible on a given moiety.

The present invention is also directed to the use of pharmaceutically acceptable salts of formula I for the preparation of a medicament for treating a disease mediated by p38 within a host. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, trifluoromethanesulfonic acid, benzenesulphonic acid, *p*-toluenesulfonic acid, 1 - naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁺ Na⁺ or K⁺), alkaline earth cations (e.g., Mg⁺², Ca⁺² or Ba⁺²), the ammonium cation, as well as acid salts of organic bases, including aliphatic and aromatic substituted ammonium, and quaternary ammonium cations, such as those arising from protonation or peralkylation of triethylamine, *N,N*-diethylamine, *N,N*-dicyclohexylamine, lysine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).
A number of the compounds of Formula I possess asymmetric carbons and can therefore exist in racemic and optically active forms. Methods of separation of enantiomeric and diastereomeric mixtures are well known to one skilled in the art.

### General Preparative Methods

The compounds of Formula I may be prepared by the use of known chemical reactions and procedures, some from starting materials which are commercially available. Nevertheless, general preparative methods are provided below to aid one skilled in the art in synthesizing these compounds, with more detailed examples being provided in the Experimental section which follows.

Substituted and unsubstituted aminoquinolines, aminoisoquinolines and aminopyridines may be prepared using standard methods (see, for example: A.R. Katritzky et al. (Eds.). Comprehensive Heterocyclic Chemistry II, Vol. 5. M.H. Palmer. Heterocyclic Compounds; Arnold Ltd., London (1967). C.K. Esser et al. WO 96/18616. C.J. Donahue et al. Inorg. Chem. 30, 1991, 1588. E. Cho et al. WO 98/00402. A. Cordi et al. Bioorg. Med. Chem.. 3, 1995, 129). In addition, many aminoquinolines, aminoisoquinolines and aminopyridines are commercially available.

Substituted anilines may be generated using standard methods (March. Advanced Organic Chemistry, 3rd Ed.; John Wiley: New York (1985). Larock. Comprehensive Organic Transformations; VCH Publishers: New York (1989)). As shown in Scheme I, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. Hydrogenation Methods; Academic Press: London, UK (1985)). Nitroaryls may also be directly reduced using a strong hydride source, such as LiAlH₄ (Seyden-Penne. Reductions by the Alumino- and Borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)).

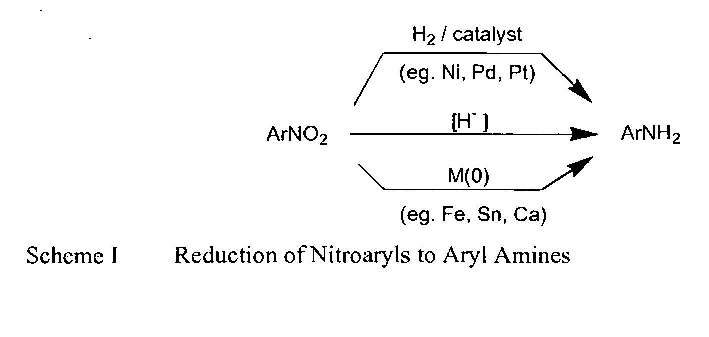

Nitroaryls are commonly formed by electrophilic aromatic nitration using HNO₃, or an alternative NO₂⁺ source. Nitroaryls may be further elaborated prior to reduction. Thus, nitroaryls substituted with

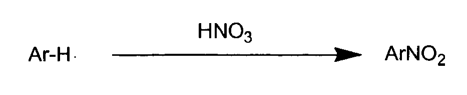

potential leaving groups (eg. F, Cl, Br, etc.) may undergo substitution reactions on treatment with nucleophiles, such as thiolate (exemplified in Scheme II) or phenoxide. Nitroaryls may also undergo Ullman-type coupling reactions (Scheme II).

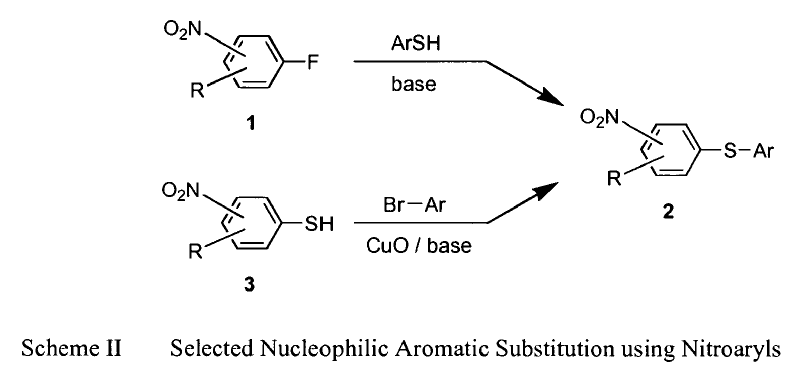

Nitroaryls may also undergo transition metal mediated cross coupling reactions. For example, nitroaryl electrophiles, such as nitroaryl bromides, iodides or triflates, undergo palladium mediated cross coupling reactions with aryl nucleophiles, such as arylboronic acids (Suzuki reactions, exemplified below), aryltins (Stille reactions) or arylzincs (Negishi reaction) to afford the biaryl (5).

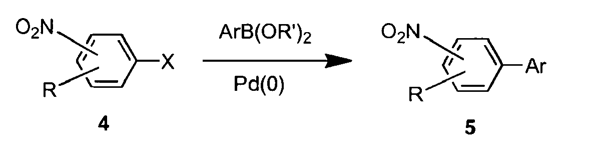

Either nitroaryls or anilines may be converted into the corresponding arenesulfonyl chloride (7) on treatment with chlorosulfonic acid. Reaction of the sulfonyl chloride with a fluoride source, such as KF then affords sulfonyl fluoride (**8**). Reaction of sulfonyl fluoride 8 with trimethylsilyl trifluoromethane in the presence of a fluoride source, such as tris(dimethylamino)sulfonium difluorotrimethylsiliconate (TASF) leads to the corresponding trifluoromethylsulfone (**9**). Alternatively, sulfonyl chloride 7 may be reduced to the arenethiol (**10**), for example with zinc amalgum. Reaction of thiol **10** with CHClF₂ in the presence of base gives the difluoromethyl mercaptan (11), which may be oxidized to the sulfone (**12**) with any of a variety of oxidants, including CrO₃-acetic anhydride (Sedova et al. Zh. Org. Khim. 1970, 6, (568).

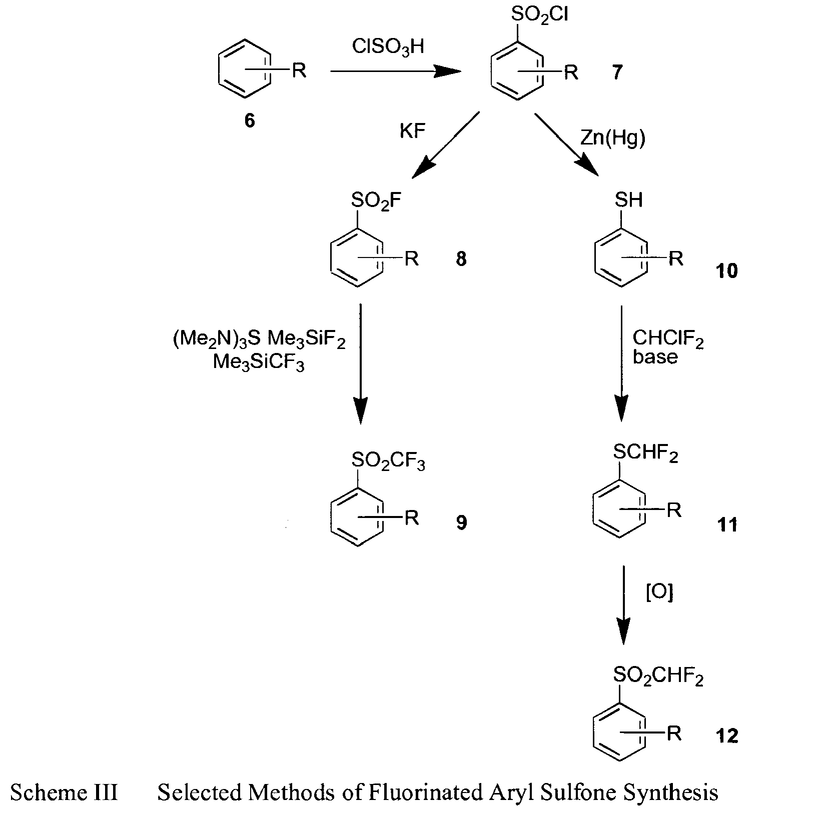

As shown in Scheme IV, non-symmetrical urea formation may involve reaction of an aryl isocyanate (**14**) with an aryl amine (**13**). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N*'-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **16** with an azide source, followed by rearrangement affords the isocyanate. The corresponding carboxylic acid (17) may also be subjected to Curtius-type rearrangements using diphenylphosphoryl azide (DPPA) or a similar reagent.

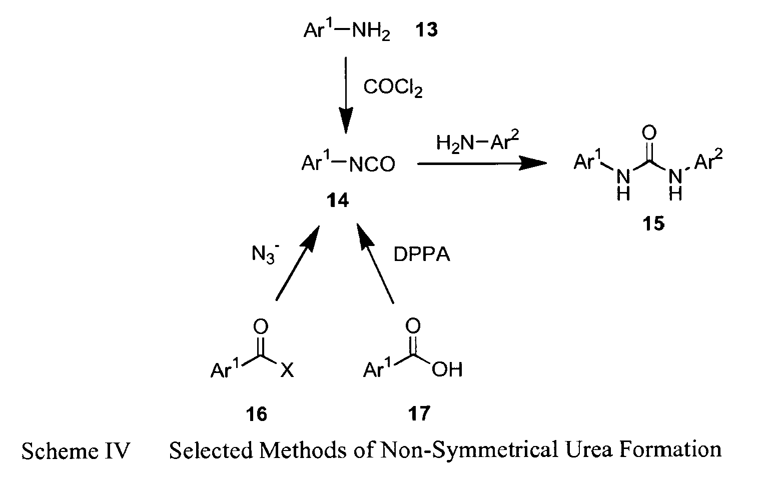

Finally, ureas may be further manipulated using methods familiar to those skilled in the art.

The invention also includes pharmaceutical compositions including a compound of Formula I, and a physiologically acceptable carrier.

The compounds may be administered orally, dermally, parenterally, by injection, by inhalation or spray, or sublingually, rectally or vaginally in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients. Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl, *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oil phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administered transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures one or more materials selected from lower alcohols, lower ketones , lower carboxylic acid esters, polar ethers, lower hydrocarbons, halogenated hydrocarbons.

Suitable penetration enhancing materials for transdermal delivery systems are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, *n*-butyl, *sec*-butyl isobutyl *tert*-butyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated dicarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene coploymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regimen will preferably be from 0.01 to 200 mg/Kg of total body weight. The daily vaginal dosage regimen will preferably be from 0.01 to 200 mg/Kg of total body weight. These daily dosages can be administered incrementally during the day, on a weekly basis on a biweekly basis or longer periods. Long term dosages typically range from 100-600 mg/kg of total body weight and preferably range from 100-400 mg/kg of total body weight.

Dosages for oral, vaginal and rectal administration and administration by injection can range from 0.01 mg - 600 mg/kg of total body weight.

The daily topical dosage regimen will preferably be from 0.1 to 200 mg administered between one to four times daily. The transdermal concentration will preferably be that required to maintain a daily dose of from 0.01 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, but not limited to the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, ie., the mode of treatment and the daily or weekly number of doses of a compound of Formula I or a pharmaceutically acceptable salt thereof given for a defined number of days, can be ascertained by those skilled in the art using conventional treatment tests.

The entire disclosure of all applications, patents and publications cited above and below are hereby incorporated by reference, including provisional application Serial No. 60/115,878 filed January 13, 1999 and non-provisional applications
Serial No. 09/778,039, filed February 7, 2001, and
Serial No. 09/257,265, filed February 25, 1999 and
Serial No. 09/425,229, filed October 22, 1999.

The compounds of formula I are producible from known compounds (or from starting materials which, in turn, are producible from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construed to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg. Unless otherwise stated, the term 'under high vacuum' refers to a vacuum of 0.4 - 1.0 mmHg.

All temperatures are reported in degrees Celsius (°C). Unless otherwise indicated, all parts and percentages are by weight.

Commercial grade reagents and solvents were used without further purification. N-cyclohexyl-N'-(methylpolystyrene)carbodiimide was purchased from Calbiochem-Novabiochem Corp. 5-(Trifluoromethyl)-2-aminopyridine, 3-aminoqunioline, 3-aminoisoquinoline, 1-(4-methylpiperazinyl)-3-aminoisoquinoline, ethyl4-isocyanatobenzoate, N-acetyl-4-chloro-2-methoxy-5-(trifluoromethyl)aniline, 4-(4-nitrobenzyl)pyridine, 4-phenoxyaniline, 4-(4-methylphenoxy)aniline, 4-(4-chlorophenoxy)aniline and 4-chloro-3-(trifluoromethyl)phenyl isocyanate were purchased and used without further purification. Syntheses of 2-amino-4-*tert*-butylpyridine (C.K. Esser et al. WO 96/18616; C.J. Donahue et al. Inorg. Chem. 30, 1991, 1588), 3-amino-2-methoxyquinoline (E. Cho et al. WO 98/00402; A. Cordi et al. EP 542,609; IBID Bioorg. Med. Chem.. 3, 1995, 129), 4-(3-carbamoylphenoxy)-1-nitrobenzene (K. Ikawa Yakugaku Zasshi 79,1959, 760*;* Chem. Abstr. 53, 1959, 12761 b), 4-[(4-methoxyphenyl)methylamino]aniline (P. Brenneisen et al. US 3,755,406; *IBID* US 3,839,582; *IBID* DE 1,935,388), 4-(4-pyridylcarbonyl)aniline (M.L. Carmello et al. Pestic. Sci. 45, 1995, 227), 3-*tert*-butylphenyl isocyanate (O. Rohr et al. DE 2,436,108) and 2-methoxy-5-(trifluoromethyl)phenyl isocyanate (K. Inukai et al. JP 42,025,067; *IBID* Kogyo Kagaku Zasshi 70, 1967, 491) have previously been described.

Thin-layer chromatography (TLC) was performed using Whatman^{®} pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science^{®} silica gel.

Melting points (mp) were determined using a Thomas-Hoover melting point apparatus or a Mettler FP66 automated melting point apparatus and are uncorrected. Fourier transform infrared sprectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (* 0.00) or residual protonated solvent (CHCl₃ * 7.26; MeOH * 3.30; DMSO * 2.49) as standard. Carbon (¹³C) NMR spectra were measured with a General Electric GN-Omega 300 (75 MHz) spectrometer with solvent (CDCl₃ * 77.0; MeOD-d₃; * 49.0; DMSO-d₆ * 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane or ammonia as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (∼1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV). Elemental analyses were conducted by Robertson Microlit Labs, Madison NJ.

All compounds displayed NMR spectra, LRMS and either elemental analysis or HRMS consistant with assigned structures.

### List of Abbreviations and Acronyms:

- AcOH: acetic acid
- anh: anhydrous
- atm: atmosphere(s)
- BOC: *tert*-butoxycarbonyl
- CDI: 1,1'-carbonyl diimidazole
- conc: concentrated
- dec: decomposition
- DMAC: *N,N*-dimethylacetamide
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EDCI: 1-(3-dimethylaminopropyl)-3-ethylcarbodiimide
- EtOAc: ethyl acetate
- EtOH: ethanol (100%)
- Et₂O: diethyl ether
- Et₃N: triethylamine
- HOBT: 1-hydroxybenzotriazole
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Substituted Anilines

### A1. General Method for Substituted Aniline Formation via Hydrogenation of a Nitroarene

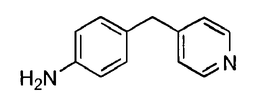

**4-(4-Pyridinylmethyl)aniline:** To a solution of 4-(4-nitrobenzyl)pyridine (7.0 g, 32.68 mmol) in EtOH (200 mL) was added 10% Pd/C (0.7 g) and the resulting slurry was shaken under a H₂ atmosphere (50 psi) using a Parr shaker. After 1 h, TLC and ¹H-NMR of an aliquot indicated complete reaction. The mixture was filtered through a short pad of Celite^{®}. The filtrate was concentrated *in vacuo* to afford a white solid (5.4 g, 90%): ¹H-NMR (DMSO-d₆) * 3.74 (s, 2H), 4.91 (br s, 2H), 6.48 (d, *J*=8.46 Hz, 2H), 6.86 (d, *J*=8.09 Hz, 2H), 7.16 (d, *J*=5.88 Hz, 2H), 8.40 (d, *J*=5.88 Hz, 2H); EI-MS *m*/*z* 184 (M⁺). This material was used in urea formation reactions without further purification.

### A2. General Method for Substituted Aniline Formation via Dissolving Metal Reduction of a Nitroarene

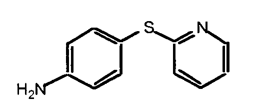

**4-(2-Pyridinylthio)aniline:** To a solution of 4-(2-pyridinylthio)-1-nitrobenzene (Menai ST 3355A; 0.220 g, 0.95 mmol) and H₂O (0.5 mL) in AcOH ( 5 mL) was added iron powder (0.317 g, 5.68 mmol) and the resulting slurry stirred for 16 h at room temp. The reaction mixture was diluted with EtOAc (75 mL) and H₂O (50 mL), basified to pH 10 by adding solid K₂CO₃ in portions (***Caution*:** foaming). The organic layer was washed with a saturated NaCl solution, dried (MgSO₄), concentrated *in vacuo.* The residual solid was purified by MPLC (30% EtOAc/70% hexane) to give the desired product as a thick oil (0.135 g, 70%): TLC (30% EtOAc/70% hexanes) R*_{f}* 0.20.

### A3a. General Method for Substituted Aniline Formation via Nitroarene Formation Through Nucleophilic Aromatic Substitution, Followed by Reduction

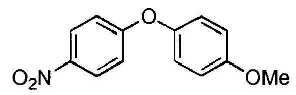

**Step 1. 1-Methoxy-4-(4-nitrophenoxy)benzene:** To a suspension ofNaH (95%, 1.50 g, 59 mmol) in DMF (100 mL) at room temp. was added dropwise a solution of 4-methoxyphenol (7.39 g, 59 mmol) in DMF (50 mL). The reaction was stirred 1 h, then a solution of 1-fluoro-4-nitrobenzene (7.0 g, 49 mmol) in DMF (50 mL) was added dropwise to form a dark green solution. The reaction was heated at 95 °C overnight, then cooled to room temp., quenched with H₂O, and concentrated *in vacuo.* The residue was partitioned between EtOAc (200 mL) and H₂O (200 mL). The organic layer was sequentially washed with H₂O (2 x 200 mL), a saturated NaHCO₃ solution (200 mL), and a saturated NaCl solution (200 mL), dried (Na₂SO₄), and concentrated *in vacuo.* The residue was triturated (Et₂O/hexane) to afford 1-methoxy-4-(4-nitrophenoxy)benzene (12.2 g, 100%): ¹H-NMR (CDCl₃) * 3.83 (s, 3H), 6.93-7.04 (m, 6H), 8.18 (d, *J*=9.2 Hz, 2H); EI-MS *m*/*z* 245 (M⁺).

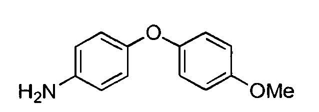

**Step 2. 4-(4-Methoxyphenoxy)aniline:** To a solution of 1-methoxy-4-(4-nitrophenoxy)benzene (12.0 g, 49 mmol) in EtOAc (250 mL) was added 5% Pt/C (1.5 g) and the resulting slurry was shaken under a H₂ atmosphere (50 psi) for 18 h. The reaction mixture was filtered through a pad of Celite^{®} with the aid of EtOAc and concentrated *in vacuo* to give an oil which slowly solidified (10.6 g, 100%): ¹H-NMR (CDCl₃) * 3.54 (br s, 2H), 3.78 (s, 3H), 6.65 (d, *J*=8.8 Hz, 2H), 6.79-6.92 (m, 6H); EI-MS *m*/*z* 215 (M⁺).

### A3b. General Method for Substituted Aniline Formation via Nitroarene Formation Through Nucleophilic Aromatic Substitution, Followed by Reduction

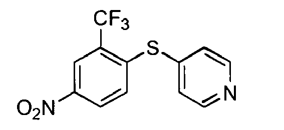

**Step 1. 3-(Trifluoromethyl)-4-(4-pyridinylthio)nitrobenzene:** A solution of 4-mercaptopyridine (2.8 g, 24 mmoles), 2-fluoro-5-nitrobenzotrifluoride (5 g, 23.5 mmoles), and potassium carbonate (6.1 g, 44.3 mmoles) in anhydrous DMF (80 mL) was stirred at room temperature and under argon overnight. TLC showed complete reaction. The mixture was diluted with Et₂O (100 mL) and water (100 mL) and the aqueous layer was back-extracted with Et₂O (2 x 100 mL). The organic layers were washed with a saturated NaCl solution (100 mL), dried (MgSO₄), and concentrated under reduced pressure. The solid residue was triturated with Et₂O to afford the desired product as a tan solid (3.8 g, 54%): TLC (30% EtOAc/70% hexane) R*_{f}* 0.06; ¹H-NMR (DMSO-d₆) * 7.33 (dd, *J*=1.2, 4.2 Hz, 2H), 7.78 (d, *J*=8.7 Hz, 1H), 8.46 (dd, *J*=2.4, 8.7Hz, 1H), 8.54-8.56 (m, 3H).

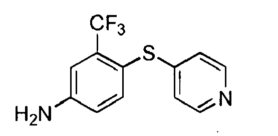

**Step 2. 3-(Trifluoromethyl)-4-(4-pyridinylthio)aniline:** A slurry of3-trifluoromethyl-4-(4-pyridinylthio)nitrobenzene (3.8 g, 12.7 mmol), iron powder (4.0 g, 71.6 mmol), acetic acid (100 mL), and water (1 mL) were stirred at room temp. for 4 h. The mixture was diluted with Et₂O (100 mL) and water (100 mL). The aqueous phase was adjusted to pH 4 with a 4 N NaOH solution. The combined organic layers were washed with a saturated NaCl solution (100 mL), dried (MgSO₄), and concentrated under reduced pressure. The residue was filtered through a pad of silica (gradient from 50% EtOAc/50% hexane to 60% EtOAc/40% hexane) to afford the desired product (3.3 g): TLC (50% EtOAc/50% hexane) R*_{f}* 0.10; ¹H-NMR (DMSO-d₆) * 6.21 (s, 2H), 6.84-6.87 (m, 3H), 7.10 (d, *J*=2.4 Hz, 1H), 7.39 (d, *J*=8.4 Hz, 1H), 8.29 (d, *J*=6.3 Hz, 2H).

### A3c. General Method for Substituted Aniline Formation via Nitroarene Formation Through Nucleophilic Aromatic Substitution, Followed by Reduction

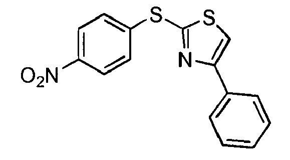

**Step 1. 4-(2-(4-Phenyl)thiazolyl)thio-1-nitrobenzene:** A solution of 2-mercapto-4-phenylthiazole (4.0 g, 20.7 mmoles) in DMF (40 mL) was treated with 1-fluoro-4-nitrobenzene (2.3 mL, 21.7 mmoles) followed by K₂CO₃ (3.18 g, 23 mmol), and the mixture was heated at approximately 65 °C overnight. The reaction mixture was then diluted with EtOAc (100 mL), sequentially washed with water (100 mL) and a saturated NaCl solution (100 mL), dried (MgSO₄) and concentrated under reduced pressure. The solid residue was triturated with a Et₂O/hexan solution to afford the desired product (6.1 g): TLC (25% EtOAc/75% hexane) R*_{f}* 0.49; ¹H-NMR (CDCl3) * 7.35-7.47 (m, 3H), 7.58-7.63 (m, 3H), 7.90 (d, *J*=6.9 Hz, 2H), 8.19 (d, *J*=9.0 Hz, 2H).

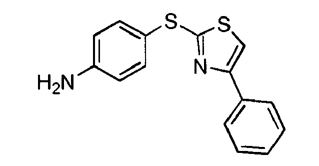

**Step 2. 4-(2-(4-Phenyl)thiazolyl)thioaniline:** 4-(2-(4-Phenyl)thiazolyl)thio-1-nitrobenzene was reduced in a manner analagous to that used in the preparation of 3-(trifluoromethyl)-4-(4-pyridinylthio)aniline: TLC (25% EtOAc/75% hexane) R*_{f}* 0.18; ¹H-NMR (CDCl₃) * 3.89 (br s, 2H), 6.72-6.77 (m, 2H), 7.26-7.53 (m, 6H), 7.85-7.89 (m, 2H).

### A3d. General Method for Substituted Aniline Formation via Nitroarene Formation Through Nucleophilic Aromatic Substitution, Followed by Reduction

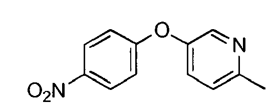

**Step 1. 4-(6-Methyl-3-pyridinyloxy)-1-nitrobenzene:** To a solution of 5-hydroxy-2-methylpyridine (5.0 g, 45.8 mmol) and 1-fluoro-4-nitrobenzene (6.5 g, 45.8 mmol) in anh DMF (50 mL) was added K₂CO₃ (13.0 g, 91.6 mmol) in one portion. The mixture was heated at the reflux temp. with stirring for 18 h and then allowed to cool to room temp. The resulting mixture was poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* to afford the desired product (8.7 g, 83%). This material was carried to the next step without further purification.

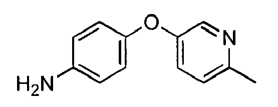

**Step 2. 4-(6-Methyl-3-pyridinyloxy)aniline:** A solution of 4-(6-methyl-3-pyridinyloxy)-1-nitrobenzene (4.0 g, 17.3 mmol) in EtOAc (150 mL) was added to 10% Pd/C (0.500 g, 0.47 mmol) and the resulting mixture was placed under a H₂ atmosphere (balloon) and was allowed to stir for 18 h at room temp. The mixture was then filtered through a pad of Celite^{®} and concentrated *in vacuo* to afford the desired product as a tan solid (3.2 g, 92%): EI-MS *m*/*z* 200 (M⁺).

### A3e. General Method for Substituted Aniline Formation via Nitroarene Formation Through Nucleophilic Aromatic Substitution, Followed by Reduction

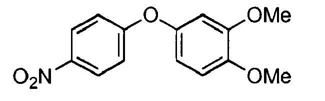

**Step 1. 4-(3,4-Dimethoxyphenoxy)-1-nitrobenzene:** To a solution of 3,4-dimethoxyphenol (1.0 g, 6.4 mmol) and 1-fluoro-4-nitrobenzene (700 µL, 6.4 mmol) in anh DMF (20 mL) was added K₂CO₃ (1.8 g, 12.9 mmol) in one portion. The mixture was heated at the reflux temp with stirring for 18 h and then allowed to cool to room temp. The mixture was then poured into water (100 mL) and extracted with EtOAc (3 x 100 mL). The combined organics were sequentially washed with water (3 x 50 mL) and a saturated NaCl solution (2 x 50 mL), dried (Na₂SO₄), and concentrated *in vacuo* to afford the desired product (0.8 g, 54%). The crude product was carried to the next step without further purification.

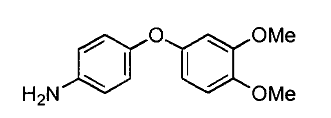

**Step 2. 4-(3,4-Dimethoxyphenoxy)aniline:** A solution of 4-(3,4-dimethoxy-phenoxy)-1-nitrobenzene (0.8 g, 3.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a white solid (0.6 g, 75%): EI-MS *m*/*z* 245 (M⁺).

### A3f. General Method for Substituted Aniline Formation via Nitroarene Formation Through Nucleophilic Aromatic Substitution, Followed by Reduction

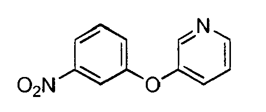

**Step 1. 3-(3-Pyridinyloxy)-1-nitrobenzene:** To a solution of 3-hydroxypyridine (2.8 g, 29.0 mmol), 1-bromo-3-nitrobenzene (5.9 g, 29.0 mmol) and copper(I) bromide (5.0 g, 34.8 mmol) in anh DMF (50 mL) was added K₂CO₃ (8.0 g, 58.1 mmol) in one portion. The resulting mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (2.0 g, 32 %). This material was used in the next step without further purification.

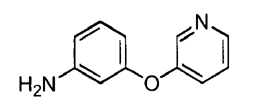

**Step 2. 3-(3-Pyridinyloxy)aniline:** A solution of 3-(3-pyridinyloxy)-1-nitrobenzene (2.0 g, 9.2 mmol) in EtOAc (100 mL) was added to 10% Pd/C (0.200 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (1.6 g, 94%): EI-MS *m*/*z* 186 (M⁺).

### A3g. General Method for Substituted Aniline Formation via Nitroarene Formation Through Nucleophilic Aromatic Substitution, Followed by Reduction

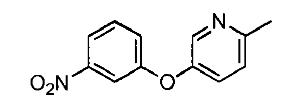

**Step 1. 3-(5-Methyl-3-pyridinyloxy)-1-nitrobenzene:** To a solution of 3-hydroxy-5-methylpyridine (5.0 g, 45.8 mmol), 1-bromo-3-nitrobenzene (12.0 g, 59.6 mmol) and copper(I) iodide (10.0 g, 73.3 mmol) in anh DMF (50 mL) was added K₂CO₃ (13.0 g, 91.6 mmol) in one portion. The mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (1.2 g, 13%).

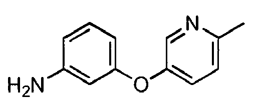

**Step 2. 3-(5-Methyl-3-pyridinyloxy)-1-nitrobenzene:** A solution of 3-(5-methyl-3-pyridinyloxy)-1-nitrobenzene (1.2 g, 5.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and *concentrated in vacuo* to afford the desired product as a red oil (0.9 g, 86%): CI-MS *m*/*z* 201 ((M+H)⁺).

### A3h. General Method for Substituted Aniline Formation via Nitroarene Formation Through Nucleophilic Aromatic Substitution, Followed by Reduction

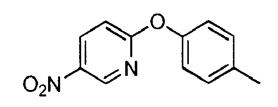

**Step 1. 5-Nitro-2-(4-methylphenoxy)pyridine:** To a solution of 2-chloro-5-nitropyridine (6.34 g, 40 mmol) in DMF (200 mL) were added of 4-methylphenol (5.4 g, 50 mmol, 1.25 equiv) and K₂CO₃ (8.28 g, 60 mmol, 1.5 equiv). The mixture was stirred overnight at room temp. The resulting mixture was treated with water (600 mL) to generate a precipitate. This mixture was stirred for 1 h, and the solids were separated and sequentially washed with a 1 N NaOH solution (25 mL), water (25 mL) and pet ether (25 mL) to give the desired product (7.05 g, 76%): mp 80-82 °C; TLC (30% EtOAc/70% pet ether) R*_{f}* 0.79; ¹H-NMR (DMSO-d₆) * 2.31 (s, 3H), 7.08 (d, *J*=8.46 Hz, 2H), 7.19 (d, *J*=9.20 Hz, 1H), 7.24 (d, *J*=8.09 Hz, 2H), 8.58 (dd, *J*=2.94, 8.82 Hz, 1H), 8.99 (d, *J*=2.95 Hz, 1H); FAB-MS *m*/*z* (rel abundance) 231 ((M+H)⁺), 100%).

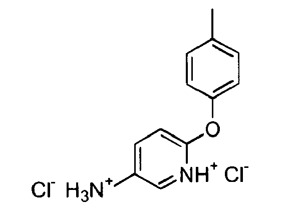

**Step 2. 5-Amino-2-(4-methylphenoxy)pyridine Dihydrochloride:** A solution 5-nitro-2-(4-methylphenoxy)pyridine (6.94 g, 30 mmol, 1 eq) and EtOH (10 mL) in EtOAc (190 mL) was purged with argon then treated with 10% Pd/C (0.60 g). The reaction mixture was then placed under a H₂ atmosphere and was vigorously stirred for 2.5 h. The reaction mixture was filtered through a pad of Celite^{®}. A solution of HCl in Et₂O was added to the filtrate was added dropwise. The resulting precipitate was separated and washed with EtOAc to give the desired product (7.56 g, 92%): mp 208-210 °C (dec); TLC (50% EtOAc/50% pet ether) R*_{f}* 0.42; ¹H-NMR (DMSO-d₆) 2.25 (s, 3H), 6.98 (d, *J*=8.45 Hz, 2H), 7.04 (d, *J*=8.82 Hz, 1H), 7.19 (d, *J*=8.09 Hz, 2H), 8.46 (dd, *J*=2.57, 8.46 Hz, 1H), 8.63 (d, *J*=2.57 Hz, 1H); EI-MS *m*/*z* (rel abundance) (M⁺, 100%).

### A3i. General Method for Substituted Aniline Formation via Nitroarene Formation Through Nucleophilic Aromatic Substitution, Followed by Reduction

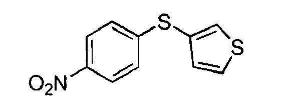

**Step 1. 4-(3-Thienylthio)-1-nitrobenzene:** To a solution of4-nitrothiophenol (80%pure; 1.2 g, 6.1 mmol), 3-bromothiophene (1.0 g, 6.1 mmol) and copper(II) oxide (0.5 g, 3.7 mmol) in anhydrous DMF (20 mL) was added KOH (0.3 g, 6.1 mmol), and the resulting mixture was heated at 130 °C with stirring for 42 h and then allowed to cool to room temp. The reaction mixture was then poured into a mixture of ice and a 6N HCl solution (200 mL) and the resulting aqueous mixture was extracted with EtOAc (3 x 100 mL). The combined organic layers were sequentially washed with a 1M NaOH solution (2 x 100 mL) and a saturated NaCl solution (2 x 100 mL), dried (MgSO₄) and tertconcentrated *in vacuo .* The residual oil was purified by MPLC (silica gel; gradient from 10% EtOAc/90% hexane to 5% EtOAc/95% hexane) to afford of the desired product (0.5 g, 34%). GC-MS *m*/*z* 237 (M⁺).

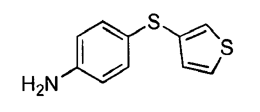

**Step 2. 4-(3-Thienylthio)aniline:** 4-(3-Thienylthio)-1-nitrobenzene was reduced to the aniline in a manner analogous to that described in Method A1.

### A3j. General Method for Substituted Aniline Formation via Nitroarene Formation Through Nucleophilic Aromatic Substitution, Followed by Reduction

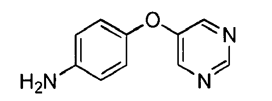

**4-(5-Pyrimininyloxy)aniline:** 4-Aminophenol (1.0 g, 9.2 mmol) was dissolved in DMF (20 mL) then 5-bromopyrimidine (1.46 g, 9.2 mmol) and K₂CO₃ (1.9 g, 13.7 mmol) were added. The mixture was heated to 100 °C for 18 h and at 130°C for 48 h at which GC-MS analysis indicated some remaining starting material. The reaction mixture was cooled to room temp. and diluted with water (50 mL). The resulting solution was extracted with EtOAc(100 mL). The organic layer was washed with a saturated NaCl solution (2 x 50 mL), dried (MgSO₄) and concentrated *in vacuo.* The residual solids were purified by MPLC (50% EtOAc/50% hexanes) to give the desired amine (0.650 g, 38%).

### A3k. General Method for Substituted Aniline Formation via Nitroarene Formation Through Nucleophilic Aromatic Substitution, Followed by Reduction

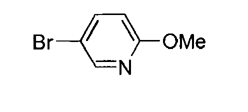

**Step 1. 5-Bromo-2-methoxypyridine:** A mixture of 2,5-dibromopyridine (5.5 g, 23.2 mmol) and NaOMe (3.76g, 69.6 mmol) in MeOH (60 mL) was heated at 70 °C in a sealed reaction vessel for 42 h, then allowed to cool to room temp. The reaction mixture was treated with water (50 mL) and extracted with EtOAc (2 x 100 mL). The combined organic layers were dried (Na₂SO₄) and concentrated under reduced pressure to give a pale yellow, volatile oil (4.1g, 95% yield): TLC (10% EtOAc / 90% hexane) R*_{ƒ}*0.57.

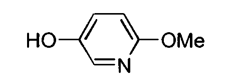

**Step 2. 5-Hydroxy-2-methoxypyridine:** To a stirred solution of 5-bromo-2-methoxypyridine (8.9 g, 47.9 mmol) in THF (175 mL) at -78 °C was added an n-butyllithium solution (2.5 M in hexane; 28.7 mL, 71.8 mmol) dropwise and the resulting mixture was allowed to stir at -78 °C for 45 min. Trimethyl borate (7.06 mL, 62.2 mmol) was added via syringe and the resulting mixture was stirred for an additional 2 h. The bright orange reaction mixture was warmed to 0 °C and was treated with a mixture of a 3 N NaOH solution (25 mL, 71.77 mmol) and a hydrogen peroxide solution (30%; approx. 50 mL). The resulting yellow and slightly turbid reaction mixture was warmed to room temp. for 30 min and then heated to the reflux temp. for 1 h. The reaction mixture was then allowed to cool to room temp. The aqueous layer was neutralized with a 1N HCl solution then extracted with Et₂O (2 x 100 mL). The combined organic layers were dried (Na₂SO₄) and concentrated under reduced pressure to give a viscous yellow oil (3.5g, 60%).

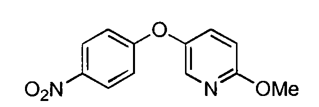

**Step 3. 4-(5-(2-Methoxy)pyridyl)oxy-1-nitrobenzene:** To a stirred slurry of NaH (97%, 1.0 g, 42 mmol) in anh DMF (100 mL) was added a solution of 5-hydroxy-2-methoxypyridine (3.5g, 28 mmol) in DMF (100 mL). The resulting mixture was allowed to stir at room temp. for 1 h, 4-fluoronitrobenzene (3 mL, 28 mmol) was added via syringe. The reaction mixture was heated to 95 °C overnight, then treated with water (25 mL) and extracted with EtOAc (2 x 75 mL). The organic layer was dried (MgSO₄ and concentrated under reduced pressure. The residual brown oil was crystalized EtOAc/hexane) to afford yellow crystals (5.23 g, 75%).

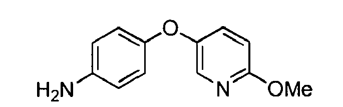

**Step 4. 4-(5-(2-Methoxy)pyridyl)oxyaniline:** 4-(5-(2-Methoxy)pyridyl)oxy-1-nitrobenzene was reduced to the aniline in a manner analogous to that described in Method A3d, Step2.

### A4a. General Method for Substituted Aniline Synthesis via Nucleophilic Aromatic Substitution using a Halopyridine

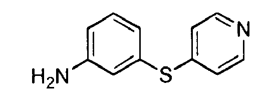

**3-(4-Pyridinylthio)aniline:** To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄) and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R*_{ƒ}*0.29;¹H-NMR (DMSO-d₆) * 5.41 (s, 2H), 6.64-6.74 (m, 3H), 7.01 (d, J=4.8, 2H), 7.14 (t, J=7.8 Hz, 1H), 8.32 (d, J=4.8, 2H).

### A4b. General Method for Substituted Aniline Synthesis via Nucleophilic Aromatic Substitution using a Halopyridine

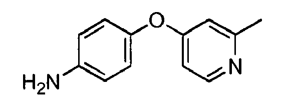

**4-(2-Methyl-4-pyridinyloxy)aniline:** To a solution of 4-aminophenol (3.6 g, 32.8 mmol) and 4-chloropicoline (5.0 g, 39.3 mmol) in anh DMPU (50 mL) was added potassium *tert-*butoxide (7.4 g, 65.6 mmol) in one portion. The reaction mixture was heated at 100°C with stirring for 18 h, then was allowed to cool to room temp. The resulting mixture was poured into water (200 mL) and extracted with EtOAc (3 x 150 mL). The combined extracts were sequentially washed with water (3 x 100 mL) and a saturated NaCl solution (2 x 100 mL), dried (Na₂SO₄), and concentrated *in vacuo.* The resulting oil was purified by flash chromatography (50 % EtOAc/50% hexane) to afford the desired product as a yellow oil (0.7 g, 9%): CI-MS *m*/*z* 201 ((M+H)⁺).

### A4c. General Method for Substituted Aniline Synthesis via Nucleophilic Aromatic Substitution using a Halopyridine

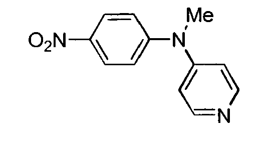

**Step 1. Methyl(4-nitrophenyl)-4-pyridylamine:** To a suspension of *N-*methyl-4-nitroaniline (2.0 g, 13.2 mmol) and K₂CO₃ (7.2 g, 52.2 mmol) in DMPU (30mL) was added 4-chloropyridine hydrochloride (2.36 g, 15.77 mmol). The reaction mixture was heated at 90°C for 20 h, then cooled to room temperature. The resulting mixture was diluted with water (100 mL) and extracted with EtOAc (100 mL). The organic layer was washed with water (100 mL), dried (Na₂SO₄) and concentrated under reduced pressure. The residue was purified by column chromatography (silica gel, gradient from 80% EtOAc /20% hexanes to 100% EtOAc) to afford methyl(4-nitrophenyl)-4-pyridylamine (0.42 g)

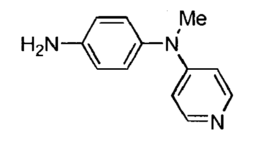

**Step 2. Methyl(4-aminophenyl)-4-pyridylamine:** Methyl(4-nitrophenyl)-4-pyridylamine was reduced in a manner analogous to that described in Method A1.

### A5. General Method of Substituted Aniline Synthesis via Phenol Alkylation Followed by Reduction of a Nitroarene

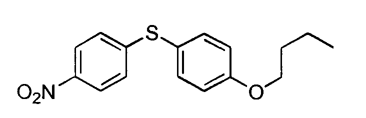

**Step 1. 4-(4-Butoxyphenyl)thio-1-nitrobenzene:** To a solution of 4-(4-nitrophenyl-thio)phenol (1.50 g, 6.07 mmol) in anh DMF (75 ml) at 0 °C was added NaH (60% in mineral oil, 0.267 g, 6.67 mmol). The brown suspension was stirred at 0 °C until gas evolution stopped (15 min), then a solution of iodobutane (1.12 g, .690 ml, 6.07 mmol) in anh DMF (20 mL) was added dropwise over 15 min at 0 °C. The reaction was stirred at room temp. for 18 h at which time TLC indicated the presence ofunreacted phenol, and additional iodobutane (56 mg, 0.035 mL, 0.303 mmol, 0.05 equiv) and NaH (13 mg, 0.334 mmol) were added. The reaction was stirred an additional 6 h at room temp., then was quenched by the addition of water (400 mL). The resulting mixture was extracted with Et₂O (2 x 500 mL). The combined organics were washed with water (2 x 400 mL), dried (MgSO₄) and concentrated under reduced pressure to give a clear yellow oil, which was purified by silica gel chromatography (gradient from 20% EtOAc/80% hexane to 50% EtOAc/50% hexane) to give the product as a yellow solid (1.24 g, 67%): TLC (20% EtOAc/80% hexane) R*_{ƒ}*0.75; ¹H-NMR (DMSO-d₆) * 0.92 (t, *J*= 7.5 Hz, 3H), 1.42 (app hex, *J*=7.5 Hz, 2H), 1.70 (m, 2H), 4.01 (t, *J=* 6.6 Hz, 2H), 7.08 (d, *J*=8.7 Hz, 2H), 7.17 (d, *J*=9 Hz, 2H), 7.51 (d, *J*= 8.7 Hz, 2H), 8.09 (d, *J=* 9 Hz, 2H).

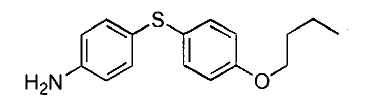

**Step 2. 4-(4-Butoxyphenyl)thioaniline:** 4-(4-Butoxyphenyl)thio-1-nitrobenzene was reduced to the aniline in a manner analagous to that used in the preparation of 3-(trifluoromethyl)-4-(4-pyridinylthio)aniline (Method A3b, Step 2): TLC (33% EtOAc/77% hexane) R*_{ƒ}*0.38.

### A6. General Method for Synthesis of Substituted Anilines by the Acylation of Diaminoarenes

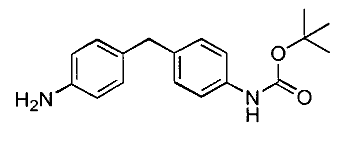

**4-(4-*tert*-Butoxyearbamoylbenzyl)aniline:** To a solution of 4,4'-methylenedianiline (3.00 g, 15.1 mmol) in anh THF (50 mL) at room temp was added a solution of di-*tert-*butyl dicarbonate (3.30 g, 15.1 mmol) in anh THF (10 mL). The reaction mixture was heated at the reflux temp. for 3 h, at which time TLC indicated the presence of unreacted methylenedianiline. Additional di-*tert-*butyl dicarbonate (0.664 g, 3.03 mmol, 0.02 equiv) was added and the reaction stirred at the reflux temp. for 16 h. The resulting mixture was diluted with Et₂O (200 mL), sequentially washed with a saturated NaHCO₃ solution (100 ml), water (100 mL) and a saturated NaCl solution (50 mL), dried (MgSO₄), and concentrated under reduced pressure. The resulting white solid was purified by silica gel chromatography (gradient from 33% EtOAc/67% hexane to 50% EtOAc/50% hexane) to afford the desired product as a white solid (2.09 g, 46%): TLC (50% EtOAc/50% hexane) *R_{f}*0.45; ¹H-NMR (DMSO-d₆) * 1.43 (s, 9H), 3.63 (s, 2H), 4.85 (br s, 2H), 6.44 (d, *J*=8.4 Hz, 2H), 6.80 (d, *J*=8.1 Hz, 2H), 7.00 (d, *J*=8.4 Hz, 2H), 7.28 (d, *J*=8.1 Hz, 2H), 9.18 (br s, 1H); FAB-MS *m*/*z* 298 (M⁺).

### A7. General Method for the Synthesis of Aryl Amines via Electrophilic Nitration Followed by Reduction

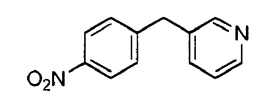

**Step 1. 3-(4-Nitrobenzyl)pyridine:** A solution of 3-benzylpyridine (4.0 g, 23.6 mmol) and 70% nitric acid (30 mL) was heated overnight at 50°C. The resulting mixture was allowed to cool to room temp. then poured into ice water (350 mL). The aqueous mixture then made basic with a 1N NaOH solution, then extracted with Et₂O (4 x 100 mL). The combined extracts were sequentially washed with water (3 x 100 mL) and a saturated NaCl solution (2 x 100 mL), dried (Na₂SO₄) and concentrated *in vacuo.* The residual oil was purified by MPLC (silica gel; 50 % EtOAc/50% hexane) then recrystallization (EtOAc/hexane) to afford the desired product (1.0 g, 22%): GC-MS *m*/*z* 214 (M⁺).

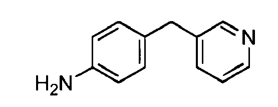

**Step 2. 3-(4-Pyridinyl)methylaniline:** 3-(4-Nitrobenzyl)pyridine was reduced to the aniline in a manner analogous to that described in Method A1**.**

### A8. General Method for Synthesis of Aryl Amines via Substitution with Nitrobenzyl Halides Followed by Reduction

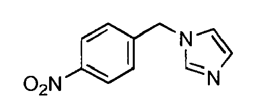

**Step 1. 4-(1-Imidazolylmethyl)-1-nitrobenzene:** To a solution of imidazole (0.5 g, 7.3 mmol) and 4-nitrobenzyl bromide (1.6 g, 7.3 mmol) in anh acetonitrile (30 mL) was added K₂CO₃ (1.0 g, 7.3 mmol). The resulting mixture was stirred at room temp. for 18 h and then poured into water (200 mL) and the resulting aqueous solution was extracted with EtOAc (3 x 50 mL). The combined organic layers were sequentially washed with water (3 x 50 mL) and a saturated NaCl solution (2 x 50 mL), dried (MgSO₄) and concentrated *in vacuo.* The residual oil was purified by MPLC (silica gel; 25% EtOAc/75% hexane) to afford the desired product (1.0 g, 91%): EI-MS *m*/*z* 203 (M⁺).

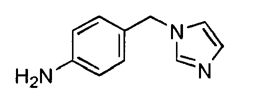

**Step 2. 4-(1-Imidazolylmethyl)aniline:** 4-(1-Imidazolylmethyl)-1-nitrobenzene was reduced to the aniline in a manner analogous to that described in Method A2.

### A9. Formation of Substituted Hydroxymethylanilines by Oxidation of Nitrobenzyl Compounds Followed by Reduction

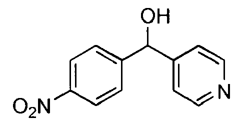

**Step 1. 4-(1-Hydroxy-1-(4-pyridyl)methyl-1-nitrobenzene:** To a stirred solution of 3-(4-nitrobenzyl)pyridine (6.0 g, 28 mmol) in CH₂Cl₂ (90 mL) was added *m*-CPBA (5.80 g, 33.6 mmol) at 10°C, and the mixture was stirred at room temp. overnight. The reaction mixture was successively washed with a 10% NaHSO₃ solution (50 mL), a saturated K₂CO₃ solution (50 mL) and a saturated NaCl solution (50 mL), dried (MgSO₄) and concentrated under reduced pressure. The resulting yellow solid (2.68 g) was dissolved in anh acetic anhydride (30 mL) and heated at the reflux temperature overnight. The mixture was concentrated under reduced pressure. The residue was dissolved in MeOH (25 mL) and treated with a 20% aqueous NH₃ solution (30 mL). The mixture was stirred at room temp. for 1 h, then was concentrated under reduced pressure. The residue was poured into a mixture of water (50 mL) and CH₂Cl₂ (50 mL). The organic layer was dried (MgSO₄), concentrated under reduced pressure, and purified by column chromatography (80% EtOAc/ 20% hexane) to afford the desired product as a white solid. (0.53 g, 8%): mp 110-118 °C; TLC (80% EtOAc/20% hexane) R*_{f}*0.12*;* FAB-MS *m*/*z* 367 ((M+H)⁺, 100%).

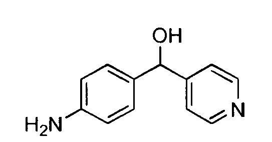

**Step 2. 4-(1-Hydroxy-1-(4-pyridyt)methytaniline:** 4-(1-Hydroxy-1-(4-pyridyl)-methyl-1-nitrobenzene was reduced to the aniline in a manner analogous to that described in Method A3d, Step2.

### A10. Formation of 2-(N-methylcarbamoyl)pyridines via the Menisci reaction

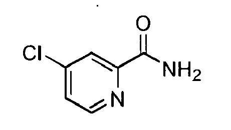

**Step 1. 2-(*N-*methylcarbamoyl)-4-chloropyridine.** (**Caution:** this is a highly hazardous, potentially explosive reaction.) To a solution of 4-chloropyridine (10.0 g) in *N-*methylformamide (250 mL) under argon at ambient temp was added conc. H₂SO₄ (3.55 mL) (exotherm). To this was added H₂O₂ (17 mL, 30% wt in H₂O followed by FeSO₄ 7H₂O (0.55 g) to produce an exotherm. The reaction was stirred in the dark at ambient temp for 1h then was heated slowly over 4 h at 45°C. When bubbling subsided,the reaction was heated at 60°C for 16 h. The resulting opaque brown solution was diluted with H₂O (700 mL) followed by a 10% NaOH solution (250 mL). The resulting aqueous mixture was extracted with EtOAc (3 x 500 mL) and the organic layers were washed separately with a saturated NaCl solution (3 x 150 mL). The combined organics phases were dried (MgSO₄) and filtered through a pad of silica gel eluting with EtOAc. The solvent was removed in vacuo and the brown residue was purified by silica gel chromatography (gradient from 50% EtOAc / 50% hexane to 80% EtOAc / 20% hexane). The resulting yellow oil crystallized at 0 °C over 72 h to give 2-(*N-*methylcarbamoyl)-4-chloropyridine in yield (0.61 g, 5.3%): TLC (50% EtOAc/50% hexane) R*_{ƒ}*0.50; MS; ¹H NMR (CDCl₃): d 8.44 (d, 1H, J= 5.1 Hz, CHN), 8.21 (s, 1H, CHCCO), 7.96 (b s, 1H, NH), 7.43 (dd, 1H, J = 2.4, 5.4 Hz, ClCHCN), 3.04 (d, 3H, J = 5.1 Hz, methyl); CI-MS *m*/*z* 171 ((M+H)+).

### Step 1b. Synthesis of 4-chloropyridine-2-carbonyl chloride HCl salt via picolinic acid

Anhydrous DMF (6.0 mL) was slowly added to SOCl₂ (180 mL) between 40° and 50°C. The solution was stirred in that temperature range for 10 min. then picolinic acid (60.0 g, 487 mmol) was added in portions over 30 min. The resulting solution was heated at 72°C (vigorous SO₂ evolution) for 16 h to generate a yellow solid precipitate. The resulting mixture was cooled to room temp., diluted with toluene (500 mL) and concentrated to 200 mL. The toluene addition/concentration process was repeated twice. The resulting nearly dry residue was filtered and the solids were washed with toluene (2 x 200 mL) and dried under high vacuum for 4 h to afford 4-chloropyridine-2-carbonyl chloride HCl salt as a yellow-orange solid (92.0 g, 89%).

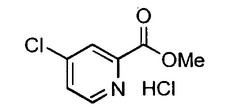

### Step 2. Synthesis of methyl 4-chloropyridine-2-carboxylate HCl salt

Anh DMF (10.0 mL) was slowly added to SOCl₂ (300 mL) at 40-48 °C. The solution was stirred at that temp. range for 10 min., then picolinic acid (100 g, 812 mmol) was added over 30 min. The resulting solution was heated at 72 °C (vigorous SO₂ evolution) for 16 h to generate a yellow solid. The resulting mixture was cooled to room temp., diluted with toluene (500 mL) and concentrated to 200 mL. The toluene addition/concentration process was repeated twice. The resulting nearly dry residue was filtered, and the solids were washed with toluene (50 mL) and dried under high vacuum for 4 hours to afford 4-chloropyridine-2-carbonyl chloride HCl salt as an off-white solid (27.2 g, 16%). This material was set aside.

The red filtrate was added to MeOH (200 mL) at a rate which kept the internal temperature below 55°C. The contents were stirred at room temp. for 45 min., cooled to 5 °C and treated with Et₂O (200 mL) dropwise. The resulting solids were filtered, washed with Et₂O (200 mL) and dried under reduced pressure at 35°C to provide methyl 4-chloropyridine-2-carboxylate HCl salt as a white solid (110 g, 65%): mp 108-112 °C; ¹H-NMR (DMSO-d₆) * 3.88 (s, 3H); 7.82 (dd, *J*=5.5*,* 2.2 Hz, 1H); 8.08 (d, *J*=2.2 Hz, 1H); 8.68 (d, *J*=5.5 Hz, 1H); 10.68 (br s, 1H); HPLC ES-MS *m*/*z* 172 ((M+H)⁺).

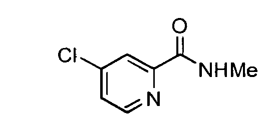

### Step 3a. Synthesis of 4-chloro-N-methyl-2-pyridinecarboxamide from methyl 4-chloropyridine-2-carboxylate

A suspension of methyl 4-chloropyridine-2-carboxylate HCl salt (89.0 g, 428 mmol) in MeOH (75 mL) at 0 °C was treated with a 2.0 M methylamine solution in THF (1 L) at a rate which kept the internal temp. below 5°C. The resulting mixture was stored at 3 °C for 5 h, then concentrated under reduced pressure. The resulting solids were suspended in EtOAc (1 L) and filtered. The filtrate was washed with a saturated NaCl solution (500 mL), dried (Na₂SO₄ and concentrated under reduced pressure to afford 4-chloro-*N-*methyl-2-pyridinecarboxamide as pale-yellow crystals (71.2 g, 97%): mp 41-43 °C; ¹H-NMR (DMSO-d₆) * 2.81 (s, 3H), 7.74 (dd, *J*=5.1, 2.2 Hz, 1H), 8.00 (d, *J*=2.2, 1H), 8.61 (d, *J*=5.1 Hz, 1H), 8.85 (br d, 1H); CI-MS *m*/*z* 171 ((M+H)⁺).

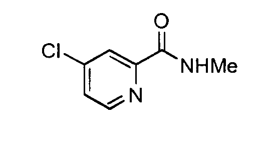

### Step 3b. Synthesis of 4-chloro-N-methyl-2-pyridinecarboxamide from 4-chloropyridine-2-carbonyl chloride

4-Chloropyridine-2-carbonyl chloride HCl salt (7.0 g, 32.95 mmol) was added in portions to a mixture of a 2.0 M methylamine solution in THF (100 mL) and MeOH (20 mL) at 0 °C. The resulting mixture was stored at 3 °C for 4 h, then concentrated under reduced pressure. The resulting nearly dry solids were suspended in EtOAc (100 mL) and filtered. The filtrate was washed with a saturated NaCl solution (2 x 100 mL), dried (Na₂SO₄) and concentrated under reduced pressure to provide 4-chloro-*N* methyl-2-pyridinecarboxamide as a yellow, crystalline solid (4.95 g, 88%): mp 37-40 °C.

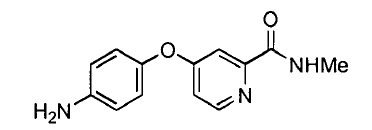

### Step 4. Synthesis of 4-(2-(N-methylcarbamoyl)-4-pyridyloxy)aniline

A solution of 4-aminophenol (9.60 g, 88.0 mmol) in anh. DMF (150 mL) was treated with potassium *tert-*butoxide (10.29 g, 91.7 mmol), and the reddish-brown mixture was stirred at room temp. for 2 h. The contents were treated with 4-chloro-*N*-methyl-2-pyridinecarboxamide (15.0 g, 87.9 mmol) and K₂CO₃ (6.50 g, 47.0 mmol) and then heated at 80°C for 8 h. The mixture was cooled to room temp. and separated between EtOAc (500 mL) and a saturated NaCl solution (500 mL). The aqueous phase was back-extracted with EtOAc (300 mL). The combined organic layers were washed with a saturated NaCl solution (4 x 1000 mL), dried (Na₂SO₄) and concentrated under reduced pressure. The resulting solids were dried under reduced pressure at 35 °C for 3 h to afford 4-(2-(*N*-methylcarbamoyl)-4-pyridyloxy)aniline as a light-brown solid 17.9 g, 84%): ¹H-NMR (DMSO-d₆) * 2.77 (d, *J*=4.8 Hz, 3H), 5.17 (br s, 2H), 6.64, 6.86 (AA'BB' quartet, *J*=8.4 Hz, 4H), 7.06 (dd, *J*=5.5, 2.5 Hz, 1H), 7.33 (d, *J*=2.5 Hz, 1H), 8.44 (d, *J*=5.5 Hz, 1H), 8.73 (br d, 1H); HPLC ES-MS *m*/*z* 244 ((M+H)⁺).

### A11. General Method for the Synthesis of 5-(4-Aminophenoxy)isoindoline-1,3-dione

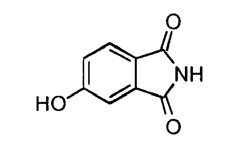

### Step 1. Synthesis of 5-hydroxyisoindoline-1,3-dione

To a mixture of ammonium carbonate (5.28 g, 54.9 mmol) in conc. AcOH (25 mL) was slowly added 4-hydroxyphthalic acid (5.0 g, 27.45 mmol). The resulting mixture was heated at 120 °C for 45 min., then the clear, bright yellow mixture was heated at 160 °C for 2 h. The resulting mixture was maintained at 160 °C and was concentrated to approximately 15 mL, then was cooled to room temp. and adjusted pH 10 with a 1N NaOH solution. This mixture was cooled to 0 °C and slowly acidified to pH 5 using a 1N HCl solution. The resultant precipitate was collected by filtration and dried under reduced pressure to yield 5-hydroxyisoindoline-1,3-dione as a pale yellow powder as product (3.24 g, 72%): ¹H NMR (DMSO-d₆) * 7.00-7.03 (m, 2H), 7.56 (d, *J*=9.3Hz, 1H).

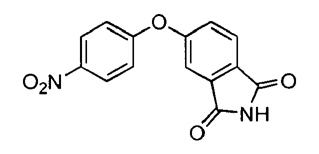

### Step 2. Synthesis of 5-(4-nitrophenoxy)isoindoline-1,3-dione

To a stirring slurry ofNaH (1.1 g, 44.9 mmol) in DMF (40 mL) at 0 °C was added a solution of 5-hydroxyisoindoline-1,3-dione (3.2 g, 19.6 mmol) in DMF (40 mL) dropwise. The bright yellow-green mixture was allowed to return to room temp. and was stirred for 1 h, then 1-fluoro-4-nitrobenzene (2.67 g, 18.7 mmol) was added via syringe in 3-4 portions. The resulting mixture was heated at 70 °C overnight, then cooled to room temp. and diluted slowly with water (150 mL), and extracted with EtOAc (2 x 100 mL). The combined organic layers were dried (MgSO₄ and concentrated under reduced pressure to give 5-(4-nitrophenoxy)isoindoline-1,3-dione as a yellow solid (3.3 g, 62%): TLC (30% EtOAc/70% hexane) R*_{ƒ}* 0.28; 1H NMR (DMSO-d₆) * 7.32 (d, *J*=12 Hz, 2H), 7.52-7.57 (m, 2H), 7.89(d, *J*=7.8 Hz, 1H), 8.29 (d, *J*=9 Hz, 2H), 11.43 (br s, 1H); CI-MS *m*/*z* 285 ((M+H)⁺, 100%).

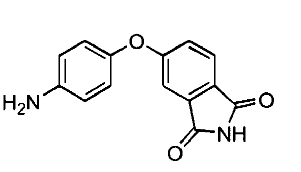

### Step 3. Synthesis of 5-(4-aminophenoxy)isoindoline-1,3-dione

A solution of 5-(4-nitrophenoxy)isoindoline-1,3-dione (0.6 g, 2.11 mmol) in conc. AcOH (12 mL) and water (0.1 mL) was stirred under stream of argon while iron powder (0.59 g, 55.9 mmol) was added slowly. This mixture stirred at room temp. for 72 h, then was diluted with water (25 mL) and extracted with EtOAc (3 x 50 mL). The combined organic layers were dried (MgSO₄) and concentrated under reduced pressure to give 5-(4-aminophenoxy)isoindoline-1,3-dione as a brownish solid (0.4 g, 75%): TLC (50% EtOAc/50% hexane) R*_{ƒ}* 0.27; ¹H NMR (DMSO-d₆) * 5.14 (br s, 2H), 6.62 (d, *J*=8.7 Hz, 2H), 6.84 (d, *J*=8.7 Hz, 2H), 7.03 (d, *J*=2.1 Hz, 1H), 7.23 (dd, 1H), 7.75 (d, *J*=8.4 Hz, 1H), 11.02 (s, 1H); HPLC ES-MS *m*/*z* 255 ((M+H)⁺, 100%).

### A12. General Method for the Synthesis of *-Sulfonylphenyl Anilines

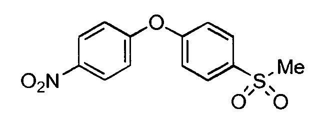

**Step 1. 4-(4-Methylsulfonylphenoxy)-1-nitrobenzene:** To a solution of 4-(4-methylthiophenoxy)-1-ntirobenzene (2 g, 7.66 mmol) in CH₂Cl₂ (75 mL) at 0 °C was slowly added *m*CPBA (57-86%, 4 g), and the reaction mixture was stirred at room temperature for 5 h. The reaction mixture was treated with a 1 N NaOH solution (25 mL). The organic layer was sequentially washed with a 1N NaOH solution (25 mL), water (25 mL) and a saturated NaCl solution (25 mL), dried (MgSO₄) and concentrated under reduced pressure to give 4-(4-methylsulfonylphenoxy)-1-nitrobenzene as a solid (2.1 g).

**Step 2. 4-(4-Methylsulfonylphenoxy)-1-aniline:** 4-(4-Methylsulfonylphenoxy)-1-nitrobenzene was reduced to the aniline in a manner anaologous to that described in Method A3d, step 2.

### A13. General Method for Synthesis of *-Alkoxy-*-carboxyphenyl Anilines

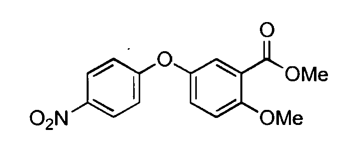

**Step 1. 4-(3-Methoxycarbonyl-4-methoxyphenoxy)-1-nitrobenzene**: To a solution of -(3-carboxy-4-hydroxyphenoxy)-1-nitrobenzene (prepared in a manner analogous to that described in Method A3a, step 1, 12 mmol) in acetone (50 mL) was added K₂CO₃ (5 g) and dimethyl sulfate (3.5 mL). The resulting mixture was heated at the reflux temperature overnight, then cooled to room temperature and filtered through a pad of Celite^{®}. The resulting solution was concentrated under reduced pressure, absorbed onto silica gel, and purified by column chromatography (50% EtOAc / 50% hexane) to give 4-(3-methoxycarbonyl-4-methoxyphenoxy)-1-nitrobenzene as a yellow powder (3 g): mp 115-118°C.

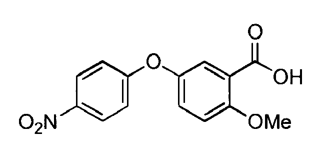

**Step 2. 4-(3-Carboxy-4-methoxyphenoxy)-1-nitrobenzene:** A mixture of 4-(3-methoxycarbonyl-4-methoxyphenoxy)-1-nitrobenzene (1.2 g), KOH (0.33 g),and water (5 mL) in MeOH (45 mL) was stirred at room temperature overnight and then heated at the reflux temperature for 4 h. The resulting mixture was cooled to room temperature and concentrated under reduced pressure. The residue was dissolved in water (50 mL), and the aqueous mixture was made acidic with a 1N HCl solution. The resulting mixture was extracted with EtOAc (50 mL). The organic layer was dried (MgSO₄) and concentrated under reduced pressure to give 4-(3-carboxy-4-methoxyphenoxy)-1-nitrobenzene (1.04 g).

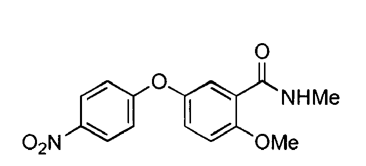

### Step 3. 4-(3-(N-Methylcarbamoly)-4-methoxyphenoxy)-1-nitrobenzene:

To a solution of 4-(3-carboxy-4-methoxyphenoxy)-1-nitrobenzene (0.50 g, 1.75 mmol) in CH₂Cl₂ (12 mL) was added SOCl₂ (0.64 mL, 8.77 mmol) in portions. The resulting solution was heated at the reflux temp. for 18 h, cooled to room temp., and concentrated under reduced pressure. The resulting yellow solids were dissolved in CH₂Cl₂ (3 mL) then the resulting solution was treated with a methylamine solution (2.0 M in THF, 3.5 mL, 7.02 mmol) in portions (CAUTION: gas evolution), and stirred at room temp. for 4 h. The resulting mixture was treated with a 1N NaOH solution, then extracted with CH₂Cl₂ (25 mL). The organic layer was dried (Na₂SO₄) and concentrated under reduced pressure to give 4-(3-(*N-*methylcarbamoly)-4-methoxyphenoxy)-1-nitrobenzene as a yellow solid (0.50 g, 95%).

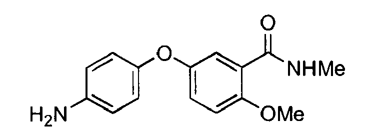

### Step 4. 4-(3-(N-Methylcarbamoly)-4-methoxyphenoxy)aniline:

A slurry of 4-(3-(*N-*methylcarbamoly)-4-methoxyphenoxy)-1-nitrobenzene (0.78 g, 2.60 mmol) and 10% Pd/C (0.20 g) in EtOH (55 mL) was stirred under 1 atm of H₂ (balloon) for 2.5 d, then was filtered through a pad of Celite^{®}. The resulting solution was concentrated under reduced pressure to afford 4-(3-(*N-*methylcarbamoly)-4-methoxyphenoxy)aniline as an off-white solid (0.68 g, 96%): TLC (0.1% Et₃N/99.9% EtOAc) R*_{ƒ}* 0.36.

### A14. General Method for the Synthesis of 4-(3-N-Methylcarbamoylphenoxy)aniline.

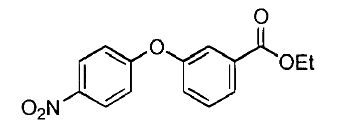

### Step 1. Synthesis of 4-(3-ethoxycarbonylphenoxy)-1-nitrobenzene

A mixture of 4-fluoro-1-nitrobenzene (16 mL, 150 mmol), ethyl 3-hydroxybenzoate 25 g, 150 mmol) and K₂CO₃ (41 g, 300 mmol) in DMF (125 mL) was heated at the reflux temp. overnight, cooled to room temp. and treated with water (250 mL). The resulting mixture was extracted with EtOAc (3 x 150 mL). The combined organic phases were sequentially washed with water (3 x 100 mL) and a saturated NaCl solution (2 x 100 mL), dried (Na₂SO₄) and concentrated under reduced pressure. The residue was purified by column chromatography (10% EtOAc/90% hexane) to afford 4-(3-ethoxycarbonylphenoxy)-1-nitrobenzene as an oil (38 g).

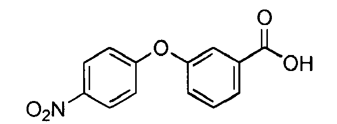

### Step 2. Synthesis of 4-(3-carboxyphenoxy)-1-nitrobenzene

To a vigorously stirred mixture of 4-(3-ethoxycarbonylphenoxy)-1-nitrobenzene (5.14 g, 17.9 mmol) in a 3:1 THF/water solution (75 mL) was added a solution LiOH•H₂O (1.50 g, 35.8 mmol) in water (36 mL). The resulting mixture was heated at 50 °C overnight, then cooled to room temp., concentrated under reduced pressure, and adjusted to pH 2 with a 1M HCl solution. The resulting bright yellow solids were removed by filtration and washed with hexane to give 4-(3-carboxyphenoxy)-1-nitrobenzene (4.40 g, 95%).

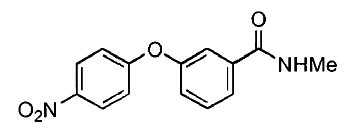

### Step 3. Synthesis of 4-(3-(N-methylcarbamoyl)phenoxy)-1-nitrobenzene

A mixture of 4-(3-carboxyphenoxy)-1-nitrobenzene (3.72 g, 14.4 mmol), EDCl·HCl (3.63 g, 18.6 mmol), *N-*methylmorpholine (1.6 mL, 14.5 mmol) and methylamine (2.0 M in THF; 8 mL, 16 mmol) in CH₂Cl₂ (45 mL) was stirred at room temp. for 3 d, then concentrated under reduced pressure. The residue was dissolved in EtOAc (50 mL) and the resulting mixture was extracted with a 1M HCl solution (50 mL). The aqueous layer was back-extracted with EtOAc (2 x 50 mL). The combined organic phases were washed with a saturated NaCl solution (50 mL), dried (Na₂SO₄), and concentrated under reduced pressure to give 4-(3-(*N*-methylcarbamoyl)phenoxy)-1-nitrobenzene as an oil (1.89 g).

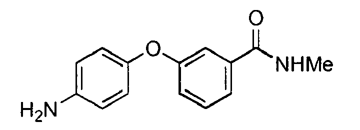

### Step 4. Synthesis of 4-(3-(N-methylcarbamoyl)phenoxy)aniline

A slurry of 4-(3-(*N*-methylcarbamoyl)phenoxy)-1-nitrobenzene (1.89 g, 6.95 mmol) and 5% Pd/C (0.24 g) in EtOAc (20 mL) was stirred under an H₂ atm (balloon) overnight. The resulting mixture was filtered through a pad of Celite^{®} and concentrated under reduced pressure. The residue was purified by column chromatography (5% MeOH/95% CH₂Cl₂). The resulting oil solidified under vacuum overnight to give 4-(3-(*N-*methylcarbamoyl)phenoxy)aniline as a yellow solid (0.95 g, 56%).

### B. General Methods of Urea Formation

### B1. Reaction of a Heterocyclic Amine with an Aryl Isocyanate

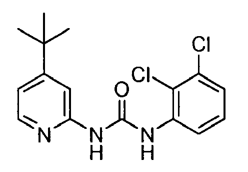

***N*-(4-*tert*-butylpyridyl)-*N*'-(2,3-dichlorophenyl) urea:** A solution of 2-amino-4-*tert-*butylpyridine (192 mg) and 2,3-dichlorophenyl isocyanate (240 mg) in anh. toluene (15 mL) was heated at 70 °C under argon for 24 h. The resulting mixture was diluted with EtOAc (200 mL) then washed with water (125 mL). The organic layer was dried (MgSO₄) and concentrated under reduced pressure to give a gum. Trituration of the gum with hexanes afforded *N*-(4-*tert*-butylpyridyl)-*N*'-(2,3-dichlorophenyl) urea as a white solid (394 mg, 91%): TLC (2:1 hexanes/ethyl acetate) R*_{ƒ}* 0.40; FAB-MS *m*/*z* 338 ((M+H)⁺).

### B2a. Reaction of a Heterocyclic Amine with N,N'-Carbonyldiimidazole Followed by Reaction with a Substituted Aniline

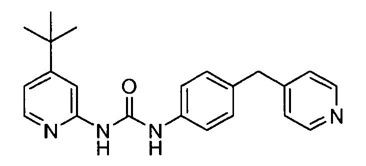

***N*-(4-*tert*-butylpyridyl)-*N*'-(4-(4-pyridinylmethyl)phenyl urea:** To a stirring solution of 4-*tert-*butyl-2-aminopyridine (192 mg) in anh. CH₂Cl₂ (15 mL) under argon at 0 °C was added CDI (207 mg). The resulting solution was allowed to warm to ambient temp over 2 h. To this mixture was added 4-(4-pyridylmethyl)aniline (prepared according to Method A1, 235 mg). The resulting solution was stirred at room temperature for 24 h, then was quenched with water (125 mL). The resulting mixture was extracted with EtOAc (200 mL). The organic layer was washed with water (100 mL), dried (MgSO₄) and concentrated under reduced pressure. The residue was purified by chromatography (SiO₂ EtOAc) to afford *N*-(4-*tert-*butylpyridyl)-*N*'-(4-(4-pyridinylmethyl)phenyl urea as a white solid (200 mg, 43%): TLC (EtOAc) R*_{ƒ}*0.47; FAB-MS *m*/*z* 361 ((M+H)⁺).

### B2b. Reaction of a Heterocyclic Amine with N,N'-Carbonyldiimidazole Followed by Reaction with a Substituted Aniline

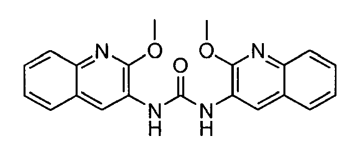

***N*,*N*'-(Bis(3-(2-methoxyquinolinyl)) urea):** To a stirring solution of 3-amino-2-methoxyquinoline (138 mg) in anh CH₂Cl₂ (15 mL) under argon at 0 °C was added CDI (128 mg). The resulting solution was warmed to ambient temp over 1 h. After 16 h 4-(2-*N-*Methylcarbamyl-4-pyridyloxy)aniline (175 mg) was added and the resulting yellow solution was stirred at room temperature under argon for 72 h. The solution was treated with water (125 mL) and the resulting mixture was extracted with EtOAc (2 x 150 mL). The combined organics were washed with a saturated NaCl solution (100 mL), dried (MgSO₄ and concentrated under reduced pressure. The residue was triturated with a 10% hexane/90% EtOAc solution. The resulting white crystals were washed with EtOAc. The resulting filtrate was purified by chromatography (SiO₂ 50% EtOAc/50% hexane) to give *N*,*N*'-(bis(3-(2-methoxyquinolinyl)) urea) (30 mg, 20% yield): TLC (50% EtOAc/50% hexane) R*_{ƒ}*0.45; HPLC ES-MS *m*/*z* 375 ((M+H)⁺).

### B2c. Reaction of a Heterocyclic Amine with N,N'-Carbonyldiimidazole Followed by Reaction with a Substituted Aniline

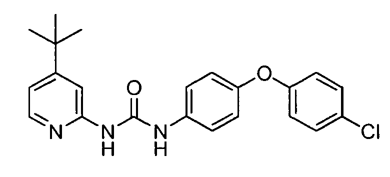

***N*-(4-*tert*-Butylpyridyl)-*N*'-(4-(4-chlorophenoxy)phenyl) urea:** A solution of 4-*tert-*butyl-2-aminopyridine (0.177 g, 1.18 mmol, 1 equiv.) in 1.2 mL ofanh. CH₂Cl₂ (1.2 mL) was added to CDI (0.200 g, 1.24 mmol, 1.05 equiv) and the mixture was allowed to stir under argon at room temperature 1 d. To the resulting solution was added 4-(4-chlorophenoxy)aniline (0.259 g, 1.18 mmol, 1 equiv.) in one portion. The resulting mixture was stirred at room temperature for 1 d, then was treated with a 10% citric acid solution (2 mL) and allowed to stir for 1 h. The resulting organic layer was extracted with EtOAc (3 x 5 mL). The combined organic layers were dried (MgSO₄) and concentrated *in vacuo.* The resultant residue was treated with CH₂Cl₂ (10 mL) and a 1 N aqueous NaOH solution. This mixture was allowed to stir overnight. The resulting organic layer was extracted with CH₂Cl₂ (3 x 5 mL). The combined organic layers were (MgSO₄) and concentrated *in vacuo.* The resultant solids were suspended in diethyl ether (10 mL) and sonicated for 15 minutes. The resulting white solids were dried to give *N*-(4-*tert-*butylpyridyl)-*N*'-(4-(4-chlorophenoxy)phenyl) urea (42 mg, 9%): mp 198-199 °C.

### B3. Reaction of Substituted Aniline with N,N'-Carbonyldiimidazole Followed by Reaction with a Heterocyclic Amine

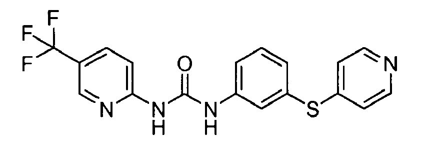

***N*-(2-(5-trifluoromethyl)pyridyloxy)-*N*'-(3-(4-pyridylthio)phenyl) urea:** A solution of 3-(4-pyridylthio)aniline (300 mg, 1.48 mmoles) in CH₂Cl₂ (12 mL) was treated with CDI (253 mg, 1.56 mmoles). The solution was stirred at room temperature and under argon for 2 h. The resulting mixture was treated with 2-amino-5-(trifluoromethyl)pyridine (238 mg, 1.47 mmoles) and heated at 40°C overnight. The reaction mixture was then diluted with EtOAc (25 mL), washed with water (10 mL) and a saturated NaCl solution m(25 mL), dried (MgSO₄) and concentrated under reduced pressure. The residue was purified by column chromatography (SiO₂; gradient from 70% EtOAc/30% CH₂Cl₂ to 100% EtOAc to give *N-*(2-(5-trifluoromethyl)pyridyloxy)-*N*'-(3-(4-pyridylthio)phenyl) urea afforded (103 mg): TLC (50% EtOAc/50% CH₂Cl₂) R*_{ƒ}*0.33; ¹H-NMR (DMSO-d₆) 6.06 (d, *J*=6Hz, 2H), 7.25 (dt, *J*=1.2,7.8 Hz, 1H), 7.48 (t, *J*=8.1 Hz, 1H), 7.59-7.63 (m, 1H), 7.77 (d, *J*=8.7 Hz, 1H), 7.86 (t, *J*=1.8 Hz, 1H), 8.12 (dd, *J*=2.7,9.3 Hz, 1H), 8.37 (d, *J*=6.3 Hz, 2H), 8.67 (bs, 1H), 9.88 (s, 1H), 10.26 (s, 1 H); FAB-MS *m*/*z* 391 ((M+H)⁺).

### B4. Reaction of a Heterocyclic Amine with Phosgene, Followed by Reaction with a Substituted Aniline

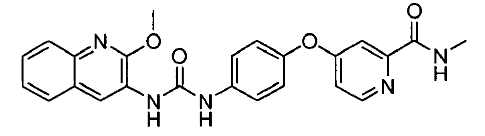

***N*-(3-(2-methoxyquinolinyl)-*N*'-(4-(4-(2-*N*-Methylcarbamyl-4-pyridyloxy)phenyl) urea:** To a stirring solution of phosgene (20% in toluene, 1.38 mL) in anh. CH₂Cl₂ (20 ml) at 0 °C under argon was added anh. pyridine (207 mg) followed by 3-amino-2-methoxyquinoline (456 mg). The resulting solution was warmed to ambient temperature over 1 h, then concentrated in vacuo at ambient temperature to give a white solid. The solid was dried under vacuum for 15 min then suspended in anh toluene (20 mL). To the resulting slurry was added 4-(4-(2-(methylcarbamoyl)pyridyloxy)aniline (prepared according to Method A2, 300 mg) and the reaction heated under argon at 80 °C for 20 h. The resulting mixture was diluted with water (200 mL), then treated with a saturated NaHCO₃ solution (10 mL) and extracted with EtOAc (2 x 300 mL). The combined organic layers were washed with a saturated NaCl solution (100 mL), dried (MgSO4) and concentrated under reduced pressure. The solid yellow residue was purified by chromatography (SiO₂, gradient from 50% EtOAc/50% hexane to 100% EtOAc), followed by recrystallization from diethyl ether and hexane to give *N*-(3-(2-methoxyquinolinyl)-*N*'-(4-(4-(2-*N*-Methylcarbamyl-4-pyridyloxy)phenyl) urea as a white solid (140 mg, 25%): TLC (EtOAc) R*_{ƒ}*0.52; FAB-MS *m*/*z* 430 ((M+H)⁺).

### B5a. Reaction of an Aniline with N,N'-Carbonyl Diimidazole Followed by Addition of a Second Aniline.

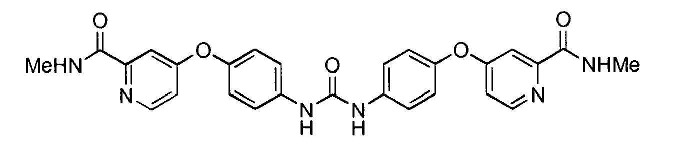

### Bis(4-(2-(N-methylcarbamoyl)-4-pyridyloxy)phenyl) Urea

To a stirring solution of 3-amino-2-methoxyquinoline (0.14 g) in anhydrous CH₂Cl₂ (15 mL) at 0 C was added CDI (0.13 g). The resulting solution was allowed to warm to room temp. over 1 h then was stirred at room temp. for 16 h. The resulting mixture was treated with 4-(2-(*N*-methylcarbamoyl)-4-pyridyloxy)aniline (0.18 g). The resulting yellow solution stirred at room temp. for 72 h, then was treated with water (125 mL). The resulting aqueous mixture was extracted with EtOAc (2 x 150 mL). The combined organic phases were washed with a saturated NaCl solution (100 ml), dried (MgSO₄) and concentrated under reduced pressure. The residue was triturated (90% EtOAc/10% hexane). The resulting white solids were collected by filtration and washed with EtOAc to give bis(4-(2-(*N-*methylcarbamoyl)-4-pyridyloxy)phenyl) urea (0.081 g, 44%): TLC (100% EtOAc) R*_{ƒ}* 0.50; ¹HNMR (DMSO-d₆) 2.76 (d, *J*=5.1 Hz, 6H), 7.1-7.6 (m, 12H), 8.48 (d, *J*=5.4 Hz, 1H), 8.75 (d, *J*=4.8 Hz, 2H), 8.86 (s, 2H); HPLC ES-MS *m*/*z* 513 ((M+H)⁺).

### B5b. Reaction of an Isocyanate with an Aniline.

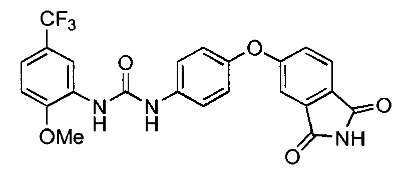

### N-(2-Methoxy-5-(trifluoromethyl)phenyl-N'-(4-(1,3-dioxoisoindolin-5-yloxy)phenyl) Urea

To a stirring solution of2-methoxy-5-(trifluoromethyl)phenyl isocyanate (0.10 g, 0.47 mmol) in CH₂Cl₂ (1.5 mL) was added 5-(4-aminophenoxy)isoindoline-1,3-dione (Method A3, Step 3; 0.12 g, 0.47 mmol) in one portion. The resulting mixture was stirred for 12 h, then was treated with CH₂Cl₂ (10 mL) and MeOH (5 mL). The resulting mixture was sequentially washed with a 1N HCl solution (15 mL) and a saturated NaCl solution (15 mL), dried (MgSO₄) and concentrated under reduced pressure to afford *N*-(2-methoxy-5-(trifluoromethyl)phenyl-*N*'-(4-(1,3-dioxoisoindolin-5-yloxy)phenyl) urea as a white solid (0.2 g, 96%): TLC (70% EtOAc/30% hexane) R*_{ƒ}*0.50; ¹H NMR (DMSO-d₆) 3.95 (s, 3H), 7.31-7.10 (m, 6H), 7.57 (d, J=9.3Hz, 2H), 7.80 (d, J=8.7 Hz, 1H), 8.53 (br s, 2H), 9.57 (s, 1H), 11.27 (br s, 1H); HPLC ES-MS 472.0 ((M+H)⁺, 100%).

### B6. Reaction of an Aniline with Phosgene Followed by Addition of a Second Aniline.

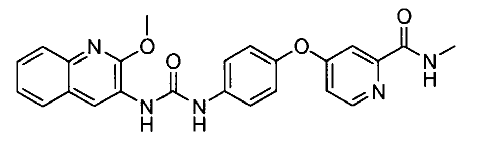

### N-(3-(2-methoxyquinolinyl)-N'-(4-(4-(2-N-Methylcarbamyl-4-pyridyloxy)phenyl) Urea

To a stirring solution of phosgene (20% in toluene, 1.38 mL) in anh. CH₂Cl₂ (20 ml) at 0 °C under argon was added anh. pyridine (207 mg) followed by 3-amino-2-methoxyquinoline (456 mg). The resulting solution was warmed to ambient temperature over 1 h, then concentrated in vacuo at ambient temperature to give a white solid. The solid was dried under vacuum for 15 min then suspended in anh toluene (20 mL). To the resulting slurry was added 4-(4-(2-(methylcarbamoyl)pyridyloxy)aniline (prepared according to Method A2, 300 mg) and the reaction heated under argon at 80 °C for 20 h. The resulting mixture was diluted with water (200 mL), then treated with a saturated NaHCO₃ solution (10 mL) and extracted with EtOAc (2 x 300 mL). The combined organic layers were washed with a saturated NaCl solution (100 mL), dried (MgSO4) and concentrated under reduced pressure. The solid yellow residue was purified by chromatography (SiO₂, gradient from 50% EtOAc/50% hexane to 100% EtOAc), followed by recrystallization from diethyl ether and hexane to give *N*-*(*3-(2-methoxyquinolinyl)-*N*'-(4-(4-(2-*N-*Methylcarbamyl-4-pyridyloxy)phenyl) urea as a white solid (140 mg, 25%): TLC (EtOAc) R*_{ƒ}*0.52; FAB-MS *m*/*z* 430 ((M+H)⁺).

### SPECIFIC COMPOUND PREPARATIONS

Descriptions of the detailed preparative steps used to prepare the specific compounds listed in Tables 1-8 are provided below. Many of the compounds listed in the Tables can be synthesized following a variety of methods. The specific examples below are therefore provided by way of illustration only and should not be construed to limit the scope of the invention in any way.

Entry 104: 4-(4-(2-(*N*-Methylcarbamoyl)pyridyloxy)aniline was prepared according to Method A10. 3-Amino-2-methoxyquinoline was reacted with 4-(4-(2-(*N-*methylcarbamoyl)pyridyloxy)aniline according to Method B4 to afford the urea.

Entry 105: 4-(3-*N-*Methylcarbamoylphenoxy)aniline was prepared according to Method A14. 3-Amino-2-methoxyquinoline was reacted with 4-(3-*N-*methylcarbamoylphenoxy)aniline according to Method B4 to afford the urea.

Entry 106: 4-Chloropyridine-2-carbonyl chloride was reacted with isopropylamine according to Method A10, Step 3b. The resulting 4-chloro-*N-*isopropyl-2-pyridinecarboxamide was reacted with 4-aminophenol according to Method A10, Step 4 to give 4-(2-(*N-*isopropylcarbamoyl)-4-pyridyloxy)aniline. 3-Amino-2-methoxyquinoline was reacted with 4-(2-(*N-*isopropylcarbamoyl)-4-pyridyloxy)aniline according to Method B5b to afford the urea.

Entry107: 4-Chloropyridine-2-carbonyl chloride HCl salt was reacted with ammonia according to Method A10, Step 3b to form 4-chloro-2-pyridinecarboxamide. 4-Chloro-2-pyridinecarboxamide was reacted with 4-aminophenol according to Method A10, Step 4 using DMAC in place of DMF to give 4-(2-carbamoyl-4-pyridyloxy)aniline. 4-(2-Carbamoyl-4-pyridyloxy)aniline was reacted with 4-(2-(*N-*isopropylcarbamoyl)-4-pyridyloxy)aniline according to Method B6 to afford the urea.

Entry108: 4-Chloro-*N-*methyl-2-pyridinecarboxamide was synthesized according to Method A10, Step 3b. 4-Chloro-*N*-methyl-2-pyridinecarboxamide was reacted with 4-aminophenol according to Method A10, Step 4 using DMAC in place of DMF to give 4-(2-(*N-*methylcarbamoyl)-4-pyridyloxy)aniline. 3-Amino-2-methoxyquinoline was reacted with 4-(2-(*N-*methylcarbamoyl)-4-pyridyloxy)aniline according to Method B6 to afford the urea.

Entry109: 4-Chloropyridine-2-carbonyl chloride HCl salt was reacted with ammonia according to Method A10, Step 3b to form 4-chloro-2-pyridinecarboxamide. 4-Chloro-2-pyridinecarboxamide was reacted with 3-aminophenol according to Method A10, Step 4 using DMAC in place of DMF to give 3-(2-carbamoyl-4-pyridyloxy)aniline. 3-Amino-2-methoxyquinoline was reacted with 3-(2-carbamoyl-4-pyridyloxy)aniline according to Method B6 to afford the urea.

Entry110: 4-Chloro-*N*-methyl-2-pyridinecarboxamide, which was synthesized according to Method A10, Step 3a, was reacted with 3-aminophenol according to Method A10, Step 4 using DMAC in place of DMF to give 3-(-2-(*N-*methylcarbamoyl)-4-pyridyloxy)aniline. 3-Amino-2-methoxyquinoline was reacted with 3-(-2-(*N-*methylcarbamoyl)-4-pyridyloxy)aniline according to Method B6 to afford the urea.

Entry 111: 4-(4-(3-(*N*-Methylcarbamoyl)-2-methoxyphenoxy)aniline was prepared according to Method A13. 3-Amino-2-methoxyquinoline was reacted with 4-(4-(3-(*N-*Methylcarbamoyl)-2-methoxyphenoxy)aniline was according to Method B6 to afford the urea.

Entry 112: 5-(4-Aminophenoxy)isoindoline-1 ,3-dione was prepared according to Method A11. 3-Amino-2-methoxyquinoline was reacted with 5-(4-Aminophenoxy)isoindoline-1,3-dione was according to Method B5b to afford the urea.

The following compounds have been synthesized according to the General Methods listed above:

**Table 7. Additional Isoquinolyl Ureas**

| | | | | | | | |
|---|---|---|---|---|---|---|---|
| 104 | 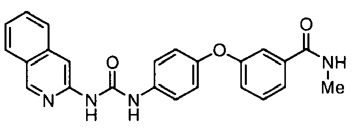 | | | 0.30 | 1% Et3N/ 99% EtOAc | 414 (M+H)+ (HPLC ES-MS) | A2 C5 |

**Table 8. 2-Methoxy-3-quinolyl Ureas with Omega Carbonyls**

| | | | | | | | |
|---|---|---|---|---|---|---|---|
| 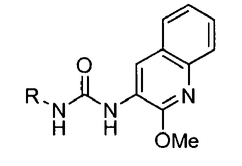 | | | | | | | |

| Entry | **R** | mp (°C) | HPLC (min.) | TLC *R_{f}* | TLC Solvent System | Mass Spec. [Source] | Synth. Method |
|---|---|---|---|---|---|---|---|
| 105 | 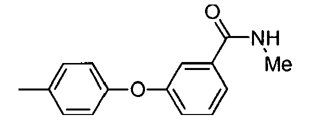 | 213-214 | | 0.20 | 5% MeOH/ 95% CHCl3 | 443 (M+H)+ (FAB) | A13 C2c |
| 106 | 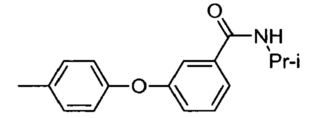 | 244-245 | | | | | A2 C2c |
| 107 | 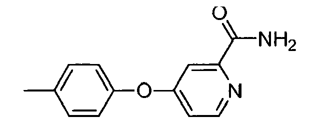 | | | 0.52 | 100% EtOAc | 430 (M+H)+ (FAB) | A2 C5 |
| 108 | 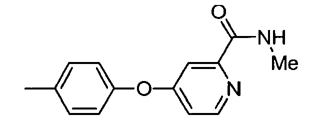 | | | 0.55 | 100% EtOAc | 444 (M+H)+ (FAB) | A2 C5 |
| 109 | 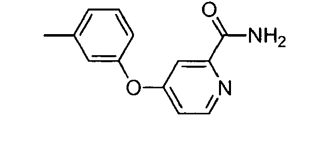 | | | 0.30 | 100% EtOAc | 430 (M+H)+ (FAB) | A2 C5 |
| 110 | 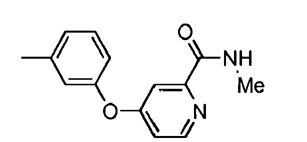 | | | 0.60 | 100% EtOAc | 444 (M+H)+ (FAB) | A2 C5 |
| 111 | 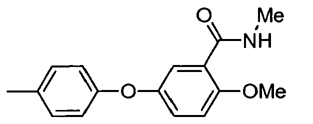 | 144-146 | | | | | A8 C5 |
| 112 | 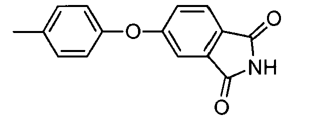 | | | | | | A3 C2c |

### BIOLOGICAL EXAMPLES

### P38 Kinase Assay:

The *in vitro* inhibitory properties of compounds were determined using a p38 kinase inhibition assay. P38 activity was detected using an *in vitro* kinase assay run in 96-well microtiter plates. Recombinant human p38 (0.5 µg/mL) was mixed with substrate (myelin basic protein, 5 µg/mL) in kinase buffer (25 mM Hepes, 20 mM MgCl₂ and 150 mM NaCl) and compound. One µCi/well of ³³P-labeled ATP (10 µM) was added to a final volume of 100 µL. The reaction was run at 32 °C for 30 min. and stopped with a 1M HCl solution. The amount of radioactivity incorporated into the substrate was determined by trapping the labeled substrate onto negatively charged glass fiber filter paper using a 1% phosphoric acid solution and read with a scintillation counter. Negative controls include substrate plus ATP alone.

All compounds exemplified displayed p38 IC₅₀s of between 1 nM and 10 µM.

### LPS Induced TNFα Production in Mice:

The *in vivo* inhibitory properties of selected compounds were determined using a murine LPS induced TNFα production *in vivo* model. BALB/c mice (Charles River Breeding Laboratories; Kingston, NY) in groups of ten were treated with either vehicle or compound by the route noted. After one hour, endotoxin (E. coli lipopolysaccharide (LPS) 100 µg) was administered intraperitoneally (i.p.). After 90 min, animals were euthanized by carbon dioxide asphyxiation and plasma was obtained from individual animals by cardiac puncture into heparinized tubes. The samples were clarified by centrifugation at 12,500 x g for 5 min at 4 °C. The supernatants were decanted to new tubes, which were stored as needed at -20 °C. TNFα levels in sera were measured using a commercial murine TNF ELISA kit (Genzyme).

The preceding examples can be repeated with similar success by substituting the generically or specifically described reactants and/or operating conditions of this invention for those used in the preceding examples.

From the foregoing discussion, one skilled in the art can easily ascertain the essential characteristics of this invention and make various changes and modifications of the invention to adapt it to various usages and conditions.

## Claims

1. A compound of Formula I:
A-D-B (I)
or a pharmaceutically acceptable salt thereof for use in a method of treating a disease mediated by p38 within a host, the disease mediated by p38 within a host being rheumatoid arthritis, osteoarthritis, septic arthritis, tumor metastasis, periodontal disease, corneal ulceration, proteinuria, coronary thrombosis from atherosclerotic plaque, aneurysmal aortic, birth control, dystrophobic epidermolysis bullosa, degenerative cartilage loss following traumatic joint injury, osteopenias mediated by MMP activity, tempero mandibular joint disease, demyelating disease of the nervous system, rheumatic fever, bone resorption, postmenopausal osteoperosis, sepsis, gram negative sepsis, septic shock, endotoxic shock, toxic shock syndrome, systemic inflammatory response syndrome, inflammatory bowel disease (Crohn's disease and ulcerative colitis), Jarisch-Herxheimer reaction, asthma, adult respiratory distress syndrome, acute pulmonary fibrotic disease, pulmonary sarcoidosis, allergic respiratory disease, silicosis, coal worker's pneumoconiosis, alveolar injury, hepatic failure, liver disease during acute inflammation, severe alcoholic hepatitis, malaria (Plasmodium falciparum malaria and cerebral malaria), non-insulin-dependent diabetes mellitus (NIDDM), congestive heart failure, damage following heart disease, atherosclerosis, Alzheimer's disease, acute encephalitis, brain injury, multiple sclerosis (demyelation and oligiodendrocyte loss in multiple sclerosis), advanced cancer, lymphoid malignancy, pancreatitis, impaired wound healing in infection, inflammation and cancer, myelodysplastic syndromes, systemic lupus erythematosus, biliary cirrhosis, bowel necrosis, psoriasis, radiation injury/ toxicity following administration of monoclonal antibodies, host-versus-graft reaction (ischemia reperfusion injury and allograft rejections of kidney, liver, heart, and skin), lung allograft rejection (obliterative bronchitis), complications due to total hip replacement, tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus or the human immunodeficiency virus (HIV); wherein
D is -NH-C(O)-NH-,
A is a substituted or unsubstituted quinolinyl or isoquinolinyl group,
B is a bridged cyclic structure of the formula -L-(ML¹)_{q}, where L is a 5 or 6 membered cyclic structure bound directly to D, L¹ comprises a substituted cyclic moiety having a least 5 members, M is a bridging group having at least one atom, q is an integer of from 1-3, and each cyclic structure of L and L¹ contains 0-4 members of the group consisting of nitrogen, oxygen and sulfur, wherein L¹ is substituted by at least one substituent selected from the group consisting of -SO₂R^{a}, -SO₂NR^{a}R^{b}, -C(O)R^{a}, -C(O)NR^{a}R^{b} and -C(NR^{a})R^{b}, wherein R^{a} and R^{b} are independently hydrogen or a carbon based moiety,
wherein the substituents for A are selected from the group consisting of halogen, up to per-halo, and Wn, where n is 0-3 and each W is independently selected from the group consisting of
C₁₋₁₀ alkyl, C₁₋₁₀ alkoxy, C₃₋₁₀ cycloalkyl having at least a five cyclic members and 0-3 heteroatoms selected from N, S and O; C₂₋₁₀ alkenyl, C₁₋₁₀ alkenoyl, C₆-C₁₄ aryl, C₇-C₂₄ alkaryl, C₇-C₂₄ aralkyl, C₃-C₁₂ heteroaryl having at least 5 cyclic members and 1-3 heteroatoms selected from O, N and S, C₄-C₂₄ alkheteroaryl having at least 5 cyclic members and 1-3 heteroatoms selected from O, N and S;
substituted C₁₋₁₀ alkyl, substituted C₁₋₁₀ alkoxy, substituted C₃₋₁₀ cycloalkyl having at least 5 cyclic members and 0-3 heteroatoms selected from N, S and O; substituted C₂₋₁₀ alkenyl, substituted C₁₋₁₀ alkenoyl, substituted C₆-C₁₄ aryl, substituted C₇-C₂₄ alkaryl, substituted C₇-C₂₄ aralkyl, substituted C₃-C₁₂ heteroaryl having at least 5 members and 1-3 heteratoms selected from O, N and S, substituted C₄-C₂₄ alkheteroaryl having at least 5 members and 1-3 heteroatoms selected from O, N and S,
-CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl, C₃-C₁₀ cycloalkyl having at least 5 cyclic members and 0-3 heteroatoms selected from O, S and N, C₆-C₁₄ aryl, C₃-C₁₀ hetaryl having at least 6 cyclic members and 0-3 heteroatoms selected from O, S and N, up to per halo substituted C₃-C₁₀ cycloalkyl having at least 5 cyclic members and 0-3 heteroatoms selected from O, S and N, up to per halo substituted C₆-C₁₄ aryl and up to per halo substituted C₃-C₁₀ hetaryl having at least 6 cyclic members and 0-3 heteroatoms selected from O, S and N,
where W is a substituted group, it is substituted by halogen, up to per halo, or by one or more substituents independently selected from the group consisting of -CN, -CO₂R⁷, -C(O)NR⁷R^{7'}, -C(O)-R⁷, -NO₂ -OR⁷, -SR⁷, -NR⁷R^{7'}, -NR⁷C(O)OR^{7'}, and -NR⁷C(O)R^{7'}, wherein R⁷ and R^{7'} are independently as defined above.

2. The compound of Formula I or a pharmaceutically acceptable salt thereof for use in a method of treating a disease according to claim 1 wherein M in the formula -L-(ML¹)_{q}, is selected from the group consisting of -O-, -S-, -N(R⁷)-, -(CH₂)ₘ-, -C(O)-, -CH(OH)-, -(CH₂)ₘO-, -(CH₂)ₘS-, -(CH₂)ₘ N(R⁷)-, -O(CH₂)ₘ-; -CHX^{a}-, -CX^{a}₂-, -S-(CH₂)ₘ-, -CR^{a}R^{b}-, and-N(R⁷)(CH₂)ₘ-, where m=1-3, X^{a} is halogen, q is 1, and R^{a} and R^{b} are as defined in claim 1, and R⁷ is selected from the group consisting of hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl.

3. The compound of Formula I or a pharmaceutically acceptable salt thereof for use in a method of treating a disease according to claim 2 wherein L in the formula -L-(ML¹)_{q} for B is a substituted 6 member cyclic aryl moiety, a substituted 5 or 6 member heterocyclic moiety, an unsubstituted 6 member cyclic aryl moiety, or an unsubstituted 5 or 6 member heterocyclic moiety, and L¹ in the formula -L-(ML¹)_{q} for B, is a substituted aryl moiety having at least 6 cyclic members, an unsubstituted aryl moiety having at least 6 cyclic members, a substituted hetaryl moiety having at least 6 cyclic members or an unsubstituted hetaryl moiety having at least 6 cyclic members, said heterocyclic and hetaryl moieties having 1 to 4 members selected from the group of hetero atoms consisting of nitrogen, oxygen and sulfur with the balance of the hetaryl and heterocyclic moiety being carbon.

4. The compound of Formula I or a pharmaceutically acceptable salt thereof for use in a method of treating a disease according to claim 1 wherein M in the formula -L-(ML¹) for B is -O-, -CH₂-, -S-, -NH-, -C(O)-, -O-CH₂-or-CH₂-O-.

5. The compound of Formula I or a pharmaceutically acceptable salt thereof for use in a method of treating a disease according to claim 1, wherein A has 1-3 substituents selected from the group consisting of C₁₋₁₀ alkyl, up to per halo substituted C₁₋₁₀ alkyl, - CN, -OH, halogen, C₁₋₁₀ alkoxy, up to per halo substituted C₁₋₁₀ alkoxy and C₃₋₁₀ heterocyclic moieties having at least 5 cyclic members and 1 to 2 heteroatoms selected from the group of consisting of nitrogen, oxygen and sulfur.

6. The compound of Formula I or a pharmaceutically acceptable salt thereof for use in a method of treating a disease according to claim 1 wherein L¹ is substituted 1 to 3 times by one or more substituents selected from the group consisting of -CN, halogen up to per halo, C₁₋₁₀ alkyl, C₁₋₁₀ alkoxy,-OH, up to per halo substituted C₁₋₁₀ alkyl, up to per halo substituted C₁₋₁₀ alkoxy, -OR⁷, -SR⁷, -NR⁷R^{7'}, -CO₂R⁷, -C(O)NR⁷R^{7'}, -C(O)R⁷ or -NO₂, wherein each R⁷ and R^{7'} is independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl, and up to per halosubstituted C₁₋₁₀ alkenoyl.

7. The compound of Formula I or a pharmaceutically acceptable salt thereof for use in a method of treating a disease according to claim 1 wherein a pharmaceutically acceptable salt of a compound of formula I is administered which is selected from the group consisting of
a) basic salts of organic acids and inorganic acids selected from the group consisting of hydrochloric acid, hydrobromic acid, sulfuric acid, phosphoric acid, methanesulfonic acid, trifluorosulfonic acid, benzenesulfonic acid, p-toluene sulfonic acid (tosylate salt), 1-napthalene sulfonic acid, 2-napthalene sulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid; and
b) acid salts of organic and inorganic bases containing cations selected from the group consisting of alkaline cations, alkaline earth cations, the ammonium cation, aliphatic substituted ammonium cations and aromatic substittued ammonium cations.

8. The compound of Formula **I** or a pharmaceutically acceptable salt thereof for use in a method of treating a disease according to claim 1 for the treatment of a disease other than cancer.

9. The compound of Formula **I** or a pharmaceutically acceptable salt thereof for use in a method of treating a disease according to claim 1 wherein the condition within a host treated by administering a compound of formula I is rheumatoid arthritis, osteoarthritis, septic arthritis, tumor metastasis, periodontal disease, corneal ulceration, proteinuria, coronary thrombosis from atherosclerotic plaque, aneurysmal aortic, birth control, dystrophobic epidermolysis bullosa, degenerative cartilage loss following traumatic joint injury, osteopenias mediated by MMP activity, tempero mandibular joint disease or demyelating disease of the nervous system.

10. The compound of Formula **I** or a pharmaceutically acceptable salt thereof for use in a method of treating a disease according to any one of claims 1 to 8 wherein the condition within a host treated by administering a compound of formula I is rheumatic fever, bone resorption, postmenopausal osteoperosis, sepsis, gram negative sepsis, septic shock, endotoxic shock, toxic shock syndrome, systemic inflammatory response syndrome, inflammatory bowel disease (Crohn's disease and ulcerative colitis), Jarisch-Herxheimer reaction, asthma, adult respiratory distress syndrome, acute pulmonary fibrotic disease, pulmonary sarcoidosis, allergic respiratory disease, silicosis, coal worker's pneumoconiosis, alveolar injury, hepatic failure, liver disease during acute inflammation, severe alcoholic hepatitis, malaria (Plasmodium falciparum malaria and cerebral malaria), non-insulin-dependent diabetes mellitus (NIDDM), congestive heart failure, damage following heart disease, atherosclerosis, Alzheimer's disease, acute encephalitis, brain injury, multiple sclerosis (demyelation and oligiodendrocyte loss in multiple sclerosis), advanced cancer, lymphoid malignancy, pancreatitis, impaired wound healing in infection, inflammation and cancer, myelodysplastic syndromes, systemic lupus erythematosus, biliary cirrhosis, bowel necrosis, psoriasis, radiation injury/ toxicity following administration of monoclonal antibodies, host-versus-graft reaction (ischemia reperfusion injury and allograft rejections of kidney, liver, heart, and skin), lung allograft rejection (obliterative bronchitis) or complications due to total hip replacement.

11. The compound of Formula **I** or a pharmaceutically acceptable salt thereof for use in a method of treating a disease according to any one of claims 1 to 8 wherein the condition within a host treated by administering a compound of formula I is an an infectious disease selected from the group consisting of tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus, and the human immunodeficiency virus (HIV).

12. The compound of Formula **I** or a pharmaceutically acceptable salt thereof for use in a method of treating a disease according to claim 1 wherein:
Rₐ and R_{b} are,
a) independently hydrogen,
a carbon based moiety selected from the group consisting of C₁-C₁₀ alkyl, C₁-C₁₀ alkoxy, C₃₋₁₀ cycloalkyl having 0-3 hetero atoms selected from N, S and O, C₂₋₁₀ alkenyl, C₁₋₁₀ alkenoyl, C₆₋₁₄ aryl, C₃₋₁₂ hetaryl having 1-3 heteroatoms selected from O, N and S, C₇₋₂₄ aralkyl, C₇-C₂₄ alkaryl, substituted C₁₋₁₀ alkyl, substituted C₁₋₁₀ alkoxy, substituted C₃₋₁₀ cycloalkyl having 0-3 heteroatoms selected from N, S and O, substituted C₆₋₁₄ aryl, substituted C₃₋₁₂ hetaryl having 1-3 heteroatoms selected from N, S and O, substituted C₇₋₂₄ aralkyl, substituted C₇₋₂₄ alkaryl, where Rₐ and R_{b} are a substituted group, they are substituted by halogen up to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g}; or
-OSi(R_{f})₃ where R_{f} is hydrogen, C₁₋₁₀ alkyl, C₁₋₁₀ alkyl, C₁₋₁₀ alkoxy, C₃-C₁₀ cycloalkyl having 0-3 heteroatoms selected from O, S and N, C₆-₁₂ aryl, C₃-C₁₂ hetaryl having 1-3 heteroatoms selected from O, S and N, C₇₋₂₄ aralkyl, substituted C₁₋₁₀ alkyl, substituted C₁-C₁₀ alkoxy, substituted C₃-C₁₂ cycloalkyl having 0-3 heteroatoms selected from O, S and N, substituted C₃-C₁₂ heteraryl having 1-3 heteroatoms selected from O, S, and N, substituted C₆₋₁₂ aryl, and substituted C₇₋₂₄ alkaryl, where R_{f} is a substituted group it is substituted halogen up to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C_{y-}-C₂₄ alkaryl, C₇-C₂₄ aralkyl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g}, or
b) Rₐ and R_{b} together form a 5-7 member heterocyclic structure of 1-3 heteroatoms selected from N, S and O, or a substituted 5-7 member heterocyclic structure of 1-3 heteroatoms selected from N, S and O with substituents selected from the group consisting of halogen up to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C₇-C₂₄ alkaryl, C₇-C₂₄ aralkyl, halo substituted C₁₋₆ alkyl up to per halo alkyl, halo substituted C₆-C₁₂ aryl up to per halo aryl, halo substituted C₃-C₁₂ cycloalkyl having 0-3 heteroatoms selected from N, S and O, up to per halo cycloalkyl, halo substituted C₃-C₁₂ hetaryl up to per halo heteraryl, halo substituted C₇-C₁₂ aralkyl up to per halo aralkyl, halo substituted C₇-C₂₄ alkaryl up to per halo alkaryl, and -C(O)R_{g},
or
c) one of Rₐ or R_{b} is -C(O)-, a C₁-C₅ divalent alkylene group or a substituted C₁-C₅ divalent alkylene group bound to the moiety L to form a cyclic structure with at least 5 members,
wherein the substituents of the substituted C₁-C₅ divalent alkylene group are selected from the group consisting of halogen, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and 0, C₁₋₁₀ alkoxy, C₆₋₁₂ aryl, C₇-C₂₄ alkaryl, C₇-C₂₄ aralkyl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)R_{g},
where R_{g} is C₁₋₁₀ alkyl; -CN, -CO₂R_{d}, -OR_{d}, -SR_{d}, -NO₂, -C(O) Rₑ, -NR_{d}Rₑ, -NR_{d} C(O)ORₑ and -NR_{d} C(O)Rₑ, and R_{d} and Rₑ are independently selected from the group consisting of hydrogen, C₁₋₁₀, alkyl, C₁₋₁₀ alkoxy, C₃₋₁₀ cycloalkyl having 0-3 heteroatoms selected from O, N and S, C₆₋₁₂ aryl, C₃-C₁₂ hetaryl with 1-3 heteroatoms selected from O, N and S and C₇-C₂₄ aralkyl, C₇-C₂₄ alkaryl, up to per halo substituted C₁-C₁₀ alkyl, up to per halo substituted C₃-C₁₀ cycloalkyl having 0-3 heteroatoms selected from O, N and S, up to per halo substituted C₆-C₁₄ aryl, up to per halo substituted C₃-C₁₂ hetaryl having 1-3 heteroatoms selected from O, N, and S, halo substituted C₇-C ₂₄ alkaryl up to per halo alkaryl, and up to per halo substituted C₇-C₂₄ aralkyl.

13. The compound of Formula I or a pharmaceutically acceptable salt thereof for use in a method of treating a disease according to claim 2, wherein said substituted cyclic moiety L¹ is phenyl, pyridyl or pyrimidinyl.

14. The compound of Formula I or a pharmaceutically acceptable salt thereof for use in a method of treating a disease according to claim 1 wherein L¹ is substituted by - C(O)NR^{a}R^{b} or -SO₂NR^{a}R^{b}.

15. Use of a compound of Formula I:
A-D-B (I)
or a pharmaceutically acceptable salt thereof for the preparation of a medicament for treating a disease mediated by p38 within a host, the disease mediated by p38 within a host being rheumatoid arthritis, osteoarthritis, septic arthritis, tumor metastasis, periodontal disease, corneal ulceration, proteinuria, coronary thrombosis from atherosclerotic plaque, aneurysmal aortic, birth control, dystrophobic epidermolysis bullosa, degenerative cartilage loss following traumatic joint injury, osteopenias mediated by MMP activity, tempero mandibular joint disease, demyelating disease of the nervous system, rheumatic fever, bone resorption, postmenopausal osteoperosis, sepsis, gram negative sepsis, septic shock, endotoxic shock, toxic shock syndrome, systemic inflammatory response syndrome, inflammatory bowel disease (Crohn's disease and ulcerative colitis), Jarisch-Herxheimer reaction, asthma, adult respiratory distress syndrome, acute pulmonary fibrotic disease, pulmonary sarcoidosis, allergic respiratory disease, silicosis, coal worker's pneumoconiosis, alveolar injury, hepatic failure, liver disease during acute inflammation, severe alcoholic hepatitis, malaria (Plasmodium falciparum malaria and cerebral malaria), non-insulin-dependent diabetes mellitus (NIDDM), congestive heart failure, damage following heart disease, atherosclerosis, Alzheimer's disease, acute encephalitis, brain injury, multiple sclerosis (demyelation and oligiodendrocyte loss in multiple sclerosis), advanced cancer, lymphoid malignancy, pancreatitis, impaired wound healing in infection, inflammation and cancer, myelodysplastic syndromes, systemic lupus erythematosus, biliary cirrhosis, bowel necrosis, psoriasis, radiation injury/ toxicity following administration of monoclonal antibodies, host-versus-graft reaction (ischemia reperfusion injury and allograft rejections of kidney, liver, heart, and skin), lung allograft rejection (obliterative bronchitis), complications due to total hip replacement, tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus or the human immunodeficiency virus (HIV); wherein
D is -NH-C(O)-NH-,
A is a substituted or unsubstituted quinolinyl or isoquinolinyl group,
B is a bridged cyclic structure of the formula -L-(ML¹)_{q}, where L is a 5 or 6 membered cyclic structure bound directly to D, L¹ comprises a substituted cyclic moiety having a least 5 members, M is a bridging group having at least one atom, q is an integer of from 1-3, and each cyclic structure of L and L¹ contains 0-4 members of the group consisting of nitrogen, oxygen and sulfur, wherein L¹ is substituted by at least one substituent selected from the group consisting of -SO₂R^{a}, -SO₂NR^{a}R^{b}, -C(O)R^{a}, -C(O)NR^{a}R^{b} and -C(NR^{a})R^{b} , wherein R^{a} and R^{b} are independently hydrogen or a carbon based moiety,
wherein the substituents for A are selected from the group consisting of halogen, up to per-halo, and Wn, where n is 0-3 and each W is independently selected from the group consisting of
C₁₋₁₀ alkyl, C₁₋₁₀ alkoxy, C₃₋₁₀ cycloalkyl having at least a five cyclic members and 0-3 heteroatoms selected from N, S and O; C₂₋₁₀ alkenyl, C₁₋₁₀ alkenoyl, C₆-C₁₄ aryl, C₇-C₂₄ alkaryl, C₇-C₂₄ aralkyl, C₃-C₁₂ heteroaryl having at least 5 cyclic members and 1-3 heteroatoms selected from O, N and S, C₄-C₂₄ alkheteroaryl having at least 5 cyclic members and 1-3 heteroatoms selected from O, N and S;
substituted C₁₋₁₀ alkyl, substituted C₁₋₁₀ alkoxy, substituted C₃₋₁₀ cycloalkyl having at least 5 cyclic members and 0-3 heteroatoms selected from N, S and O; substituted C₂₋₁₀ alkenyl, substituted C₁₋₁₀ alkenoyl, substituted C₆-C₁₄ aryl, substituted C₇-C₂₄ alkaryl, substituted C₇-C₂₄ aralkyle, substituted C₃-C₁₂ heteroaryl having at least 5 members and 1-3 heteratoms selected from O, N and S, substituted C₄-C₂₄ alkheteroaryl having at least 5 members and 1-3 heteroatoms selected from O, N and S,
-CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl, C₃-C₁₀ cycloalkyl having at least 5 cyclic members and 0-3 heteroatoms selected from O, S and N, C₆-C₁₄ aryl, C₃-C₁₀ hetaryl having at least 6 cyclic members and 0-3 heteroatoms selected from O, S and N, up to per halo substituted C₃-C₁₀ cycloalkyl having at least 5 cyclic members and 0-3 heteroatoms selected from O, S and N, up to per halo substituted C₆-C₁₄ aryl and up to per halo substituted C₃-C₁₀ hetaryl having at least 6 cyclic members and 0-3 heteroatoms selected from O, S and N,
where W is a substituted group, it is substituted by halogen, up to per halo, or by one or more substituents independently selected from the group consisting of -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, and -NR⁷C(O)R^{7'}, wherein R⁷ and R^{7'} are independently as defined above.

16. A compound selected from the group consisting of
*N*-(2-Methoxy-3-quinolyl)-*N*'-(4-[3-(*N*-methylcarbamoyl)phenoxy]phenyl)urea
*N*-(2-Methoxy-3-quinolyl)-*N*'-(4-[2-(*N*-methylcarbamoyl)-4-pyridyloxy]phenyl)urea
*N*-(2-Methoxy-3-quinolyl)-*N*'-(4-(2-carbamoyl-4-pyridyloxy)phenyl)urea
*N*-(2-Methoxy-3-quinolyl)-*N*'-(3-[2-(*N*-methylcarbamoyl)-4-pyridyloxy]phenyl)urea
*N*-(2-Methoxy-3-quinolyl)-*N*'-(3-(2-carbamoyl)-4-pyridyloxy)phenyl)urea
*N*-(2-Methoxy-3-quinolyl)-*N*'-(4-[3-(N-isopropylcarbamoyl)phenoxy]phenyl)urea
*N*-(2-Methoxy-3-quinolyl)-*N*'-(4-[4-methoxy-3-(*N-*methylcarbamoyl)phenoxy]phenyl)urea
*N*-(3-Isoquinolyl)-*N*'-(4-[2-(*N*-methylcarbamoyl)-4-pyridyloxy]phenyl)urea and pharmaceutically acceptable salts thereof;
for use in a method of treating a disease mediated by p38 kinase as defined in claim 1, other than cancer.

17. Use of a compound selected from the group consisting of
*N*-(2-Methoxy-3-quinolyl)-*N*'-(4-[3-(*N*-methylcarbamoyl)phenoxy]phenyl)urea
*N*-(2 -Methoxy-3-quinolyl)-*N*'-(4-[2-(*N*-methylcarbamoyl)-4-pyridyloxy]phenyl)urea
*N*-(2-Methoxy-3-quinolyl)-*N*'-(4-(2-carbamoyl-4-pyridyloxy)phenyl)urea
*N*-(2-Methoxy-3-quinolyl)-*N*'-(3-[2-(*N*-methylcarbamoyl)-4-pyridyloxy]phenyl)urea
*N*-(2-Methoxy-3-quinolyl)-*N*'-(3-(2-carbamoyl)-4-pyridyloxy)phenyl)urea
*N*-(2-Methoxy-3-quinolyl)-*N*'-(4-[3-(*N*-isopropylcarbamoyl)phenoxy]phenyl)urea
*N*-(2-Methoxy-3-quinolyl)-*N*'-(4-[4-methoxy-3-(*N-*methylcarbamoyl)phenoxy]phenyl)urea
N-(3-Isoquinolyl)-*N*'-(4-[2-(*N*-methylcarbamoyl)-4-pyridyloxy]phenyl)urea and pharmaceutically acceptable salts thereof;
for the preparation of a medicament for the treatment of a disease mediated by p38 kinase as defined in claim 1, other than cancer.

18. A pharmaceutical composition for the treatment of a disease within a host mediated by p38 comprising an amount of a compound of Formula I effective to inhibit p38 mediated events,
A-D-B (I)
or a pharmaceutically acceptable salt thereof, in an amount effective to treat a disease mediated by p38 and a physiologically acceptable carrier;
wherein
D is -NH-C(O)-NH-,
A is as defined in claim 1
B is a bridged cyclic structure of the formula -L-(ML¹)_{q}, where L is a 5 or 6 membered cyclic structure bound directrly to D, L¹ comprises a substituted cyclic moiety having a least 5 members, M is a bridging group having at least one atom, q is an integer of from 1-3, and each cyclic structure of L and L¹ contains 0-4 members of the group consisting of nitrogen, oxygen and sulfur, wherein L¹ is substituted by at least one substituent selected from the group consisting of -SO₂Rₓ, -C(O)Rₓ, and -C(NR_{y})R_{z} wherein Ry is hydrogen or a carbon based moiety of up to 24 carbon atoms optionally containing heteroatoms selected from N, S and O and optionally halosubstituted, up to per halo,
R_{z} is hydrogen or a carbon based moiety of up to 30 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen;
Rₓ is R_{z} or NRₐR_{b} where Rₐ and R_{b} are
a) independently hydrogen,
a carbon based moiety of up to 30 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen, or -OSi(R_{f})₃ where R_{f} is hydrogen or a carbon based moiety of up to 24 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen; or
b) Rₐ and R_{b} together form a 5-7 member heterocyclic structure of 1-3 heteroatoms selected from N, S and O, or a substituted 5-7 member heterocyclic structure of 1-3 heteroatoms selected from N, S and O substituted by halogen, hydroxy or carbon based substituents of up to 24 carbon atoms, which optionally contain heteroatoms selected from N, S and O and are optionally substituted by halogen; or
c) one of Rₐ or R_{b} is -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, and carbon based substituents of up to 24 carbon atoms, which optionally contain heteroatoms selected from N, S and O and are optionally substituted by halogen.

19. A pharmaceutical composition as in claim 18 wherein the cyclic structures of B and L bound directly to D are not substituted in the ortho position by -OH or a moiety having an ionizable hydrogen and a pKa of 10 or less.

20. A pharmaceutical composition as in claim 18 wherein B of Formula I is a substituted or unsubstituted six member aryl moiety or at least a five member heterocylic moiety, said heterocyclic moiety having 1 to 4 members selected from the group of hetaryl atoms consisting of nitrogen, oxygen and sulphur with the balance of the heterocylic moiety being carbon.

21. A pharmaceutical composition as in claim 18, wherein L, the 5 or 6 member cyclic structure bound directly to D, is a substituted or unsubstituted 6 member heteroaryl moiety, wherein said heteroaryl moiety has 1 to 4 members selected from the group of heteroatoms consisting of nitrogen, oxygen and sulphur with the balance of said hetaryl moiety being carbon, wherein the one or more substituents are selected from the group consisting of halogen and Wn, wherein W and n are as defined in claim 18.

22. A pharmaceutical composition as in claim 18 wherein L¹ is substituted by -C(O)Rₓ.

23. A pharmaceutical composition as in claim 18 wherein L¹ is substituted by -C(O)Rₓ or -SO₂Rₓ, wherein Rₓ is NRₐR_{b}.

24. A pharmaceutical composition for the treatment of a disease within a host mediated by p38 comprising a compound selected from the group consisting of
*N*-(2-Methoxy-3-quinolyl)-*N*'-(4-[3-(*N*-methylcarbamoyl)phenoxy]phenyl)urea
*N*-(2-Methoxy-3-quinolyl)-*N*'-(4-[2-(*N*-methylcarbamoyl)-4-pyridyloxy]phenyl)urea
*N*-(2-Methoxy-3-quinolyl)-*N*'-(4-(2-carbamoyl-4-pyridyloxy)phenyl)urea
*N*-(2-Methoxy-3-quinolyl)-*N*'-(3-[2-(*N*-methylcarbamoyl)-4-pyridyloxy]phenyl)urea
*N*-(2-Methoxy-3-quinolyl)-*N*'-(3-(2-carbamoyl)-4-pyridyloxy)phenyl)urea
*N*-(2-Methoxy-3-quinolyl)-*N*'-(4-[3-(*N*-isopropylcarbamoyl)phenoxy]phenyl)urea
*N*-(2-Methoxy-3-quinolyl)-*N*'-(4-[4-methoxy-3-(*N-*methylcarbamoyl)phenoxy] phenyl)urea
*N*-(3-Isoquinolyl)-*N*¹-(4-[2-(*N*-methylcarbamoyl)-4-pyridyloxy]phenyl)urea and pharmaceutically acceptable salts thereof.

25. Use of a composition of any one of claims 18 to 24 for the preparation of a medicament for treating a disease mediated by p38 within a host, wherein the disease mediated by p38 is as defined in claim 1.

## Patentansprüche

1. Verbindung der Formel I:
A-D-B (1)
oder pharmazeutisch verträgliches Salz davon zur Verwendung in einem Verfahren zur Behandlung einer Krankheit, die durch p38 in einem Wirt vermittelt wird, wobei die durch p38 in einem Wirt vermittele Krankheit rheumatoide Arthritis, Osteoarthritis, septische Arthritis, Tumormetastase, Periodontalkrankheit, Cornealgeschwür, Albuminurie, Koronarthrombose von arteriosklerotischen Plaques, Aortenaneurysma, Geburtskontrolle, dystrophe Epidermolysis bullosa, degenerativer Knorpelschwund in Folge traumatischer Gelenksverletzung, durch MMP-Aktivität vermittelte Osteopenie, temporomandibulare Gelenkskrankheit, demyelierende Krankheit des Nervensystems, rheumatisches Fieber, Knochenresorption, postmenopausale Osteoporose, Sepsis, gram-negative Sepsis, septischer Schock, endotoxischer Schock, toxisches Schocksyndrom, systemisches Entzündungsantwortssyndrom, entzündliche Darmkrankheit (Crohn-Krankheit und Kolitisgeschwür), Jarisch-Herxheimer-Reaktion, Asthma, adultes Atemnotsyndrom, akute Lungenfibrosekrankheit, pulmonale Sarkoidose, allergische Atemwegskrankheit, Silikosis, Kohlenarbeiter-Pneumokoniose, alveolare Verletzung, hepatitisches Versagen, Leberkrankheit bei akuter Entzündung, schwere alkoholische Hepatitis, Malaria (Plasmodium falciparum Malaria und Zerebralmalaria), nicht insulinabhängiger Diabetes mellitus (NIDDM), kongestives Herzversagen, Schädigung in Folge von Herzkrankheit, Arteriosklerose, Alzheimer, akute Enzephalitis, Gehirnverletzung, Multiple Sklerose (Demylierung und oligodendrocytischer Verlust bei Multipler Sklerose), fortgeschrittener Krebs, lymphoide Malignität, Pankreatitis, beeinträchtigte Wundheilung bei Infektion, Entzündung und Krebs, myelodysplastische Syndrome, systemischer Lupus erythematosus, biliäre Zirrhose, Darmnekrose, Psoriasis, Strahlenverletzung/Toxizität in Folge von Verabreichung von monoklonalen Antikörpern, Wirt-gegen-Transplantat-Reaktion (Ischämie-Reperfusionsverletzung und allogene Abstoßungen von Niere, Leber, Herz und Haut), allogene Lungenabstoßung (Bronchitis obliterans), Komplikationen aufgrund vollständigen Hüftaustauschs, Tuberkulose, Helicobacter-pylori-Infektion bei Magengeschwürkrankheit, Chaga-Krankheit, welche aus Trypanosoma-cruzi-Infektion resultiert, Wirkungen von Shiga-artigem Toxin, die aus E.coli-Infektion resultieren, Wirkungen von Enterotoxin A, die aus Staphylococcus-Infektion resultieren, meningococcale Infektion und Infektionen aus Borrelia burgdorferi, Treponema pallidum, Zytomegalovirus, Grippevirus, Theiler-Enzephalomyelitis-Virus oder der menschliche Immunschwächevirus (HIV) ist; wobei
D -NH-C(O)-NH- ist,
A eine gegebenenfalls substituierte Quinolinyl- oder Isoquinolinyl-Gruppe ist,
B eine verbrückte zyklische Struktur der Formel -L-(ML¹)q ist, wobei L eine 5- oder 6-gliedrige zyklische Struktur ist, die direkt an D gebunden ist, L¹ eine substituierte zyklische Einheit mit wenigstens 5 Gliedern umfaßt, M eine Verbrückungsgruppe mit wenigstens einem Atom ist, q eine ganze Zahl von 1 - 3 ist und jede zyklische Struktur von L und L¹ 0 - 4 Glieder der Gruppe enthält, die aus Stickstoff, Sauerstoff und Schwefel besteht, wobei L¹ substituiert ist durch wenigstens einen Substituenten, der aus der Gruppe gewählt ist, die aus -SO₂R^{a}, -SO₂NR^{a}R^{b}, -C(O)R^{a}, -C(O)N^{a}R^{b} und -C(NR^{a})R^{b} besteht, wobei R^{a} und R^{b} unabhängig Wasserstoff oder eine kohlenstoffbasierte Einheit sind,
wobei die Substituenten für A aus der Gruppe gewählt sind, die aus Halogen bis Perhalogen und Wn besteht, wobei n 0 - 3 ist und jedes W unabhängig aus der Gruppe gewählt ist, die besteht aus
C₁₋₁₀Alkyl, C₁₋₁₀Alkoxy, C₃₋₁₀Zykloalkyl mit wenigstens 5 zyklischen Gliedern und 0 - 3 Heteroatomen, die unter N, S und O gewählt sind, C₂₋₁₀Alkenyl, C₁₋₁₀Alkenoyl, C₆-C₁₄Aryl, C₇-C₂₄Alkaryl, C₇-C₂₄Aralkyl, C₃-C₁₂Heteroaryl mit wenigstens 5 zyklischen Gliedern und 1 - 3 Heteroatomen, die unter O, N und S gewählt sind, C₄-C₂₄Alkheteroaryl mit wenigstens 5 zyklischen Gliedern und 1 - 3 Heteroatomen, die unter O, N und S gewählt sind,
substituiertes C₁₋₁₀Alkyl, substituiertes C₁₋₁₀Alkoxy, substituiertes C₃₋₁₀Zykloalkyl mit wenigstens 5 zyklischen Gliedern und 0 - 3 Heteroatomen, die unter N, S und O gewählt sind, substituiertes C₂₋₁₀Alkenyl, substituiertes C₁₋₁₀Alkenoyl, substituiertes C₆-C₁₄Aryl, substituiertes C₇-C₂₄ Alkylaryl, substituiertes C₇-C₂₄Aralkyl, substituiertes C₃-C₁₂Heteroaryl mit wenigstens 5 Gliedern und 1 - 3 Heteroatomen, die unter O, N und S gewählt sind, substituiertes C₄-C₂₄ Alkheteroaryl mit wenigstens 5 Gliedern und 1 - 3 Heteroatomen, die unter O, N und S gewählt sind,
-CN, -CO₂R⁷, -C(O)NR⁷R^{7'}, -C(O)-R⁷, -NO₂ -OR⁷, -SR⁷, -NR⁷R^{7'}, - NR⁷C(O)OR^{7'}, -NR⁷C(O)R^{7'}, mit jedem R⁷ and R^{7'} unabhängig gewählt aus Wasserstoff C₁₋₁₀Alkyl, C₁₋₁₀Alkoxy, C₂₋₁₀Alkenyl, C₁₋₁₀Alkenoyl und bis zu perhalogensubstituiertem C₁₋₁₀Alkyl, bis zu perhalogensubstituiertem C₁₋₁₀Alkoxy, bis zu perhalogensubstituiertem C₂₋₁₀Alkenyl und bis zu perhalogensubstituiertem C₁₋₁₀Alkenoyl, C₃-C₁₀Zykloalkyl mit wenigstens 5 zyklischen Gliedern und 0 - 3 Heteroatomen, die unter O, S und N gewählt sind, C₆-C₁₄Aryl, C₃-C₁₀Hetaryl mit wenigstens 6 zyklischen Gliedern und 0 - 3 Heteroatomen, die unter O, S und N gewählt sind, bis zu perhalogensubstituiertem C₃-C₁₀Zykloalkyl mit wenigstens 5 zyklischen Gliedern und 0 - 3 Heteroatomen, die unter O, S und N gewählt sind, bis zu perhalogensubstituiertem C₆-C₁₄Aryl und bis zu perhalogensubstituiertem C₃-C₁₀Hetaryl mit wenigstens 6 zyklischen Gliedern und 0 - 3 Heteroatomen, die unter O, S und N gewählt sind,
wo W eine substituierte Gruppe ist, ist sie durch Halogen bis zu Perhalogen oder durch einen oder mehrere Substituenten substituiert, die unabhängig gewählt sind aus der Gruppe, die aus -CN, -CO₂R⁷, -C(O)NR⁷R^{7'}, -C(O)-R⁷, -NO₂, -OR⁷, - SR⁷, -NR⁷R^{7'}, -NR⁷C(O)OR⁷¹ und -NR⁷C(O)R^{7'} besteht, wobei R⁷ und R^{7'} unabhängig wie oben definiert sind.

2. Verbindung der Formel I oder pharmazeutisch verträgliches Salz davon zur Verwendung in einem Verfahren zur Behandlung einer Krankheit nach Anspruch 1, wobei M in der Formel-L-(ML¹)q, gewählt ist aus der Gruppe, die besteht aus -O-, -S-, -N(R⁷)-, -(CH₂)ₘ-, -C(O)-, -CH(OH)-, -(CH₂)ₘO-, -(CH₂)ₘS-, -(CH₂)ₘN(R⁷)-, -O(CH₂)ₘ-; - CHX^{a}-, -CX^{a}₂-, -S-(CH₂)ₘ-, -CR^{a}R^{b}- und -N(R⁷)(CH₂)ₘ wobei m = 1 - 3, X^{a} Halogen, q 1 und R^{a} und R^{b} wie in Anspruch 1 definiert sind, und R⁷ gewählt ist aus der Gruppe, die besteht aus Wasserstoff, C₁₋₁₀Alkyl, C₁₋₁₀Alkoxy C₂₋₁₀Alkenyl, C₁₋₁₀Alkenoyl, bis zu perhalogensubstituiertem C₁₋₁₀Alkyl, bis zu perhalogensubstituiertem C₁₋₁₀Alkoxy, bis zu perhalogensubstituiertem C₂₋₁₀Alkenyl und bis zu perhalogensubstituiertem C₁₋₁₀Alkenoyl.

3. Verbindung der Formel I oder pharmazeutisch verträgliches Salz davon zur Verwendung in einem Verfahren zur Behandlung einer Krankheit nach Anspruch 2, wobei -L- in der Formel -L-(ML¹)_{q} für B eine substituierte 6-gliedrige zyklische Aryleinheit, eine substituierte 5 - 6-gliedrige heterozyklische Einheit, eine unsubstituierte 6-gliedrige zyklische Aryleinheit oder eine unsubstituierte 5- oder 6-gliedrige heterozyklische Einheit ist, und L¹ in der Formel -L-(ML¹)ₐ für B eine substituierte Aryleinheit mit wenigstens 6 zyklischen Gliedern, eine unsubstituierte Aryleinheit mit wenigstens 6 zyklischen Gliedern, eine substituierte Heteroaryleinheit mit wenigstens 6 zyklischen Gliedern oder eine unsubstituierte Heteroaryleinheit mit wenigstens 6 zyklischen Gliedern ist, die heterozyklischen Einheiten und Hetaryleinheiten mit 1 bis 4 Gliedern gewählt sind aus der Gruppe von Heteroatomen, die besteht aus Stickstoff, Sauerstoff und Schwefel, wobei der Ausgleich der hetarylischen und heterozyklischen Einheit Kohlenstoff ist.

4. Verbindung der Formel I oder pharmazeutisch verträgliches Salz davon zur Verwendung in einem Verfahren zur Behandlung einer Krankheit nach Anspruch 1, wobei M in der Formel -L-(ML¹) für B -O-, -CH₂-, -S-, -NH-, -C(O)-, -O-CH₂- oder - CH₂-O- ist.

5. Verbindung der Formel I oder pharmazeutisch verträgliches Salz davon zur Verwendung in einem Verfahren zur Behandlung einer Krankheit nach Anspruch 1, wobei A 1 - 3 Substituenten hat, die gewählt sind aus der Gruppe, die besteht aus C₁₋₁₀Alkyl, bis zu perhalogensubstituiertem C₁₋₁₀Alkyl, CN, -OH, Halogen, C₁₋₁₀Alkoxy, bis zu perhalogensubstituiertem C₁₋₁₀Alkoxy, und heterozyklischen C₃₋₁₀Einheiten mit wenigstens 5 zyklischen Gliedern und 1 bis 2 Heteroatomen, die aus der aus Stickstoff, Sauerstoff und Schwefel bestehenden Gruppe gewählt sind.

6. Verbindung der Formel I oder pharmazeutisch verträgliches Salz davon zur Verwendung in einem Verfahren zur Behandlung einer Krankheit nach Anspruch 1, wobei L¹ 1- bis 3-fach substituiert ist durch einen oder mehrere Substituenten, die gewählt sind aus der Gruppe, die besteht aus -CN, Halogen bis Perhalogen, C₁₋₁₀Alkyl, C₁₋₁₀Alkoxy, -OH, bis zu perhalogensubstituiertem C₁₋₁₀ Alkyl, bis zu perhalogensubstituiertem C₁₋₁₀Alkoxy, -OR⁷, SR⁷, -NR⁷R^{7'}, -CO₂R⁷, -C(O)NR⁷R^{7'}, -C(O)R⁷ oder -NO₂, wobei jeder R⁷ und R^{7'} unabhängig gewählt ist aus Wasserstoff, C₁₋₁₀Alkyl, C₁₋₁₀Alkoxy, C₂₋₁₀Alkenyl, C₁₋₁₀Alkenoyl, bis zu perhalogensubstituiertem C₁₋₁₀Alkyl, bis zu perhalogensubstituiertem C₁₋₁₀Alkoxy, bis zu perhalogensubstituiertem C₂₋₁₀Alkenyl und bis zu perhalogensubstituiertem C₁₋₁₀Alkenoyl.

7. Verbindung der Formel I oder pharmazeutisch verträgliches Salz davon zur Verwendung in einem Verfahren zur Behandlung einer Krankheit nach Anspruch 1, wobei ein pharmazeutisch verträgliches Salz einer Verbindung der Formel I verabreicht wird, welches gewählt ist aus der Gruppe, die besteht aus
a) basischen Salzen von organischen und anorganischen Säuren, gewählt aus der Gruppe, die besteht aus Salzsäure, Bromwasserstoff, Schwefelsäure, Phosphorsäure, Methansulfonsäure, Trifluorsulfonsäure, Benzoesoulfonsäure, p-Toluolsulfonsäure (Tosylatsalz), 1-Napthalinsulfonsäure, 2-Napthalinsulfonsäure, Essigsäure, Trifluoressigsäure, Apfelsäure, Weinsäure, Zitronensäure, Milchsäure, Oxalsäure, Succinsäure, Fumarsäure, Maleinsäure, Benzoesäure, Salicylsäure, Phenylessigsäure und Mandelsäure, und
b) sauren Salzen von organischen und anorganischen Basen, die Kationen enthalten, die gewählt sind aus der Gruppe, die besteht aus Alkalikationen, Erdalkalikationen, Ammoniumkationen, aliphatisch substituierten Ammoniumkationen und aromatisch substituierten Ammoniumkationen.

8. Verbindung der Formel I oder pharmazeutisch verträgliches Salz davon zur Verwendung in einem Verfahren zur Behandlung einer Krankheit nach Anspruch 1 zur Behandlung einer anderen Krankheit als Krebs.

9. Verbindung der Formel I oder pharmazeutisch verträgliches Salz davon zur Verwendung in einem Verfahren zur Behandlung einer Krankheit nach Anspruch 1, wobei der Zustand in einem durch Verabreichung einer Verbindung der Formel I behandelten Wirt rheumatoide Arthritis, Osteoarthritis, septische Arthritis, Tumormetastase, Periodontalkrankheit, Cornealgeschwür, Albuminurie, Koronarthrombose von arteriosklerotischen Plaques, Aortenaneurysma, Geburtskontrolle, dystrophe Epidermolysis bullosa, degenerativer Knorpelschwund in Folge traumatischer Gelenksverletzung, durch MMP-Aktivität vermittelte Osteopenie, temporomandibulare Gelenkskrankheit oder demyelierende Krankheit des Nervensystems ist.

10. Verbindung der Formel I oder pharmazeutisch verträgliches Salz davon zur Verwendung in einem Verfahren zur Behandlung einer Krankheit nach einem der Ansprüche 1 bis 8, wobei der Zustand in einem durch Verabreichung einer Verbindung der Formel I behandelten Wirt rheumatisches Fieber, Knochenresorption, postmenopausale Osteoporose, Sepsis, gram-negative Sepsis, septischer Schock, endotoxischer Schock, toxisches Schocksyndrom, systemisches Entzündungsantwortssyndrom, entzündliche Darmkrankheit (Crohn-Krankheit und Kolitisgeschwür), Jarisch-Herxheimer-Reaktion, Asthma, adultes Atemnotsyndrom, akute Lungenfibrosekrankheit, pulmonale Sarkoidose, allergische Atemwegskrankheit, Silikosis, Kohlenarbeiter-Pneumokoniose, alveolare Verletzung, hepatitisches Versagen, Leberkrankheit bei akuter Entzündung, schwere alkoholische Hepatitis, Malaria (Plasmodium falciparum Malaria und Zerebralmalaria), nicht insulinabhängiger Diabetes mellitus (NIDDM), kongestives Herzversagen, Schädigung in Folge von Herzkrankheit, Arteriosklerose, Alzheimer, akute Enzephalitis, Gehirnverletzung, Multiple Sklerose (Demylierung und oligodendrocytischer Verlust bei Multipler Sklerose), fortgeschrittener Krebs, lymphoide Malignität, Pankreatitis, beeinträchtigte Wundheilung bei Infektion, Entzündung und Krebs, myelodysplastische Syndrome, systemischer Lupus erythematosus, biliäre Zirrhose, Darmnekrose, Psoriasis, Strahlenverletzung/Toxizität in Folge von Verabreichung von monoklonalen Antikörpern, Wirt-gegen-Transptantat-Reaktion (Ischämie-Reperfusionsverletzung und allogene Abstoßungen von Niere, Leber, Herz und Haut), allogene Lungenabstoßung (Bronchitis obliterans) oder Komplikationen aufgrund vollständigen Hüftaustauschs ist.

11. Verbindung der Formel I oder pharmazeutisch verträgliches Salz davon zur Verwendung in einem Verfahren zur Behandlung einer Krankheit nach einem der Ansprüche 1 bis 8, wobei der Zustand in einem durch Verabreichung einer Verbindung der Formel I behandelten Wirt eine Infektionskrankheit ist, die gewählt ist aus der Gruppe, die besteht aus Tuberkulose, Helicobacter-pylori-Infektion bei Magengeschwürkrankheit, Chaga-Krankheit, welche aus Trypanosoma-cruzi-Infektion resultiert, Wirkungen von Shiga-artigem Toxin, die aus E.coli-Infektion resultieren, Wirkungen von Enterotoxin A, die aus Staphylococcus-Infektion resultieren, meningococcale Infektion und Infektionen aus Borrelia burgdorferi, Treponema pallidum, Zytomegalovirus, Grippevirus, Theiler-Enzephalomyelitis-Virus und dem menschlichen Immunschwächevirus (HIV).

12. Verbindung der Formel I oder pharmazeutisch verträgliches Salz davon zur Verwendung in einem Verfahren zur Behandlung einer Krankheit nach Anspruch 1, wobei:
Rₐ und R_{b}
a) unabhängig Wasserstoff,
eine kohlenstoffbasierte Einheit, die gewählt ist aus der Gruppe, die besteht aus C₁₋₁₀Alkyl, C₁₋₁₀ Alkoxy, C₃₋₁₀Cycloalkyl mit 1 - 3 Heteroatomen, die unter N, S und O gewählt sind, C₂₋₁₀ Alkenyl, C₁₋₁₀Alkenoyl, C₆₋₁₄ Aryl, C₃₋₁₂Hetaryl mit 1 - 3 Heteroatomen, die unter O, N und S gewählt sind, C₇₋₂₄ Aralkyl, C₇ -C₂₄Alkaryl, substituiertes C₁₋₁₀Alkyl, substituiertes C₁₋₁₀Alkoxy, substituiertes C₃₋₁₀Cykloalkyl mit 0 - 3 Heteroatomen, die unter N, S und O gewählt sind, substituiertes C₆₋₁₄Aryl, substituiertes C₃₋₁₂ Hetaryl mit 1 - 3 Heteroatomen, die unter N, S und O gewählt sind, substituiertes C₇₋₂₄Aralkyl, substituiertes C₇₋₂₄Alkaryl, wenn Rₐ und R_{b} eine substituierte Gruppe sind, sie substituiert sind durch Halogen bis zu Perhalogen, Hydroxy, C₁₋₁₀Alkyl, C₃₋₁₂Cycloalkyl mit 0-3 Heteroatomen, die unter O, S und N gewählt sind, C₃-₁₂Hetaryl mit 1 - 3 Heteroatomen, die unter N, S und O gewählt sind, C₁₋₁₀Alkoxy, C₆₋₁₂Aryl, halogensubsituiertes bis zu perhalogensubstituiertes C₁₋₆Alkyl, halogensubsituiertes bis perhalogensubstituiertes C₆₋₁₂Aryl, halogensubstituiertes bis zu perhalogensubstituiertes C₃-C₁₂Cycloalkyl mit 0 - 3 Heteroatomen, die unter N, S und O gewählt sind, halogen- bis perhalogensubstituiertes C₃-C₁₂Hetaryl, halogen- bis perhalogensubstituiertes C₇-C₂₄Aralkyl, halogen- bis perhalogensubstituiertes C₇-C₂₄Alkaryl und -C(O)R_{g}, oder
-OSi(R_{f})₃ sind, wobei R_{f} Wasserstoff, C₁₋₁₀Alkyl, C₁₋₁₀Alkyl, C₁₋₁₀Alkoxy, C₃₋₁₀Cycloalkyl mit 0 - 3 Heteroatomen, die unter O, S und N gewählt sind, C₆₋₁₂Aryl, C₃₋₁₂Hetaryl mit 1 - 3 Heteroatomen, die unter O, S und N gewählt sind, C₇₋₂₄Aralkyl, substituiertes C₁₋₁₀Alkyl, substituiertes C₁₋₁₀Alkoxy, substituiertes C₃₋₁₂cycloalkyl mit 0 - 3 Heteroatomen, die unter O, S und N gewählt sind, substituiertes C₃₋₁₂Hetaryl, mit 1 - 3 Heteroatomen, die unter O, S und N gewählt sind, substituiertes C₆₋₁₂Aryl und substituiertes C₇₋₂₄ Alkaryl ist, wobei R_{f} eine substituierte Gruppe ist, die substituiert ist durch Halogen bis Perhalogen, Hydroxy, C₁₋₁₀Alkyl, C₃₋₁₂Cycloalkyl mit 0 - 3 Heteroatomen, die unter O, S und N gewählt sind, C₃₋₁₂Hetaryl, mit 1 - 3 Heteroatomen, die unter O, S und N gewählt sind, C ₁₋₁₀Alkoxy, C₆₋₁₂Aryl, C_{y-}-C₂₄Alkaryl, C₇-C₂₄Aralkyl, halogen- bis perhalogensubstituiertes C₁₋₆Alkyl, halogen- bis perhalogensubstituiertes C₆-C₁₂Aryl, halogensubstituiertes C₃-C₁₂Cykloalkyl mit 0 - 3 Heteroatomen, die unter N, S und O gewählt sind, bis perhalogensubstituiertes Cykloalkyl, halogensubstituiertes bis perhalogensubstituiertes C₃₋₁₂Hetaryl, halogensubstituiertes bis perhalogensubstituiertes C₇-C₂₄Aralkyl, halogensubstituiertes bis perhalogensubstituiertes C₇-C₂₄Alkaryl und -C(O)R_{g},
oder
b) Rₐ und R_{b} zusammen eine 5 - 7-gliedrige heterozyklische Struktur mit 1 - 3 Heteroatomen bilden, die unter N, S und O gewählt sind, oder eine substituierte 5 - 7-gliedrige heterozyklische Struktur mit 1 - 3 Heteroatomen bilden, die unter N, S und O gewählt sind, mit Substituenten, die gewählt sind aus der Gruppe, die besteht aus Halogen bis Perhalogen, Hydroxy, C₁₋₁₀Alkyl, C₃₋₁₂ Cycloalkyl mit 0 - 3 Heteroatomen, die unter O, S und N gewählt sind, C₃₋₁₂Hetaryl mit 1 - 3 Heteroatomen, die gewählt sind aus N, S und O, C₁₋₁₀Alkoxy, C₆₋₁₂ Aryl, C₇₋₂₄Alkaryl, C₇-C₂₄Aralkyl, halogensubstituiertes bis perhatogensubstituiertes C₁₋₆ Alkyl, halogensubstituiertes bis perhalogensubstituiertes C₆₋₁₂Aryl, halogensubstituiertes bis perhalogensubstituiertes C₃-C₁₂Cykloalkyl mit 0 - 3 Heteroatomen, die aus N, S und O gewählt sind, halogensubstituiertes bis perhalogensubstituiertes C₃₋₁₂Hetaryl, halogensubstituiertes bis perhalogensubstiuiertes C₇-C₁₂Aralkyl, halogensubstituiertes bis perhalogensubstituiertes C₇-C₂₄Alkaryl und -C(O)R_{g},
oder
c) eines von Rₐ oder R_{b} -C(O)-, eine divalente C₁₋ C₅ Alkenylgruppe oder eine substituierte divalente C₁₋C₅Alkenylgruppe ist, die an eine Einheit L gebunden ist, um eine zyklische Struktur mit wenigstens fünf Gliedern zu bilden,
wobei die Substituenten der substituierten divalenten C₁₋C₅Alkenylgruppe gewählt sind aus der Gruppe, die besteht aus Halogen, Hydroxy, C₁₋₁₀Alkyl, G₃₋₁₂Cycloalkyl mit 0 - 3 Heteroatomen, die unter O, S und N gewählt sind, C₃₋₁₂Hetaryl mit 1 - 3 Heteroatomen, die unter N, S und O gewählt sind, C₁₋₁₀Alkoxy, C₆₋₁₂Aryl, C₇-C₂₄Alkaryl, C₇-C₂₄Aralkyl, halogensubstituiertes bis perhalogensubstituiertes C₁₋₆Alkyl, halogensubstituiertes bis perhalogensubstituiertes C₆-C₁₂Aryl, halogensubstituiertes bis perhalogensubstituiertes C₃-C₁₂Cykloalkyl mit 0 - 3 Heteroatomen, die aus N, S und O gewählt sind, halogensubstituiertes bis perhalogensubstituiertes C₃-C₁₂Hetaryl, halogensubstituiertes bis perhalogensubstituiertes C₇-C₂₄Aralkyl, halogensubstituiertes bis perhalogensubstituiertes C₇-C₂₄Alkaryl und -C(O)R_{g},
wobei R_{g} C₁₋₁₀Alkyl, -CN, -CO₂R_{d}, -OR_{d}, -SR_{d}, -NO₂, -C(O)Rₑ, -NR_{d}Rₑ, -NR_{d} C(O)ORₑ und -NR_{d}C(O)Rₑ ist und R_{d} und Rₑ unabhängig gewählt sind aus der Gruppe, die besteht aus Wasserstoff, C₁₋₁₀Alkyl, C₁₋₁₀Alkoxy, C₃₋₁₀Cycloalkyl mit 0 - 3 Heteroatomen, die unter O, N und S gewählt sind, C₆₋₁₂Aryl, C₃- C₁₂Hetaryl mit 1 - 3 Heteroatomen, die unter O, N und S gewählt sind, und C₇-C₂₄Aralkyl, C₇-C₂₄Alkaryl, bis perhalogensubstituiertes C₁-C₁₀Alkyl, bis perhalogensubstituiertes C₃-C₁₀Cycloalkyl mit 0 - 3 Heteroatomen, die unter O, N und S gewählt sind, bis perhalogensubstituiertes C₆-C₁₄Aryl, bis perhalogensubstituiertes C₃-C₁₂Hetaryl mit 1 - 3 Heteroatomen, die unter O, N und S gewählt sind, halogensubstituiertes bis perhalogensubstituiertes C₇-C₂₄Alkaryl und bis perhalogensubstituiertes C₇-C₂₄Aralkyl.

13. Verbindung der Formel I oder pharmazeutisch verträgliches Salz davon zur Verwendung in einem Verfahren zur Behandlung einer Krankheit nach Anspruch 2, wobei die substituierte zyklische Einheit L¹ Phenyl, Pyridyl oder Pyrimidinyl ist.

14. Verbindung der Formel I oder pharmazeutisch verträgliches Salz davon zur Verwendung in einem Verfahren zur Behandlung einer Krankheit nach Anspruch 1, wobei L¹ substituiert ist durch -C(O)NR^{a}R^{b} oder -SO₂ NR^{a}R^{b}.

15. Verwendung einer Verbindung der Formel I:
A-D-B (1)
oder pharmazeutisch verträgliches Salz davon zur Herstellung eines Medikaments zur Behandlung einer Krankheit, die durch p38 in einem Wirt vermittelt wird, wobei die durch p38 in einem Wirt vermittelte Krankheit rheumatoide Arthritis, Osteoarthritis, septische Arthritis, Tumormetastase, Periodontalkrankheit, Cornealgeschwür, Albuminurie, Koronarthrombose von arteriosklerotischen Plaques, Aortenaneurysma, Geburtskontrolle, dystrophe Epidermolysis bullosa, degenerativer Knorpelschwund in Folge traumatischer Gelenksverletzung, durch MMP-Aktivität vermittelte Osteopenie, temporomandibulare Gelenkskrankheit, demyelierende Krankheit des Nervensystems, rheumatisches Fieber, Knochenresorption, postmenopausale Osteoporose, Sepsis, gram-negative Sepsis, septischer Schock, endotoxischer Schock, toxisches Schocksyndrom, systemisches Entzündungsantwortssyndrom, entzündliche Darmkrankheit (Crohn-Krankheit und Kolitisgeschwür), Jarisch-Herxheimer-Reaktion, Asthma, adultes Atemnotsyndrom, akute Lungenfibrosekrankheit, pulmonale Sarkoidose, allergische Atemwegskrankheit, Silikosis, Kohlenarbeiter-Pneumokoniose, alveolare Verletzung, hepatitisches Versagen, Leberkrankheit bei akuter Entzündung, schwere alkoholische Hepatitis, Malaria (Plasmodium falciparum Malaria und Zerebralmalaria), nicht insulinabhängiger Diabetes mellitus (NIDDM), kongestives Herzversagen, Sehädigung in Folge von Herzkrankheit, Arteriosklerose, Alzheimer, akute Enzephalitis, Gehirnverletzung, Multiple Sklerose (Demylierung und oligodendrocytischer Verlust bei Multipler Sklerose), fortgeschrittener Krebs, lymphoide Malignität, Pankreatitis, beeinträchtigte Wundheilung bei Infektion, Entzündung und Krebs, myelodysplastische Syndrome, systemischer Lupus erythematosus, biliäre Zirrhose, Darmnekrose, Psoriasis, Strahlenverletzung/Toxizität in Folge von Verabreichung von monoklonalen Antikörpern, Wirt-gegen-Transplantat-Reaktion (Ischämie-Reperfusionsverletzung und allogene Abstoßungen von Niere, Leber, Herz und Haut), allogene Lungenabstoßung (Bronchitis obliterans), Komplikationen aufgrund vollständigen Hüftaustauschs, Tuberkulose, Helicobacter-pylori-Infektion bei Magengeschwürkrankheit, Chaga-Krankheit, welche aus Trypanosoma-cruzi-Infektion resultiert, Wirkungen von Shiga-artigem Toxin, die aus E.coli-Infektion resultieren, Wirkungen von Enterotoxin A, die aus Staphylococcus-Infektion resultieren, meningococcale Infektion und Infektionen aus Borrelia burgdorferi, Treponema pallidum, Zytomegalovirus, Grippevirus, Theiler-Enzephalomyelitis-Virus oder der menschliche Immunschwächevirus (HIV) ist; wobei
D -NH-C(O)-NH- ist,
A eine gegebenenfalls substituierte Quinolinyl- oder Isoquinolinyl-Gruppe ist,
B eine verbrückte zyklische Struktur der Formel -L-(ML¹)_{q} ist, wobei L eine 5- oder 6-gliedrige zyklische Struktur ist, die direkt an D gebunden ist, L¹ eine substituierte zyklische Einheit mit wenigstens 5 Gliedern umfaßt, M eine Verbrückungsgruppe mit wenigstens einem Atom ist, q eine ganze Zahl von 1 - 3 ist und jede zyklische Struktur von L und L¹ 0 - 4 Glieder der Gruppe enthält, die aus Stickstoff, Sauerstoff und Schwefel besteht, wobei L¹ substituiert ist durch wenigstens einen Substituenten, der aus der Gruppe gewählt ist, die aus -SO₂R^{a} -SO₂NR^{a}R^{b}, -C(O)R^{a}, -C(O)NR^{a}R^{b} und -C(NR^{a})R^{b} besteht, wobei R^{a} und R^{b} unabhängig Wasserstoff oder eine kohlenstoffbasierte Einheit sind,
wobei die Substituenten für A aus der Gruppe gewählt sind, die aus Halogen bis Perhalogen und Wn besteht, wobei n 0 - 3 ist und jedes W unabhängig aus der Gruppe gewählt ist, die besteht aus
C₁₋₁₀Alkyl, C₁₋₁₀Alkoxy, C₃₋₁₀Zykloalkyl mit wenigstens 5 zyklischen Gliedern und 0-3 Heteroatomen, die unter N, S und O gewählt sind, C₂₋₁₀Alkenyl, C₁₋₁₀Alkenoyl, C₆-C₁₄Aryl, C₇-C₂₄Alkaryl, C₇-C₂₄Aralkyl, G₃-C₁₂Heteroaryl mit wenigstens 5 zyklischen Gliedern und 1 - 3 Heteroatomen, die unter O, N und S gewählt sind, C₄-C₂₄Alkheteroaryl mit wenigstens 5 zyklischen Gliedern und 1 - 3 Heteroatomen, die unter O, N und S gewählt sind,
substituiertes C₁₋₁₀Alkyl, substituiertes C₁₋₁₀Alkoxy, substituiertes C₃₋₁₀Zykloalkyl mit wenigstens 5 zyklischen Gliedern und 0 - 3 Heteroatomen, die unter N, S und O gewählt sind, substituiertes C₂₋₁₀Alkenyl, substituiertes C₁₋₁₀Alkenoyl, substituiertes C₆-C₁₄Aryl, substituiertes C₇-C₂₄ Alkylaryl, substituiertes C₇-C₂₄Aralkyl, substituiertes C₃-C₁₂Heteroaryl mit wenigstens 5 Gliedern und 1 - 3 Heteroatomen, die unter O, N und S gewählt sind, substituiertes C₄-C₂₄ Alkheteroaryl mit wenigstens 5 Gliedern und 1 - 3 Heteroatomen, die unter O, N und S gewählt sind,
-CN, -CO₂R⁷, -C(O)NR⁷R^{7'}, -C(O)-F⁷, -NO₂, -OR⁷, -SR⁷, -NR⁷R^{7'}, - NR⁷C(O)OR^{7'}, -NR⁷ C(O)R^{7'}, mit jedem R⁷ and R^{7'} unabhängig gewählt aus Wasserstoff C₁₋₁₀Alkyl, C₁₋₁₀Alkoxy, C₂₋₁₀Alkenyl, C₁₋₁₀Alkenoyl und bis zu perhalogensubstituiertem C₁₋₁₀Alkyl, bis zu perhalogensubstituiertem C₁₋₁₀Alkoxy, bis zu perhalogensubstituiertem C₂₋₁₀Alkenyl und bis zu perhalogensubstituiertem C₁₋₁₀Alkenoyl, C₃-C₁₀Zykloalkyl mit wenigstens 5 zyklischen Gliedern und 0 - 3 Heteroatomen, die unter O, S und N gewählt sind, C₆-C₁₄Aryl, C₃-C₁₀Hetaryl mit wenigstens 6 zyklischen Gliedern und 0 - 3 Heteroatomen, die unter O, S und N gewählt sind, bis zu perhalogensubstituiertem C₃-C₁₀Zykloalkyl mit wenigstens 5 zyklischen Gliedern und 0 - 3 Heteroatomen, die unter O, S und N gewählt sind, bis zu perhalogensubstituiertem C₆-C₁₄Aryl und bis zu perhalogensubstituiertem C₃-C₁₀Hetaryl mit wenigstens 6 zyklischen Gliedern und 0 - 3 Heteroatomen, die unter O, S und N gewählt sind,
wo W eine substituierte Gruppe ist, sie durch Halogen bis zu Perhalogen oder durch einen oder mehrere Substituenten substituiert ist, die unabhängig gewählt sind aus der Gruppe, die aus -CN, -CO₂R⁷, -C(O)NR⁷R^{7'}, -C(O)-R⁷ -NO₂, -OR⁷, -SR⁷, - NR⁷R^{7'}, -NR⁷C(O)OR^{7'} und -NR⁷C(O)R^{7'} besteht, wobei R⁷ und R^{7'} unabhängig wie oben definiert sind.

16. Verbindung, die gewählt ist aus der Gruppe, die besteht aus
*N*-(2-Methoxy-3-Quinolyl)-N'-(4-[3-(N-Methylcarbamoyl)Phenoxy]Phenyl)-Harnstoff
*N*-(2-Methoxy-3-Quinolyl)-N'-(4-[2-(N-Methylcarbamoyl)-4-Pyridyloxy]Phenyl)-Harnstoff
*N*-(2-Methoxy-3-Quinolyl)-N'-(4-(2-Carbamoyl-4-Pyridyloxy)Phenyl)-Harnstoff
*N*-(2-Methoxy-3-Quinolyl)-N'-(3-[2-(N-Methylcarbamoyl)-4-Pyridyloxy]Phenyl)-Harnstoff
*N*-(2-Methoxy-3-Quinolyl)-N'-(3-(2-Carbamoyl)-4-Pyridyloxy)Phenyl- Harnstoff
*N*-(2-Methoxy-3-Quinolyl)-N'-(4-[3-(N-Isopropylcarbamoyl) Phenoxy]Phenyl)-Harnstoff
*N*-(2-Methoxy-3-Quinolyl)-N'-(4-[4-Methoxy-3-(N-Methylcarbamoyl) Phenoxy]Phenyl)-Harnstoff
*N*-(3-Isoquinolyl)-N'-(4-[2-(N-Methylcarbamoyl)-4-Pyridyloxy]Phenyl)-Harnstoff
und pharmazeutisch verträgliche Salze davon,
zur Verwendung in einem Verfahren zur Behandlung einer Krankheit, die durch p38 Kinase vermittelt wird, wie definiert in Anspruch 1, welche von Krebs verschieden ist.

17. Verwendung einer Verbindung, gewählt ist aus der Gruppe, die besteht aus
*N*-(2-Methoxy-3-Quinolyl)-N'-(4-[3-(N-Methylcarbamoyl) Phenoxy]Phenyl)-Harnstoff
*N*-(2-Methoxy-3-Quinolyl)-N'-(4-[2-(N-Methylcarbamoyl)-4-Pyridyloxy]Phenyl)-Harnstoff
*N*-(2-Methoxy-3-Quinolyl)-N'-(4-(2-Carbamoyl-4-Pyridyloxy)Phenyl)-Harnstoff
*N*-(2-Methoxy-3-Quinolyl)-N'-(3-[2-(N-Methylcarbamoyl)-4-Pyridyloxy]Phenyl)-Harnstoff
*N*-(2-Methoxy-3-Quinolyl)-N'-(3-(2-Carbamoyl)-4-Pyridyloxy)Phenyl-Harnstoff
*N*-(2-Methoxy-3-Quinolyl)-N'-(4-[3-(N-Isopropylcarbamoyl) Phenoxy]Phenyl)-Harnstoff
*N*-(2-Methoxy-3-Quinolyl)-N'-(4-[4-Methoxy-3-(N-Methylcarbamoyl)Phenoxy]Phenyl)-Harnstoff
*N*-(3-Isoquinolyl)-N'-(4-[2-(N-Methylcarbamoyl)-4-Pyridyloxy]Phenyl)-Harnstoff
und pharmazeutisch verträgliche Salze davon,
zur Herstellung eines Medikaments zur Behandlung einer Krankheit, die durch p38 Kinase vermittelt wird, wie definiert in Anspruch 1, welche von Krebs verschieden ist.

18. Pharmazeutische Zusammensetzung zur Behandlung einer Krankheit, die durch p38 in einem Wirt vermittelt wird, umfassend eine Menge einer Verbindung der Formel I, die wirksam ist, um p38-vermittelte Ereignisse zu inhibieren,
A-D-B (1)
oder pharmazeutisch verträgliches Salz davon, in einer Menge, die wirksam ist, um eine durch p38 vermittelte Krankheit zu behandeln, und einen physiologisch verträglichen Carrier,
wobei
D -NH-C(O)-NH- ist,
A wie in Anspruch 1 definiert ist,
B eine verbrückte zyklische Struktur der Forme -L-(ML¹)q ist, wobei L eine 5- oder 6-gliedrige zyklische Struktur ist, die direkt an D gebunden ist, L¹ eine substituierte Einheit mit wenigstens 5 Gliedern umfaßt, M eine Verbrückungsgruppe mit wenigstens einem Atom ist, q eine ganze Zahl von 1 - 3 ist und jede zyklische Struktur von L und L¹ 0 - 4 Glieder der Gruppe enthält, die aus Stickstoff, Sauerstoff und Schwefel besteht, wobei L¹ substituiert ist durch wenigstens einen Substituenten, der aus der Gruppe gewählt ist, die aus -SO₂Rₓ, -C(O)Rₓ, und -C(NR_{y})R_{z} besteht, wobei R_{y} Wasserstoff oder eine kohlenstoffbasierte Einheit mit bis zu 24 Kohlenstoffatomen ist, welche optional Heteroatome, gewählt unter N, S und O, enthält und optional halogen- bis perhalogensubstituiert ist,
R_{z} Wasserstoff oder eine kohlenstoffbasierte Einheit mit bis zu 30 Kohlenstoffatomen ist, die optional Heteroatome, gewählt unter N, S und O, enthält und optional substituiert ist durch Halogen, Hydroxy, kohlenstoffbasierte Substituenten mit bis zu 24 Kohlenstoffatomen, welche optional Heteroatome enthalten, die gewählt sind unter N, S und O, und optional substituiert sind durch Halogen,
Rₓ R_{z} oder NRₐR_{b} ist, wobei Rₐ und R_{b}
a) unabhängig Wasserstoff,
eine kohlenstoffbasierte Einheit mit bis zu 30 Kohlenstoffatomen, die optional Heteroatome, gewählt unter N, S und O, enthält und optional substituiert ist durch Halogen, Hydroxy und kohlenstoffbasierte Substituenten mit bis zu 24 Kohlenstoffatomen, welche optional Heteroatome enthalten, die gewählt sind unter N, S und O, und optional substituiert sind durch Halogen, oder
-OSi(R_{f})₃ sind, wobei R_{f} Wasserstoff oder eine kohlenstoffbasierte Einheit mit bis zu 24 Kohlenstoffatomen ist, die optional Heteroatome, die unter N, S und O gewählt sind, enthält und optional substituiert ist durch Halogen, Hydroxy, kohlenstoffbasierte Substituenten mit bis zu 24 Kohlenstoffatomen, welche optional Heteroatome enthalten, die gewählt sind unter N, S und O, und optional substituiert sind durch Halogen, oder
b) Rₐ und R_{b} zusammen eine 5 - 7-gliedrige heterozyklische Struktur mit 1 - 3 Heteroatomen bilden, die unter N, S und O gewählt sind, oder eine substituierte 5 - 7-gliedrige heterozyklische Struktur mit 1 - 3 Heteroatomen, die unter N, S und O gewählt sind, substituiert durch Halogen, Hydroxy oder kohlenstoffbasierte Substituenten mit bis zu 24 Kohlenstoffatomen, welche optional Heteroatome enthalten, die gewählt sind unter N, S und O, und optional durch Halogen substituiert sind, oder
c) eine von Rₐ oder R_{b} -C(O)-, eine divalante C₁-C₅Alkenylgruppe oder eine substituierte divalente divalente C₁-C₅Alkenylgruppe ist, die an eine Einheit L gebunden ist, um eine zyklische Struktur mit wenigstens 5 Gliedern zu bilden, wobei die Substituenten der substituierten divalenten C₁-C₅Alkylengruppe gewählt sind aus der Gruppe, die besteht aus Halogen, Hydroxy und kohlenstoffbasierten Substituenten mit bis zu 24 Kohlenstoffatomen, welche optional Heteroatome enthalten, die gewählt sind aus N, S und O, und optional durch Halogen substituiert sind.

19. Pharmazeutische Zusammensetzung nach Anspruch 18, wobei die zyklischen Strukturen B und L, die direkt an D gebunden sind, nicht in Ortho-Position durch -OH oder eine Einheit substituiert sind, die einen ionisierbaren Wasserstoff und einen pKa von 10 oder weniger hat.

20. Pharmazeutische Zusammensetzung nach Anspruch 18, wobei B in Formel I eine gegebenenfalls substituierte 6-gliedrige Aryleinheit oder eine wenigstens 5-gliedrige heterozyklische Einheit ist, wobei die heterozyklische Einheit mit 1 bis 4 Gliedern gewählt ist aus der Gruppe aus Heteroatomen, die besteht aus Stickstoff, Sauerstoff und Schwefel, wobei die Vervollständigung der heterozyklischen Einheit Kohlenstoff ist.

21. Pharmazeutische Zusammensetzung nach Anspruch 18, wobei L, die 5- oder 6-gliedrige zyklische Struktur, die direkt an D gebunden ist, eine gegebenenfalls substituierte 6-gliedrige Heteroaryleinheit ist, wobei die Heteroaryleinheit 1 bis 4 Glieder hat, die gewählt sind aus der Gruppe von Heteroatomen, die besteht aus Stickstoff, Sauerstoff und Schwefel, wobei die Vervollständigung der Heteroaryleinheit Kohlenstoff ist, wobei der eine oder die mehreren Substituenten gewählt sind aus der Gruppe, die besteht aus Halogen und Wn, wobei W und n wie in Anspruch 18 definiert sind.

22. Pharmazeutische Zusammensetzung nach Anspruch 18, wobei L¹ substituiert ist durch -C(O)Rₓ.

23. Pharmazeutische Zusammensetzung nach Anspruch 18, wobei L¹ substituiert ist durch -C(O)Rₓ oder SO₂Rₓ, wobei Rₓ NRₐR_{b} ist.

24. Pharmazeutische Zusammensetzung zur Behandlung einer Krankheit, die in einem Wirt durch p38 vermittelt wird, umfassend eine Verbindung, die gewählt ist aus der Gruppe, die besteht aus
*N*-(2-Methoxy-3-Quinolyl)-N'-(4-[3-(N-Methylcarbamoyl) Phenoxy]Phenyl)-Harnstoff
*N*-(2-Methoxy-3-Quinolyl)-N'-(4-[2-(N-Methylcarbamoyl)-4-Pyridyloxy]Phenyl)-Harnstoff
*N*-(2-Methoxy-3-Quinolyl)-N'-(4-(2-Carbamoyl-4-Pyridyloxy)Phenyl)-Harnstoff
*N*-(2-Methoxy-3-Quinolyl)-N'-(3-[2-(N-Methylcarbamoyl)-4-Pyridyloxy]Phenyl)-Harnstoff
*N*-(2-Methoxy-3-Quinolyl)-N'-(3-(2-Carbamoyl)-4-Pyridyloxy)Phenyl- Harnstoff
*N*-(2-Methoxy-3-Quinolyl)-N'-(4-[3-(N-Isopropylcarbamoyl)Phenoxy]Phenyl)-Harnstoff
*N*-(2-Methoxy-3-Quinolyl)-N'-(4-[4-Methoxy-3-(N-Methylcarbamoyl)Phenoxy]Phenyl)-Harnstoff
*N*-(3-Isoquinolyl)-N'-(4-[2-(N-Methylcarbamoyl)-4-Pyridyloxy]Phenyl)-Harnstoff
und pharmazeutisch verträgliche Salze davon.

25. Verwendung einer Zusammensetzung nach einem der Ansprüche 18 bis 24 zur Herstellung eines Medikaments zur Behandlung einer in einem Wirt durch p38 vermittelten Krankheit, wobei die durch p38 vermittelte Krankheit wie in Anspruch 1 definiert ist.

## Revendications

1. Composé de Formule I :
A-D-B (I)
ou sel pharmaceutiquement acceptable de celui-ci à utiliser dans un procédé de traitement d'une maladie associée à la p38 au sein d'un hôte, la maladie associée à la p38 au sein d'un hôte étant la polyarthrite rhumatoïde, l'arthrose, l'arthrite purulente, la métastase tumorale, la parodontopathie, l'ulcère cornéen, la protéinurie, la thrombose coronaire associée à une plaque athéroscléreuse, l'anévrisme aortique, la contraception, l'épidermolyse bulleuse dystrophique, la perte dégénérative de cartilage suite à une lésion traumatique des articulations, les ostéopénies liées à l'activité des MMP, le syndrome de l'articulation temporo-mandibulaire, la démyélinisation du système nerveux, le rhumatisme articulaire aigu, la résorption osseuse, l'ostéoporose post-ménopausique, la sepsie, la sepsie gram négative, le choc infectieux, le choc endotoxique, le choc toxique staphylococcique, le syndrome de réponse inflammatoire systémique, l'affection abdominale inflammatoire (la maladie de Crohn et la recto-colite hémorragique), la réaction de Jarisch-Herxheimer, l'asthme, le syndrome de détresse respiratoire de l'adulte, la fibrose pulmonaire aiguë, la sarcoïdose pulmonaire, la maladie respiratoire allergique, la silicose, l'anthracose, la lésion alvéolaire, l'insuffisance hépatique, la maladie du foie lors d'une inflammation aiguë, l'hépatite alcoolique sévère, la malaria (la malaria due au Plasmodium falciparum et la malaria cérébrale), le diabète de type 2 (NIDDM), l'insuffisance cardiaque congestive, les séquelles de la cardiopathie, l'athérosclérose, la maladie d'Alzheimer, l'encéphalite aiguë, la lésion cérébrale, la sclérose en plaques (démyélinisation et perte des oligodendrocytes dans la sclérose en plaques), le cancer à un stade avancé, la tumeur maligne du système lymphoïde, la pancréatite, l'altération de la cicatrisation lors d'une infection, l'inflammation et le cancer, les syndromes myélodysplasiques, le lupus érythémateux systémique, la cirrhose biliaire, la nécrose intestinale, le psoriasis, le mal des rayons/la toxicité suite à l'administration d'anticorps monoclonaux, la réaction hôte contre greffon (lésion due à l'ischémie-reperfusion et rejet d'allogreffe du rein, du foie, du coeur et de la peau), le rejet d'allogreffe du poumon (bronchite oblitérante), les complications dues au remplacement total de la hanche, la tuberculose, l'infection causée par Helicobacter pylori suite à un ulcère gastroduodénal, la maladie de Chaga suite à une infection causée par Trypanosoma cruzi, les effets d'une toxine de type Shiga dus à une infection par E. coli, les effets de l'entérotoxine A dus à une infection par Staphylococcus, la méningococcie et les infections dues à Borrelia burgdorferi, à Treponema pallidum, au cytomégalovirus, au virus de la grippe, au virus de l'encéphalomyélite de Theiler ou au virus de l'immunodéficience humaine (VIH) ; dans laquelle
D représente un groupe -NH-C(O)-NH-,
A représente un groupe quinolinyle ou isoquinolinyle substitué ou non substitué,
B représente une structure cyclique pontée de formule -L-(ML¹)_{q}, où L représente une structure cyclique de 5 ou 6 chaînons reliée directement à D, L¹ comprend un groupe fonctionnel cyclique substitué comportant au moins 5 chaînons, M représente un groupe de pontage comportant au moins un atome, q représente un nombre entier de 1 à 3, et chaque structure cyclique de L et L¹ contient de 0 à 4 membres du groupe constitué de l'azote, de l'oxygène et du soufre, dans lequel L¹ est substitué par au moins un substituant choisi dans le groupe constitué des groupes -SO₂R^{a}, -SO₂NR^{a}R^{b}, -C(O)R^{a}, -C(O)NR^{a}R^{b} et -C(NR^{a})R^{b}, dans lesquels R^{a} et R^{b} représentent indépendamment un atome d'hydrogène ou un groupe fonctionnel à base de carbone,
dans lequel les substituants de A sont choisis dans le groupe constitué d'un groupe halogéno, jusqu'à per-halogéno, et Wn, où n vaut de 0 à 3 et chaque W est indépendamment choisi dans le groupe constitué des groupes
alkyle en C₁ à C₁₀, alcoxy en C₁ à C₁₀, cycloalkyle en C₃ à C₁₀ comportant au moins cinq chaînons cycliques et de 0 à 3 hétéroatomes choisis parmi N, S et O ; alcényle en C₂ à C₁₀, alcénoyle en C₁ à C₁₀, aryle en C₆ à C₁₄, alkaryle en C₇ à C₂₄, aralkyle en C₇ à C₂₄, hétéroaryle en C₃ à C₁₂ comportant au moins 5 chaînons cycliques et de 1 à 3 hétéroatomes choisis parmi O, N et S, alkhétéroaryle en C₄ à C₂₄ comportant au moins 5 chaînons cycliques et de 1 à 3 hétéroatomes choisis parmi O, N et S ;
alkyle en C₁ à C₁₀ substitué, alcoxy en C₁ à C₁₀ substitué, cycloalkyle en C₃ à C₁₀ substitué comportant au moins 5 chaînons cycliques et de 0 à 3 hétéroatomes choisis parmi N, S et O ; alcénoyle en C₂ à C₁₀ substitué, alcénoyle en C₁ à C₁₀ substitué, aryle en C₆ à C₁₄ substitué, alkaryle en C₇ à C₂₄ substitué, aralkyle en C₇ à C₂₄ substitué, hétéroaryle en C₃ à C₁₂ substitué comportant au moins 5 chaînons et de 1 à 3 hétéroatomes choisis parmi O, N et S, alkhétéroaryle en C₄ à C₂₄ substitué comportant au moins 5 chaînons et de 1 à 3 hétéroatomes choisis parmi O, N et S,
-CN, -CO₂R⁷, -C(O)NR⁷R^{7'}, -C(O)-R⁷, -NO₂, -OR⁷, -SR⁷, NR⁷R^{7'}, -NR⁷C(O)OR^{7'}, - NR⁷C(O)R^{7'}, chaque R⁷ et R^{7'} étant indépendamment choisi parmi un atome d'hydrogène, un groupe alkyle en C₁ à C₁₀, alcoxy en C₁ à C₁₀, alcényle en C₂ à C₁₀, alcénoyle en C₁ à C₁₀, alkyle en C₁ à C₁₀ jusqu'à per-halogénosubstitué, alcoxy en C₁ à C₁₀ jusqu'à per-halogénosubstitué, alcényle en C₂ à C₁₀ jusqu'à per-halogénosubstitué et alcénoyle en C₁ à C₁₀ jusqu`à per-halogénosubstitué, cycloalkyle en C₃ à C₁₀ comportant au moins 5 chaînons cycliques et de 0 à 3 hétéroatomes choisis parmi O, S et N, aryle en C₆ à C₁₄, hétaryle en C₃ à C₁₀ comportant au moins 6 chaînons cycliques et de 0 à 3 hétéroatomes choisis parmi O, S et N, cycloalkyle en C₃ à C₁₀ jusqu'à per-halogénosubstitué comportant au moins 5 chaînons cycliques et de 0 à 3 hétéroatomes choisis parmi O, S et N, aryle en C₆ à C₁₄ jusqu'à per-halogénosubstitué et hétaryle en C₃ à C₁₀ jusqu'à per-halogénosubstitué comportant au moins 6 chaînons cycliques et de 0 à 3 hétéroatomes choisis parmi O, S et N,
où, lorsque W représente un groupe substitué, il est substitué par un groupe halogéno, jusqu'à per-halogéno, ou par un ou plusieurs substituants indépendamment choisis dans le groupe constitué des groupes -CN, -CO₂R⁷, - C(O)NR⁷R^{7'}, -C(O)-R⁷, -NO₂, -OR⁷, -SR⁷, NR⁷R^{7'}, -NR⁷C(O)OR^{7'} et -NR⁷C(O)R^{7'}, où R⁷ et R^{7'} sont indépendamment tels que définis ci-dessus.

2. Composé de Formule I ou sel pharmaceutiquement acceptables de celui-ci à utiliser dans un procédé de traitement d'une maladie selon la revendication 1, dans lequel M dans la formule -L-(ML¹)_{q}, est choisi dans le groupe constitué des groupes - O-, -S-, -N(R⁷)-, -(CH₂)ₘ-, -C(O)-, -CH(OH)-, -( CH₂)ₘO-, -( CH₂)ₘS-, -( CH₂)ₘ N(R⁷)-, -O(CH₂)ₘ-; -CHX^{a}-, -CX^{a}₂-, -S-(CH₂)ₘ-, -CR^{a}R^{b}- et -N(R⁷)(CH₂)ₘ-, où m=1-3, X³ représente un atome d'halogène, q vaut 1, et R^{a} et R^{b} sont tels que définis dans la revendication 1, et R⁷ est choisi dans le groupe constitué de l'atome d'hydrogène et des groupes alkyle en C₁ à C₁₀, alcoxy en C₁ à C₁₀, alcényle en C₂ à C₁₀, alcénoyle en C₁ à C₁₀, alkyle en C₁ à C₁₀ jusqu'à per-halogénosubstitué, alcoxy en C₁ à C₁₀ jusqu'à per-halogénosubstitué, alcényle en C₂ à C₁₀ jusqu'à per-halogénosubstitué et alcénoyle en C₁ à C₁₀ jusqu'à per-halogénosubstitué.

3. Composé de Formule I ou sel pharmaceutiquement acceptable de celui-ci à utiliser dans un procédé de traitement d'une maladie selon la revendication 2, dans lequel L dans la formule -L-(ML¹)_{q} pour B représente un groupe fonctionnel aryle cyclique substitué de 6 chaînons, un groupe fonctionnel hétérocyclique substitué à 5 ou 6 chaînons, un groupe fonctionnel aryle cyclique non substitué de 6 chaînons, ou un groupe fonctionnel hétérocyclique non substitué à 5 ou 6 chaînons, et L¹ dans la formule -L-(ML¹)_{q} pour B représente un groupe fonctionnel aryle substitué comportant au moins 6 chaînons cycliques, un groupe fonctionnel aryle non substitué comportant au moins 6 chaînons cycliques, un groupe fonctionnel hétaryle substitué comportant au moins 6 chaînons cycliques ou un groupe fonctionnel hétaryle non substitué comportant au moins 6 chaînons cycliques, lesdits groupes fonctionnels hétérocycliques et hétaryles comportant de 1 à 4 chaînons choisis dans le groupe constitué des hétéroatomes constitué de l'azote, de l'oxygène et du soufre, le reste des groupes fonctionnels hétaryles et hétérocycliques étant constitué par du carbone.

4. Composé de Formule I ou sel pharmaceutiquement acceptable de celui-ci à utiliser dans un procédé de traitement d'une maladie selon la revendication 1, dans lequel M dans la formule -L-(ML¹) pour B représente un groupe -O-, -CH₂-, -S-, -NH-, -C(O)-, -O-CH₂- ou -CH₂-O-.

5. Composé de Formule I ou sel pharmaceutiquement acceptable de celui-ci à utiliser dans un procédé de traitement d'une maladie selon la revendication 1, dans lequel A comporte de 1 à 3 substituants choisis dans le groupe constitué des groupes alkyle en C₁ à C₁₀, alkyle en C₁ à C₁₀ jusqu'à per-halogénosubstitué, -CN, -OH, halogéno, alcoxy en C₁ à C₁₀, alcoxy en C₁ à C₁₀ jusqu'à per-halogénosubstitué et des groupes fonctionnels hétérocycliques en C₃ à C₁₀ comportant au moins 5 chaînons cycliques et de 1 à 2 hétéroatomes choisis dans le groupe constitué de l'azote, de l'oxygène et du soufre.

6. Composé de Formule I ou sel pharmaceutiquement acceptable de celui-ci à utiliser dans un procédé de traitement d'une maladie selon la revendication 1, dans lequel L¹ est substitué 1 à 3 fois par un ou plusieurs substituants choisis dans le groupe constitué des groupes -CN, halogéno jusqu'à per-halogéno, alkyle en C₁ à C₁₀, alcoxy en C₁ à C₁₀, -OH, alkyle en C₁à C₁₀ jusqu'à per-halogénosubstitué, alcoxy en C₁ à C₁₀ jusqu'à per-halogénosubstitué, -OR⁷, -SR⁷, NR⁷R^{7'}, -CO₂R⁷, -C(O)NR⁷R^{7'}, - C(O)-R⁷ ou -NO₂, chaque R⁷ et R^{7'} étant indépendamment choisi parmi un atome d'hydrogène, un groupe alkyle en C₁ à C₁₀, alcoxy en C₁ à C₁₀, alcényle en C₂ à C₁₀, alcénoyle en C₁ à C₁₀, alkyle en C₁ à C₁₀ jusqu'à per-halogénosubstitué, alcoxy en C₁ à C₁₀ jusqu'à per-halogénosubstitué, alcényle en C₂ à C₁₀ jusqu'à per-halogénosubstitué et alcénoyle en C₁ à C₁₀ jusqu'à per-halogénosubstitué.

7. Composé de Formule I ou sel pharmaceutiquement acceptable de celui-ci à utiliser dans un procédé de traitement d'une maladie selon la revendication 1, dans lequel un sel pharmaceutiquement acceptable d'un composé de Formule I est administré, qui est choisi dans le groupe constitué des
a) sels basiques d'acides organiques et d'acides inorganiques choisis dans le groupe constitué de l'acide chlorhydrique, de l'acide bromhydrique, de l'acide sulfurique, de l'acide phosphorique, de l'acide méthanesulfonique, de l'acide trifluorosulfonique, de l'acide benzènesulfonique, de l'acide p-toluènesulfonique (sel de tosylate), de l'acide 1-napthalènesulfonique, de l'acide 2-napthalènesulfonique, de l'acide acétique, de l'acide trifluoroacétique, de l'acide malique, de l'acide tartarique, de l'acide citrique, de l'acide lactique, de l'acide oxalique, de l'acide succinique, de l'acide fumarique, de l'acide maléique, de l'acide benzoïque, de l'acide salicylique, de l'acide phénylacétique et de l'acide mandélique ; et
b) sels acides de bases organiques et inorganiques contenant des cations choisis dans le groupe constitué des cations alcalins, des cations alcalino-terreux, du cation d'ammonium, des cations d'ammonium substitués aliphatiques et des cations d'ammonium substitués aromatiques.

8. Composé de Formule I ou sel pharmaceutiquement acceptable de celui-ci à utiliser dans un procédé de traitement d'une maladie selon la revendication 1 pour le traitement d'une maladie autre que le cancer.

9. Composé de Formule I ou sel pharmaceutiquement acceptable de celui-ci à utiliser dans un procédé de traitement d'une maladie selon la revendication 1, dans lequel la pathologie de l'hôte traité par administration d'un composé de Formule I est la polyarthrite rhumatoïde, l'arthrose, l'arthrite purulente, la métastase tumorale, la parodontopathie, l'ulcère cornéen, la protéinurie, la thrombose coronaire associée à une plaque athéroscléreuse, l'anévrisme aortique, la contraception, l'épidermolyse bulleuse dystrophique, la perte dégénérative de cartilage suite à une lésion traumatique des articulations, les ostéopénies liées à l'activité des MMP, le syndrome de l'articulation temporo-mandibulaire ou la démyélinisation du système nerveux.

10. Composé de Formule I ou sel pharmaceutiquement acceptable de celui-ci à utiliser dans un procédé de traitement d'une maladie selon l'une quelconque des revendications 1 à 8, dans lequel la pathologie de l'hôte traité par administration d'un composé de Formule I est le rhumatisme articulaire aigu, la résorption osseuse, l'ostéoporose post-ménopausique, la sepsie, la sepsie gram négative, le choc infectieux, le choc endotoxique, le choc toxique staphylococcique, le syndrome de réponse inflammatoire systémique, l'affection abdominale inflammatoire (la maladie de Crohn et la recto-colite hémorragique), la réaction de Jarisch-Herxheimer, l'asthme, le syndrome de détresse respiratoire de l'adulte, la fibrose pulmonaire aiguë, la sarcoïdose pulmonaire, la maladie respiratoire allergique, la silicose, l'anthracose, la lésion alvéolaire, l'insuffisance hépatique, la maladie du foie lors d'une inflammation aiguë, l'hépatite alcoolique sévère, la malaria (la malaria due au Plasmodium falciparum et la malaria cérébrale), le diabète de type 2 (NIDDM), l'insuffisance cardiaque congestive, les séquelles de la cardiopathie, l'athérosclérose, la maladie d'Alzheimer, l'encéphalite aiguë, la lésion cérébrale, la sclérose en plaques (démyélinisation et perte des oligodendrocytes dans la sclérose en plaques), le cancer à un stade avancé, la tumeur maligne du système lymphoïde, la pancréatite, l'altération de la cicatrisation lors d'une infection, l'inflammation et le cancer, les syndromes myélodysplasiques, le lupus érythémateux systémique, la cirrhose biliaire, la nécrose intestinale, le psoriasis, le mal des rayons/la toxicité suite à l'administration d'anticorps monoclonaux, la réaction hôte contre greffon (lésion due à l'ischémie-reperfusion et rejet d'allogreffe du rein, du foie, du coeur et de la peau), le rejet d'allogreffe du poumon (bronchite oblitérante) ou les complications dues au remplacement total de la hanche.

11. Composé de Formule I ou sel pharmaceutiquement acceptable de celui-ci à utiliser dans un procédé de traitement d'une maladie selon l'une quelconque des revendications 1 à 8, dans lequel la pathologie de l'hôte traité par administration d'un composé de Formule I est une maladie infectieuse choisie dans le groupe constitué de la tuberculose, l'infection causée par Helicobacter pylori suite à un ulcère gastroduodénal, la maladie de Chaga suite à une infection causée par Trypanosoma cruzi, les effets d'une toxine de type Shiga dus à une infection par E. coli, les effets de l'entérotoxine A dus à une infection par Staphylococcus, la méningococcie et les infections dues à Borrelia burgdorferi, à Treponema pallidum, au cytomégalovirus, au virus de la grippe, au virus de l'encéphalomyélite de Theiler et au virus de l'immunodéficience humaine (VIH).

12. Composé de Formule I ou sel pharmaceutiquement acceptable de celui-ci à utiliser dans un procédé de traitement d'une maladie selon la revendication 1, dans lequel :
Rₐ et R_{b} représentent,
a) indépendamment un atome d'hydrogène,
un groupe fonctionnel à base de carbone choisi dans le groupe constitué des groupes alkyle en C₁ à C₁₀, alcoxy en C₁ à C₁₀, cycloalkyle en C₃ à C₁₀ comportant de 0 à 3 hétéroatomes choisis parmi N, S et O, alcényle en C₂ à C₁₀, alcénoyle en C₁ à C₁₀, aryle en C₆ à C₁₄, hétaryle en C₃ à C₁₂ comportant de 1 à 3 hétéroatomes choisis parmi O, N et S, aralkyle en C₇ à C₂₄, alkaryle en C₇ à C₂₄, alkyle en C₁ à C₁₀ substitué, alcoxy en C₁ à C₁₀ substitué, cycloalkyle en C₃ à C₁₀ substitué comportant de 0 à 3 hétéroatomes choisis parmi N, S et O, aryle en C₆ à C₁₄ substitué, hétaryle en C₃ à C₁₂ substitué comportant de 1 à 3 hétéroatomes choisis parmi N, S et O, aralkyle en C₇ à C₂₄ substitué, alkaryle en C₇ à C₂₄ substitué, où, lorsque Rₐ et R_{b} représentent un groupe substitué, ils sont substitués par un groupe halogéno, jusqu'à per-halogéno, hydroxy, alkyle en C₁ à C₁₀, cycloalkyle en C₃ à C₁₂ comportant de 0 à 3 hétéroatomes choisis parmi O, S et N, hétaryle en C₃ à C₁₂ comportant de 1 à 3 hétéroatomes choisis parmi N, S et O, alcoxy en C₁ à C₁₀, aryle en C₆ à C₁₂, alkyle halogénosubstitué en C₁ à C₆ jusqu'à per-halogénoalkyle, aryle halogénosubstitué en C₆ à C₁₂ jusqu'à per-halogénoaryle, cycloalkyle halogénosubstitué en C₃ à C₁₂ comportant de 0 à 3 hétéroatomes choisis parmi N, S et O, jusqu'à per-halogénocycloalkyle, hétaryle en C₃ à C₁₂ halogénosubstitué jusqu'à per-halogénohétéraryle, aralkyle en C₇ à C₂₄ halogénosubstitué jusqu'à per-halogénoaralkyle, alkaryle en C₇ à C₂₄ halogénosubstitué jusqu'à per-halogénoalkaryle, et -C(O)R_{g}; ou
-OSi(R_{f})₃ où R_{f} représente un atome d'hydrogène, un groupe alkyle en C₁ à C₁₀, alkyle en C₁ à C₁₀, alcoxy en C₁ à C₁₀, cycloalkyle en C₃ à C₁₀ comportant de 0 à 3 hétéroatomes choisis parmi O, S et N, aryle en C₆ à C₁₂, hétaryle en C₃ à C₁₂ comportant de 1 à 3 hétéroatomes choisis parmi O, S et N, aralkyle en C₇ à C₂₄, alkyle en C₁ à C₁₀ substitué, alcoxy en C₁ à C₁₀ substitué, cycloalkyle en C₃ à C₁₂ substitué comportant de 0 à 3 hétéroatomes choisis parmi O, S et N, hétéraryle en C₃ à C₁₂ substitué comportant de 1 à 3 hétéroatomes choisis parmi O, S et N, aryle en C₆ à C₁₂ substitué, et alkaryle en C₇ à C₂₄ substitué, où, lorsque R_{f} représente un groupe substitué, il représente un groupe halogénosubstitué jusqu'à per-halogéno, hydroxy, alkyle en C₁ à C₁₀, cycloalkyle en C₃ à C₁₂ comportant de 0 à 3 hétéroatomes choisis parmi O, S et N, hétaryle en C₃ à C₁₂ comportant de 1 à 3 hétéroatomes choisis parmi N, S et O, alcoxy en C₁ à C₁₀, aryle en C₆ à C₁₂, alkaryle en C_{y}- à C₂₄, aralkyle en C₇ à C₂₄, alkyle halogénosubstitué en C₁ à C₆ jusqu'à per-halogénoalkyle, aryle halogénosubstitué en C₆ à C₁₂ jusqu'à per-halogénoaryle, cycloalkyle halogénosubstitué en C₃ à C₁₂ comportant de 0 à 3 hétéroatomes choisis parmi N, S et O, jusqu'à per-halogénocycloalkyle, hétaryle en C₃ à C₁₂ halogénosubstitué jusqu'à per-halogénohétéraryle, aralkyle en C₇ à C₂₄ halogénosubstitué jusqu'à per-halogénoaralkyle, alkaryle en C₇ à C₂₄ halogénosubstitué jusqu'à per-halogénoalkaryle et -C(O)R_{g},
ou
b) Rₐ et R_{b} forment ensemble une structure hétérocyclique de 5 à 7 chaînons de 1 à 3 hétéroatomes choisis parmi N, S et O, ou une structure hétérocyclique de 5 à 7 chaînons substituée de 1 à 3 hétéroatomes choisis parmi N, S et O, les substituants étant choisis dans le groupe constitué des groupes halogéno jusqu'à per-halogéno, hydroxy, alkyle en C₁ à C₁₀, cycloalkyle en C₃ à C₁₂ comportant de 0 à 3 hétéroatomes choisis parmi O, S et N, hétaryle en C₃ à C₁₂ comportant de 1 à 3 hétéroatomes choisis parmi N, S et O, alcoxy en C₁ à C₁₀, aryle en C₆ à C₁₂, alkaryle en C₇ à C₂₄, aralkyle en C₇ à C₂₄, alkyle en C₁ à C₆ halogénosubstitué jusqu'à per-halogénoalkyle, aryle en C₆ à C₁₂ halogénosubstitué jusqu'à per-halogénoaryle, cycloalkyle en C₃ à C₁₂ halogénosubstitué comportant de 0 à 3 hétéroatomes choisis parmi N, S et O, jusqu'à per-halogénocycloalkyle, hétaryle en C₃ à C₁₂ halogénosubstitué jusqu'à per-halogénohétéraryle, aralkyle en C₇ à C₁₂ halogénosubstitué jusqu'à per-halogénoaralkyle, alkaryle en C₇ à C₂₄ halogénosubstitué jusqu'à per-halogénoalkaryle, et -C(O)R_{g},
ou
c) l'un de Rₐ ou de R_{b} représente un groupe -C(O)-, un groupe alkylène divalent en C₁ à C₅ ou un groupe alkylène divalent en C₁ à C₅ substitué relié au groupe fonctionnel L pour former une structure cyclique comportant au moins 5 chaînons,
dans lequel les substituants du groupe alkylène divalent en C₁ à C₅ substitué sont choisis dans le groupe constitué d'un groupe halogéno, hydroxy, alkyle en C₁ à C₁₀, cycloalkyle en C₃ à C₁₂ comportant de 0 à 3 hétéroatomes choisis parmi O, S et N, hétaryle en C₃ à C₁₂ comportant de 1 à 3 hétéroatomes choisis parmi N, S et O, alcoxy en C₁ à C₁₀, aryle en C₆ à C₁₂, alkaryle en C₇ à C₂₄, aralkyle en C₇ à C₂₄, alkyle halogénosubstitué en C₁ à C₆ jusqu'à per-halogénoalkyle, aryle halogénosubstitué en C₆ à C₁₂ jusqu`à per-halogénoaryle, cycloalkyle halogénosubstitué en C₃ à C₁₂ comportant de 0 à 3 hétéroatomes choisis parmi N, S et O, jusqu'à per-halogénocycloalkyle, hétaryle en C₃ à C₁₂ halogénosubstitué jusqu'à per-halogénohétéraryle, aralkyle en C₇ à C₂₄ halogénosubstitué jusqu'à per-halogénoaralkyle, alkaryle en C₇ à C₂₄ halogénosubstitué jusqu'à per-halogénoalkaryle, et -C(O)R_{g},
dans lequel R_{g} représente un groupe alkyle en C₁ à C₁₀ ; -CN, -CO₂R_{d}, -OR_{d}, - SR_{d}, -NO₂, -C(O) Rₑ, -NR_{d}Rₑ, -NR_{d} C(O)ORₑ et -NR_{d} C(O)Rₑ, et R_{d} et Rₑ sont indépendamment choisis dans le groupe constitué de l'atome d'hydrogène et des groupes alkyle en C₁ à C₁₀, alcoxy en C₁ à C₁₀, cycloalkyle en C₃ à C₁₀ comportant de 0 à 3 hétéroatomes choisis parmi O, N et S, aryle en C₆ à C₁₂, hétaryle en C₃ à C₁₂ comportant de 1 à 3 hétéroatomes choisis parmi O, N et S et aralkyle en C₇ à C₂₄, alkaryle en C₇ à C₂₄, alkyle en C₁ à C₁₀ jusqu'à per-halogénosubstitué, cycloalkyle en C₃ à C₁₀ jusqu'à per-halogénosubstitué comportant de 0 à 3 hétéroatomes choisis parmi O, N et S, aryle en C₆, à C₁₄ jusqu'à per-halogénosubstitué, hétaryle en C₃ à C₁₂ jusqu'à per-halogénosubstitué comportant de 1 à 3 hétéroatomes choisis parmi O, N, et S, alkaryle en C₇ à C₂₄ halogénosubstitué jusqu'à per-halogénoalkaryle, et aralkyle en C₇ à C₂₄ jusqu'à per-halogénosubstitué.

13. Composé de Formule I ou sel pharmaceutiquement acceptable de celui-ci à utiliser dans un procédé de traitement d'une maladie selon la revendication 2, dans lequel ledit groupe fonctionnel cyclique substitué L¹ est un groupe phényle, pyridyle ou pyrimidinyle.

14. Composé de Formule I ou sel pharmaceutiquement acceptable de celui-ci à utiliser dans un procédé de traitement d'une maladie selon la revendication 1, dans lequel L¹ est substitué par -C(O)NR^{a}R^{b} ou -SO₂NR^{a}R^{b}.

15. Utilisation d'un composé de Formule I :
A - D - B (I)
ou d'un sel pharmaceutiquement acceptable de celui-ci pour la préparation d'un médicament destiné au traitement d'une maladie associée à la p38 au sein d'un hôte, la maladie associée à la p38 au sein d'un étang la polyarthrite rhumatoïde, l'arthrose, l'arthrite purulente, la métastase tumorale, la parodontopathie, l'ulcère cornéen, la protéinurie, la thrombose coronaire associée à une plaque athéroscléreuse, l'anévrisme aortique, la contraception, l'épidermolyse bulleuse dystrophique, la perte dégénérative de cartilage suite à une lésion traumatique des articulations, les ostéopénies liées à l'activité des MMP, le syndrome de l'articulation temporo-mandibulaire, la démyélinisation du système nerveux, le rhumatisme articulaire aigu, la résorption osseuse, l'ostéoporose post-ménopausique, la sepsie, la sepsie gram négative, le choc infectieux, le choc endotoxique, le choc toxique staphylococcique, le syndrome de réponse inflammatoire systémique, l'affection abdominale inflammatoire (la maladie de Crohn et la recto-colite hémorragique), la réaction de Jarisch-Herxheimer, l'asthme, le syndrome de détresse respiratoire de l'adulte, la fibrose pulmonaire aiguë, la sarcoïdose pulmonaire, la maladie respiratoire allergique, la silicose, l'anthracose, la lésion alvéolaire, l'insuffisance hépatique, la maladie du foie lors d'une inflammation aiguë, l'hépatite alcoolique sévère, la malaria (la malaria due au Plasmodium falciparum et la malaria cérébrale), le diabète de type 2 (NIDDM), l'insuffisance cardiaque congestive, les séquelles de la cardiopathie, l'athérosclérose, la maladie d'Alzheimer, l'encéphalite aiguë, la lésion cérébrale, la sclérose en plaques (démyélinisation et perte des oligodendrocytes dans la sclérose en plaques), le cancer à un stade avancé, la tumeur maligne du système lymphoïde, la pancréatite, l'altération de la cicatrisation lors d'une infection, l'inflammation et le cancer, les syndromes myélodysplasiques, le lupus érythémateux systémique, la cirrhose biliaire, la nécrose intestinale, le psoriasis, le mal des rayons/la toxicité suite à l'administration d'anticorps monoclonaux, la réaction hôte contre greffon (lésion due à l'ischémie-reperfusion et rejet d'allogreffe du rein, du foie, du coeur et de la peau), le rejet d'allogreffe du poumon (bronchite oblitérante), les complications dues au remplacement total de la hanche, la tuberculose, l'infection causée par Helicobacter pylori suite à un ulcère gastroduodénal, la maladie de Chaga suite à une infection causée par Trypanosoma cruzi, les effets d'une toxine de type Shiga dus à une infection par E. coli, les effets de l'entérotoxine A dus à une infection par Staphylococcus, la méningococcie et les infections dues à Borrelia burgdorferi, à Treponema pallidum, au cytomégalovirus, au virus de la grippe, au virus de l'encéphalomyélite de Theiler ou au virus de l'immunodéficience humaine (VIH) ; formule dans laquelle
D représente un groupe -NH-C(O)-NH-,
A représente un groupe quinolinyle ou isoquinolinyle substitué ou non substitué,
B représente une structure cyclique pontée de formule -L-(ML¹)_{q}, où L représente une structure cyclique de 5 ou 6 chaînons reliée directement à D, L¹ comprend un groupe fonctionnel cyclique substitué comportant au moins 5 chaînons, M représente un groupe de pontage comportant au moins un atome, q représente un nombre entier de 1 à 3, et chaque structure cyclique de L et L¹ contient de 0 à 4 membres du groupe constitué de l'azote, de l'oxygène et du soufre, dans lequel L¹ est substitué par au moins un substituant choisi dans le groupe constitué des groupes -SO₂R^{a}, -SO₂NR^{a}R^{b}, -C(O)R^{a}, -C(O)NR^{a}R^{b} et -C(NR^{a})R^{b}, dans lesquels R^{a} et R^{b} représentent indépendamment un atome d'hydrogène ou un groupe fonctionnel à base de carbone,
dans lequel les substituants de A sont choisis dans le groupe constitué d'un groupe halogéno, jusqu'à per-halogéno, et Wn, où n vaut de 0 à 3 et chaque W est indépendamment choisi dans le groupe constitué des groupes
alkyle en C₁ à C₁₀, alcoxy en C₁ à C₁₀, cycloalkyle en C₃ à C₁₀ comportant au moins cinq chaînons cycliques et de 0 à 3 hétéroatomes choisis parmi N, S et O ; alcényle en C₂ à C₁₀, alcénoyle en C₁ à C₁₀, aryle en C₆ à C₁₄, alkaryle en C₇ à C₂₄, aralkyle en C₇ à C₂₄, hétéroaryle en C₃ à C₁₂ comportant au moins 5 chaînons cycliques et de 1 à 3 hétéroatomes choisis parmi O, N et S, alkhétéroaryle en C₄ à C₂₄ comportant au moins 5 chaînons cycliques et de 1 à 3 hétéroatomes choisis parmi O, N et S;
alkyle en C₁ à C₁₀ substitué, alcoxy en C₁ à C₁₀ substitué, cycloalkyle en C₃ à C₁₀ substitué comportant au moins 5 chaînons cycliques et de 0 à 3 hétéroatomes choisis parmi N, S et O ; alcényle en C₂ à C₁₀ substitué, alcénoyle en C₁ à C₁₀ substitué, aryle en C₆ à C₁₄ substitué, alkaryle en C₇ à C₂₄ substitué, aralkyle en C₇ à C₂₄ substitué, hétéroaryle en C₃ à C₁₂ substitué comportant au moins 5 chaînons et de 1 à 3 hétéroatomes choisis parmi O, N et S, alkhétéroaryle en C₄ à C₂₄ substitué comportant au moins 5 chaînons et de 1 à 3 hétéroatomes choisis parmi O, N et S,
-CN, -CO₂R⁷, -C(C)NR⁷R^{7'}, -C(O)-R⁷, -NO₂, -OR⁷, -SR⁷, NR⁷R^{7'}, -NR⁷C(O)OR^{7'}, - NR⁷C(O)R^{7'}, chaque R⁷ et R^{7'} étant indépendamment choisi parmi un atome d'hydrogène, un groupe alkyle en C₁ à C₁₀, alcoxy en C₁ à C₁₀, alcényle en C₂ à C₁₀, alcénoyle en C₁ à 10, alkyle en C₁ à C₁₀ jusqu'à per-halogénosubstitué, alcoxy en C₁ à C₁₀ jusqu'à per-halogénosubstitué, alcényle en C₂ à C₁₀ jusqu'à per-halogénosubstitué et alcénoyle en C₁ à C₁₀ jusqu'à per-halogénosubstitué, cycloalkyle en C₃ à C₁₀ comportant au moins 5 chaînons cycliques et de 0 à 3 hétéroatomes choisis parmi O, S et N, aryle en C₆ à C₁₄, hétaryle en C₃ à C₁₀ comportant au moins 6 chaînons cycliques et de 0 à 3 hétéroatomes choisis parmi O S et N, cycloalkyle en C₃ à C₁₀ jusqu'à per-halogénosubstitué comportant au moins 5 chaînons cycliques et de 0 à 3 hétéroatomes choisis parmi O, S et N, aryle en C₆ à C₁₄ jusqu'à per-halogénosubstitué et hétaryle en C₃ à C₁₀ jusqu'à per-halogénosubstitué comportant au moins 6 chaînons cycliques et de 0 à 3 hétéroatomes choisis parmi O, S et N,
où, lorsque W représente un groupe substitué, il est substitué par un groupe halogéno, jusqu'à per-halogéno, ou par un ou plusieurs substituants indépendamment choisis dans le groupe constitué des groupes -CN, -CO₂R⁷, - C(O)NR⁷R^{7'}, -C(O)-R⁷, -NO₂, -OR⁷, -SR⁷, NR⁷R^{7'}, -NR⁷C(O)OR^{7'} et -NR⁷C(O)R^{7'}, où R⁷ et R^{7'} sont indépendamment tels que définis ci-dessus.

16. Composé choisi dans le groupe constitué des composés suivants :
*N*-(2-Méthoxy-3-quinolyl)-*N*'-(4-[3-(*N*-méthylcarbamoyl)phénoxy]phényl)urée
*N*-(2-Méthoxy-3-quinolyl)-*N*'-(4-[2-(*N*-méthylcarbamoyl)-4-pyridyloxy]phényl)urée
*N*-(2-Méthoxy-3-quinolyl)-*N*'-(4-(2-carbamoyl-4-pyridyloxy)phényl)urée
*N*-(2-Méthoxy-3-quinolyl)-*N*'-(3-[2-(*N*-méthylcarbamoyl)-4-pyridyloxy]phényl)urée
*N*-(2-Méthoxy-3-quinolyl)-*N*'-(3-(2-carbamoyl)-4-pyridyloxy)phényl)urée
*N*-(2-Méthoxy-3-quinolyl)-*N*'-(4-[3-(*N*-isopropylcarbamoyl)phénoxy]phényl)urée
*N*-(2-Méthoxy-3-quinolyl)-*N*'-(4-[4-méthoxy-3-(*N-*méthylcarbamoyl)phénoxy]phényl)urée
*N*-(3-Isoquinolyl)-*N*'-(4-[2-(*N*-méthylcarbamoyl)-4-pyridyloxy]phényl)urée et sels pharmaceutiquement acceptables de ceux-ci ;
à utiliser dans un procédé de traitement d'une maladie associée à la p38 kinase telle que définie selon la revendication 1, autre que le cancer.

17. Utilisation d'un composé choisi dans le groupe constitué des composés suivants :
*N*-(2-Méthoxy-3-quinolyl)-*N*'-(4-[3-(*N*-méthylcarbamoyl)phénoxy]phényl)urée
*N*-(2-Méthoxy-3-quinolyl)-*N*'-(4-[2-(*N*-méthylcarbamoyl)-4-pyridyloxy]phényl)urée
*N*-(2-Méthoxy-3-quinolyl)-*N*'-(4-(2-carbamoyl-4-pyridyloxy)phényl)urée
*N*-(2-Méthoxy-3-quinolyl-*N*'-(3-[2-(*N*'-méthylcarbamoyl)-4-pyridyloxy]phényl)urée
*N*-(2-Méthoxy-3-quinolyl)-*N*'-(3-(2-carbamoyl)-4-pyridyloxy)phényl)urée
*N*-(2-Méthoxy-3-quinolyl)-*N*'-(4-[3-(*N*-isopropylcarbamoyl)phénoxy]phényl)urée
*N*-(2-Méthoxy-3-quinolyl)-*N*'-(-[4-méthoxy-3-(*N-*méthylcarbamoyl)phénoxy]phényl)urée
*N*-(3-Isoquinolyl)-*N*'-(4-[2-(*N*-méthylcarbamoyl)-4-pyridyloxy]phényl)urée et sels pharmaceutiquement acceptables de ceux-ci ;
pour la préparation d'un médicament destiné au traitement d'une maladie associée à la p38 kinase telle que définie selon la revendication 1, autre que le cancer.

18. Composition pharmaceutique pour le traitement d'une maladie au sein d'un hôte associée à la p38 comprenant une quantité de composé de Formule I efficace pour inhiber les effets de la p38,
A-D-B (I)
ou sel pharmaceutiquement acceptable de celui-ci, en une quantité efficace pour traiter une maladie associée à la p38 et vecteur physiologiquement acceptable ; formule dans laquelle
D représente un groupe -*N*H-C(O)-*N*H-,
A est tel que défini selon la revendication 1
B représente une structure cyclique pontée de formule -L-(ML¹)_{q}, où L représente une structure cyclique de 5 ou 6 chaînons reliée directement à D, L¹ comprend un groupe fonctionnel cyclique substitué comportant au moins 5 chaînons, M représente un groupe de pontage comportant au moins un atome, q représente un nombre entier de 1 à 3, et chaque structure cyclique de L et L¹ contient de 0 à 4 membres du groupe constitué de l'azote, de l'oxygène et du soufre, dans lequel L¹ est substitué par au moins un substituant choisi dans le groupe constitué des groupes -SO₂Rₓ, -C(O)Rₓ et -C(*N*R_{y})R_{z} dans lesquels R_{y} représente un atome d'hydrogène ou un groupe fonctionnel à base de carbone comportant jusqu'à 24 atomes de carbone contenant facultativement des hétéroatomes choisis parmi *N*, S et O et étant facultativement halogénosubstitué, jusqu'à per-halogéno,
R_{z} représente un atome d'hydrogène ou un groupe fonctionnel à base de carbone comportant jusqu'à 30 atomes de carbone contenant facultativement des hétéroatomes choisis parmi *N*, S et O et facultativement substitué par un atome d'halogène, un groupe hydroxy et des substituants à base de carbone comportant jusqu'à 24 atomes de carbone, qui contiennent facultativement des hétéroatomes choisis parmi *N*, S et O et qui sont facultativement substitués par un atome d'halogène ;
Rₓ représente R_{z} ou NRₐR_{b} où Rₐ et R_{b} représentent
a) indépendamment un atome d'hydrogène,
un groupe fonctionnel à base de carbone comportant jusqu'à 30 atomes de carbone contenant facultativement des hétéroatomes choisis parmi N, S et O et facultativement substitué par un atome d'halogène, un groupe hydroxy et des substituants à base de carbone comportant jusqu'à 24 atomes de carbone, qui contiennent facultativement des hétéroatomes choisis parmi N, S et O et qui sont facultativement substitués par un atome d'halogène, ou
-OSi(R_{f})₃ où R_{f} représente un atome d'hydrogène ou un groupe fonctionnel à base de carbone comportant jusqu'à 24 atomes de carbone contenant facultativement des hétéroatomes choisis parmi N, S et O et facultativement substitué par un atome d'halogène, un groupe hydroxy et des substituants à base de carbone comportant jusqu'à 24 atomes de carbone, qui contiennent facultativement des hétéroatomes choisis parmi N, S et O et qui sont facultativement substitués par un atome d'halogène ; ou
b) Rₐ et R_{b} forment ensemble une structure hétérocyclique de 5 à 7 chaînons de 1 à 3 hétéroatomes choisis parmi N, S et O, ou une structure hétérocyclique de 5 à 7 chaînons substituée de 1 à 3 hétéroatomes choisis parmi N, S et O substitués par un atome d'halogène, un groupe hydroxy ou des substituants à base de carbone comportant jusqu'à 24 atomes de carbone, qui contiennent facultativement des hétéroatomes choisis parmi N, S et O et qui sont facultativement substitués par un atome d'halogène ; ou
c) l'un de Rₐ ou de R_{b} représente un groupe -C(O)-, un groupe alkylène divalent en C₁ à C₅ ou un groupe alkylène divalent en C₁ à C₅ substitué relié au groupe fonctionnel L pour former une structure cyclique comportant au moins 5 chaînons, dans lequel les substituants du groupe alkylène divalent en C₁ à C₅ substitué sont choisis dans le groupe constitué d'un atome d'halogène, d'un groupe hydroxy et de substituants à base de carbone comportant jusqu'à 24 atomes de carbone, qui contiennent facultativement des hétéroatomes choisis parmi N, S et O et qui sont facultativement substitués par un atome d'halogène.

19. Composition pharmaceutique selon la revendication 18, dans laquelle les structures cycliques de B et L reliées directement à D ne sont pas substituées en position ortho par -OH ou par un groupe fonctionnel comportant un atome d'hydrogène ionisable et présentant un pKa inférieur ou égal à 10.

20. Composition pharmaceutique selon la revendication 18, dans laquelle B de la Formule I est un groupe fonctionnel aryle à six chaînons substitué ou non substitué ou un groupe fonctionnel hétérocylique d'au moins cinq chaînons, ledit groupe fonctionnel hétérocyclique comportant de 1 à 4 chaînons choisis dans le groupe constitué des atomes hétaryles consistant en azote, oxygène et soufre, le reste des groupes fonctionnels hétérocyliques étant constitué de carbone.

21. Composition pharmaceutique selon la revendication 18, dans laquelle L, la structure cyclique à 5 ou 6 chaînons reliée directement à D, est un groupe fonctionnel hétéroaryle à 6 chaînons substitué ou non substitué, dans laquelle ledit groupe fonctionnel hétéroaryle comporte de 1 à 4 chaînons choisis dans le groupe constitué des hétéroatomes consistant en azote, oxygène et soufre, le reste des groupes fonctionnels hétaryles étant constitué de carbone, dans laquelle le ou les substituants sont choisis dans le groupe constitué d'un groupe halogéno et Wn, où W et n sont tels que définis dans la revendication 18.

22. Composition pharmaceutique selon la revendication 18 dans laquelle L¹ est substitué par -C(O)Rₓ.

23. Composition pharmaceutique selon la revendication 18 dans laquelle L¹ est substitué par -C(O)Rₓ ou -SO₂Rₓ, dans lesquels Rₓ représente un groupe NR^{a}R^{b}.

24. Composition pharmaceutique pour le traitement d'une maladie au sein d'un hôte associée à la p38 comprenant un composé choisi dans le groupe constitué des composés suivants :
*N*-(2-Méthoxy-3-quinolyl)-*N*'-(4-[3-(*N*-méthylcarbamoyl)phénoxy]phényl)urée
*N*-(2-Méthoxy-3-quinolyl)-*N*'-(4-[2-(*N*-méthylcarbamoyl)pyridyloxy]phényl)urée
*N*-(2-Méthoxy-3-quinolyl)-*N*'(4-(2-carbamoyl-4-pyridyloxy)phényl)urée
*N*-(2-Méthoxy-3-quinolyl)-*N*'-(3-[2-(*N*-méthylcarbamoyl)-4-pyridyloxy]phényl)urée
*N*-(2-Méthoxy-3-quinolyl)-*N*'-(3-(2-carbamoyl)-4-pyridyloxy)phényl)urée
*N*-(2-Méthoxy-3-quinolyl)-*N*'-(4-[3-(*N*-isopropylcarbamoyl)phénoxy]phényl)urée
*N*-(2-Méthoxy-3-quinolyl)-*N*'-(4-[4-(*N*-méthoxy-3-(*N-*méthylcarbamoyl)phénoxy]phényl)urée
*N*-(2-Méthoxy-3-quinolyl)-*N*'-(4-[2-(*N*-méthylcarbamoyl)pyridyloxy]phényl)urée et des sels pharmaceutiquement acceptables de ceux-ci.

25. Utilisation d'une composition selon l'une quelconque des revendications 18 à 24 pour la préparation d'un médicament destiné au traitement d'une maladie associée à la p38 au sein d'un hôte, dans laquelle la maladie associée à la p38 est telle que définie selon la revendication 1.
